# EUROPEAN PATENT APPLICATION

(11) **EP 4 707 305 A1**
(43) Date of publication of application: **11.03.2026**
(21) Application number: 24806527.8
(22) Date of filing: 11.05.2024
(51) Int. Cl.: C07K 16/30, C07K 16/28, C12N 15/13, C12N 15/85, C12N 15/79, C07K 16/06

(54) **ANTI-CLDN6 ANTIBODY AND USE THEREOF**

(30) Priority: 12.05.2023 WO PCT/CN2023/093998
(71) Applicant: Pediatric Immunity and Healthcare (Guangzhou) Biotechnology Co., Ltd., Guangzhou, Guangdong 510005 (CN)
(72) Inventor: HAN, Yang, Guangzhou, Guangdong 510005 (CN); LIU, Ming, Guangzhou, Guangdong 510005 (CN); GUO, Fang, Guangzhou, Guangdong 510005 (CN)
(74) Representative: Prüfer & Partner mbB Patentanwälte · Rechtsanwälte
(86) International application number: PCT/CN2024/092728
(87) International publication number: WO 2024/235175

(57) **Abstract**

Provided is an anti-claudin-6 (CLDN6) antibody or an antigen-binding fragment thereof, including a chimeric monoclonal antibody and a humanized monoclonal antibody. This application further provides a nucleic acid encoding the anti-CLDN6 antibody, an expression vector and a host cell for expressing the anti-CLDN6 antibody, a conjugate, a multispecific antibody, a chimeric antigen receptor (CAR), and a pharmaceutical composition including the anti-CLDN6 antibody, and a method for verifying a function and efficacy of the anti-CLDN6 antibody. The anti-CLDN6 antibody of this application can be used for diagnosing a cancer and/or determining whether a cancer cell expresses CLDN6, and can also be used for treating and/or preventing various cancers or diseases associated with CLDN6-expressing cells in a subject.

## Description

### CROSS REFERENCE TO RELATED APPLICATION

The present application claims the benefit and priority of the PCT application PCT/CN2023/093998 filed on May 12, 2023, which is incorporated herein by reference in its entirety for all purposes.

### TECHNICAL FIELD

The present disclosure generally relates to antibodies. Specifically, the present disclosure relates to an anti-CLDN6 chimeric antibody and humanized antibody, and a preparation method and use thereof.

### BACKGROUND

Antibodies play a significant role in clinical therapy, *in vitro* diagnostics, scientific research, and other fields. Antibody drugs have become a hotspot in global drug development. Antibody drugs demonstrate critical efficacy in tumor eradication, autoimmune disease treatment, and post-transplantation immunosuppression, and also hold irreplaceable significance in the prevention and treatment of viral infection-associated diseases. Over 100 antibody therapeutics have been approved by the Food and Drug Administration (FDA) for market release. Compared to conventional small-molecule drugs, antibody therapeutics offer distinct advantages such as high target specificity and enhanced therapeutic efficacy, and show absolute advantages especially in the treatment of tumors and autoimmune diseases. In addition to exerting remarkable efficacy in combination therapies, antibody drugs can serve as "biological missiles" through the antibody-drug conjugate technology, and carry chemotherapeutic drugs and other small-molecule drugs directly to lesions, thereby achieving precision medicine with enhanced therapeutic outcomes and reduced toxic and side effects.

Claudins (CLDNs) are tight junction proteins primarily expressed in a tissue-specific manner within endothelial or epithelial cells. Extensive research data from The Cancer Genome Atlas (TCGA) reveals that the expression level of claudin-6 (CLDN6), a member of the CLDN family, is significantly up-regulated in ovarian cancer patients compared to normal ovarian tissues. The expression level of CLDN6 is 0 transcripts per million (TPM) in normal ovarian tissues (n = 88), but is 31.4 TPM in ovarian tumors (n = 426). CLDN6 is highly expressed in fetal tissues such as stomach, pancreas, lung, and kidney. Studies have shown that the expression level of CLDN6 is 159.9 TPM in testicular cancer (n = 137) and 8.4 TPM in uterine sarcoma (n = 57), but CLDN6 is not expressed in the corresponding normal adult tissues. CLDN6 has the highest expression level in testes among normal adult tissues, which is only 0.83 TPM.

The role of CLDN6 in cancers has attracted attention. Compelling evidence indicates that the altered expression of CLDN6 is related to the development of various cancers. Malignant phenotypes of tumors affected by CLDN6 include proliferation and apoptosis, migration and invasion, and drug resistance. These phenotypes are regulated by CLDN6-mediated key signaling pathways. Kaplan-Meier analysis reveals significant differences in the overall survival and recurrence-free survival between CLDN6 high-expression and low-expression groups. The five-year survival rate is approximately 30% in the CLDN6 high-expression group and is 89% in the CLDN6 low-expression group. Besides endometrial cancer, the high expression of CLDN6 is negatively correlated with the prognosis of gastric cancer and urothelial carcinoma. Given the crucial role of CLDN6 in tumors, the low or no expression of CLDN6 in normal tissues, the high expression of CLDN6 in tumor tissues, and the negative correlation of CLDN6 with tumor prognosis, CLDN6 is an ideal target for cancer therapy.

CLDN6 is a four-transmembrane protein with four transmembrane hydrophobic domains and two extracellular loops. It is extremely challenging to express the recombinant CLDN6 protein. As a result, there is no suitable protein antigen for immunization, which complicates the immunization and screening for anti-CLDN6 antibodies. Further, due to high homologies among proteins of the CLDN family, antibodies against CLDN6 need to be screened to avoid the binding to other family members with high homologies with CLDN6, particularly the binding to CLDN3 widely expressed in normal tissues, thereby preventing potential toxicity caused by cross-binding. Additionally, it is both difficult and essential to select anti-CLDN6 antibodies suitable for different tissues or different types of tumors. The present disclosure is intended to address the aforementioned issues.

### SUMMARY

The present disclosure provides an antibody, a method, a composition, a product, etc. The benefits provided by the present disclosure are broadly applicable to the field of antibody therapy and diagnosis. The antibody of the present disclosure can be used in combination with antibodies against various targets. The present disclosure provides an antibody that binds to human CLDN6, and preferably a chimeric monoclonal antibody or a humanized monoclonal antibody. The present disclosure also provides a nucleic acid encoding the anti-CLDN6 antibody, and a vector and host cell for expressing the anti-CLDN6 antibody. The anti-CLDN6 antibody of the present disclosure can be used for diagnosing a cancer and/or determining whether a cancer cell expresses CLDN6, and can also be used for treating various cancers in a subject.

In an aspect, the present disclosure provides an isolated anti-CLDN6 antibody or an antigen-binding fragment thereof, including a heavy-chain variable region and a light-chain variable region,
where the isolated anti-CLDN6 antibody includes:
   (1) heavy-chain complementarity determining region (CDRH) 1 including or consisting of a sequence X₁YNMH, where X₁ is selected from a group consisting of D and S;
   (2) CDRH2 including or consisting of a sequence YX₁X₂PX₃X₄X₅X₆TX₇YNQKFKG, where X₁ is selected from a group consisting of V and I, X₂ is selected from a group consisting of N and Y, X₃ is selected from a group consisting of N and G, X₄ is selected from a group consisting of N, Q, S, and A, X₅ is selected from a group consisting of A, G, D, Q, and S, X₆ is selected from a group consisting of A and G, and X₇ is selected from a group consisting of S and N;
   (3) CDRH3 including or consisting of a sequence WX₁X₂YVYYYGX₃DY or a sequence shown in SEQ ID NO: 23 (that is, consisting of the sequence WX₁X₂YVYYYGX₃DY or the sequence shown in SEQ ID NO: 23), where X₁ is selected from a group consisting of D, E, S, and G, X₂ is selected from a group consisting of A, G, S, D, and Q, and X₃ is selected from a group consisting of L and M;
   (4) light-chain complementarity determining region (CDRL) 1 including or consisting of a sequence shown in SEQ ID NO: 27 or a sequence shown in SEQ ID NO: 29 (that is, consisting of the sequence shown in SEQ ID NO: 27 or the sequence shown in SEQ ID NO: 29);
   (5) CDRL2 comprising or consisting of a sequence shown in SEQ ID NO: 30 or a sequence shown in SEQ ID NO: 31 (that is, consisting of the sequence shown in SEQ ID NO: 30 or the sequence shown in SEQ ID NO: 31); and
   (6) CDRL3 comprising or consisting of a sequence QQYWX₁TPYT or a sequence shown in SEQ ID NO: 40 (that is, consisting of the sequence QQYWX₁TPYT or the sequence shown in SEQ ID NO: 40), where X₁ is selected from a group consisting of S and N; or
the isolated anti-CLDN6 antibody includes:
   (1) CDRH1 including or consisting of a sequence SYX₁IX₂, where X₁ is selected from a group consisting of N and G and X₂ is selected from a group consisting of H and S;
   (2) CDRH2 including or consisting of a sequence X₁IYPX₂X₃GX₄TX₅X₆NEX₇FKG, where X₁ is selected from a group consisting of Y and E, X₂ is selected from a group consisting of G and R, X₃ is selected from a group consisting of N and S, X₄ is selected from a group consisting of G and N, X₅ is selected from a group consisting of K and Y, X₆ is selected from a group consisting of Y and H, and X₇ is selected from a group consisting of R and K;
   (3) CDRH3 including or consisting of a sequence shown in SEQ ID NO: 16 or a sequence shown in SEQ ID NO: 19;
   (4) CDRL1 including or consisting of a sequence shown in SEQ ID NO: 24 or a sequence shown in SEQ ID NO: 26;
   (5) CDRL2 including or consisting of a sequence X₁X₂SNLX₃S, where X₁ is selected from a group consisting of L and G, X₂ is selected from a group consisting of A and T, and X₃ is selected from a group consisting of E and A; and
   (6) CDRL3 including or consisting of a sequence shown in SEQ ID NO: 34 or a sequence shown in SEQ ID NO: 179; or
the isolated anti-CLDN6 antibody includes:
   (1) CDRH1 including or consisting of a sequence X₁YX₂MS, where X₁ is selected from a group consisting of S and N and X₂ is selected from a group consisting of G and A;
   (2) CDRH2 including or consisting of a sequence TISX₁GGSYTX₂YPDX₃X₄KG, where X₁ is S or D, X₂ is selected from a group consisting of Y and S, X₃ is selected from a group consisting of S and N, and X₄ is selected from a group consisting of V and I;
   (3) CDRH3 including or consisting of the sequence shown in SEQ ID NO: 17 or a sequence shown in SEQ ID NO: 18;
   (4) CDRL1 including or consisting of a sequence shown in SEQ ID NO: 25;
   (5) CDRL2 including or consisting of a sequence X₁X₂TSLET, where X₁ is selected from a group consisting of G and A and X₂ is selected from a group consisting of A and T; and
   (6) CDRL3 including or consisting of a sequence QQYWSX₁PX₂T, where X₁ is selected from a group consisting of T and F and X₂ is selected from a group consisting of P and R.

In an aspect, the present disclosure provides an isolated anti-CLDN6 antibody or an antigen-binding fragment thereof, including a heavy-chain variable region and a light-chain variable region, where the heavy-chain variable region includes:
(1) CDRH1 including or consisting of a sequence shown in SEQ ID NO: 5;
(2) CDRH2 including or consisting of a sequence YX₁NPNX₂X₃ATX₄YNQKFKG, where X₁ is selected from a group consisting of V and I, X₂ is selected from a group consisting of N, Q, S, and A, X₃ is selected from a group consisting of A, G, D, Q, and S, and X₄ is selected from a group consisting of S and N; and
(3) CDRH3 including or consisting of a sequence WDGYVYYYGX₁DY, where X₁ is selected from a group consisting of L and M; and
(4) CDRL1 including or consisting of a sequence shown in SEQ ID NO: 27;
(5) CDRL2 including or consisting of a sequence shown in SEQ ID NO: 31; and
(6) CDRL3 including or consisting of a sequence QQYWX₁TPYT, where X₁ is selected from a group consisting of S and N.

In an aspect, the present disclosure provides an isolated anti-CLDN6 antibody or an antigen-binding fragment thereof, including a heavy-chain variable region and a light-chain variable region, where the heavy-chain variable region includes:
(1) CDRH1 including or consisting of a sequence shown in SEQ ID NO: 5;
(2) CDRH2 including or consisting of a sequence YX₁NPNNGATX₂YNQKFKG, where X₁ is selected from a group consisting of V and I and X₂ is selected from a group consisting of S and N; and
(3) CDRH3 including or consisting of a sequence WDGYVYYYGX₁DY, where X₁ is selected from a group consisting of L and M; and
(4) CDRL1 including or consisting of a sequence shown in SEQ ID NO: 27;
(5) CDRL2 including or consisting of a sequence shown in SEQ ID NO: 31; and
(6) CDRL3 including or consisting of a sequence QQYWX₁TPYT, where X₁ is selected from a group consisting of S and N.

In an aspect, the present disclosure provides an isolated anti-CLDN6 antibody or an antigen-binding fragment thereof, including a heavy-chain variable region and a light-chain variable region, where the heavy-chain variable region includes:
(i) CDRH1 including any one of sequences shown in SEQ ID NOs: 1-5 and 7 or a sequence having a sequence identity of at least 90%, 91%, 92%, 93%, 94%, 95%, 96%, 97%, 98%, or 99% with any one of the sequences shown in SEQ ID NOs: 1-5 and 7;
(ii) CDRH2 including any one of sequences shown in SEQ ID NOs: 8-13, 15, and 74-76 or a sequence having a sequence identity of at least 90%, 91%, 92%, 93%, 94%, 95%, 96%, 97%, 98%, or 99% with any one of the sequences shown in SEQ ID NOs: 8-13, 15, and 74-76; and
(iii) CDRH3 including any one of sequences shown in SEQ ID NOs: 16-21, 23, and 77-78 or a sequence having a sequence identity of at least 90%, 91%, 92%, 93%, 94%, 95%, 96%, 97%, 98%, or 99% with any one of the sequences shown in SEQ ID NOs: 16-21, 23, and 77-78; and
the light-chain variable region includes:
CDRL1 including any one of sequences shown in SEQ ID NOs: 24-27 and 29 or a sequence having a sequence identity of at least 90%, 91%, 92%, 93%, 94%, 95%, 96%, 97%, 98%, or 99% with any one of the sequences shown in SEQ ID NOs: 24-27 and 29;
CDRL2 including any one of sequences shown in SEQ ID NOs: 30-33 or a sequence having a sequence identity of at least 90% with any one of the sequences shown in SEQ ID NOs: 30-33; and
CDRL3 including any one of sequences shown in SEQ ID NOs: 34-38, 40, and 179 or a sequence having a sequence identity of at least 90%, 91%, 92%, 93%, 94%, 95%, 96%, 97%, 98%, or 99% with any one of the sequences shown in SEQ ID NOs: 34-38, 40, and 179.

In some embodiments, the present disclosure provides an isolated anti-CLDN6 antibody or an antigen-binding fragment thereof, including:
(a) CDRH1 including any one of sequences shown in SEQ ID NOs: 1-5 and 7;
(b) CDRH2 including any one of sequences shown in SEQ ID NOs: 8-13, 15, and 74-76;
(c) CDRH3 including any one of sequences shown in SEQ ID NOs: 16-21, 23, and 77-78;
(d) CDRL1 including any one of sequences shown in SEQ ID NOs: 24-27 and 29;
(e) CDRL2 including any one of sequences shown in SEQ ID NOs: 30-33; and
(f) CDRL3 including any one of sequences shown in SEQ ID NOs: 34-38, 40, and 179.

In some embodiments, the present disclosure provides an isolated anti-CLDN6 antibody or an antigen-binding fragment thereof, including:
(a) CDRH1 including or consisting of a sequence shown in SEQ ID NO: 1; CDRH2 including or consisting of a sequence shown in SEQ ID NO: 8; CDRH3 including or consisting of a sequence shown in SEQ ID NO: 16; CDRL1 including or consisting of a sequence shown in SEQ ID NO: 24; CDRL2 including or consisting of a sequence shown in SEQ ID NO: 30; and CDRL3 including or consisting of a sequence shown in SEQ ID NO: 34;
(b) CDRH1 including or consisting of a sequence shown in SEQ ID NO: 2; CDRH2 including or consisting of a sequence shown in SEQ ID NO: 9; CDRH3 including or consisting of a sequence shown in SEQ ID NO: 17; CDRL1 including or consisting of a sequence shown in SEQ ID NO: 25; CDRL2 including or consisting of a sequence shown in SEQ ID NO: 31; and CDRL3 including or consisting of a sequence shown in SEQ ID NO: 35;
(c) CDRH1 including or consisting of a sequence shown in SEQ ID NO: 3; CDRH2 including or consisting of a sequence shown in SEQ ID NO: 10; CDRH3 including or consisting of a sequence shown in SEQ ID NO: 18; CDRL1 including or consisting of the sequence shown in SEQ ID NO: 25; CDRL2 including or consisting of a sequence shown in SEQ ID NO: 32; and CDRL3 including or consisting of a sequence shown in SEQ ID NO: 36;
(d) CDRH1 including or consisting of a sequence shown in SEQ ID NO: 4; CDRH2 including or consisting of a sequence shown in SEQ ID NO: 11; CDRH3 including or consisting of a sequence shown in SEQ ID NO: 19; CDRL1 including or consisting of a sequence shown in SEQ ID NO: 26; CDRL2 including or consisting of a sequence shown in SEQ ID NO: 33; and CDRL3 including or consisting of a sequence shown in SEQ ID NO: 179;
(e) CDRH1 including or consisting of a sequence shown in SEQ ID NO: 5; CDRH2 including or consisting of a sequence shown in SEQ ID NO: 12; CDRH3 including or consisting of a sequence shown in SEQ ID NO: 20; CDRL1 including or consisting of a sequence shown in SEQ ID NO: 27; CDRL2 including or consisting of the sequence shown in SEQ ID NO: 31; and CDRL3 including or consisting of a sequence shown in SEQ ID NO: 37;
(f) CDRH1 including or consisting of the sequence shown in SEQ ID NO: 5; CDRH2 including or consisting of a sequence shown in SEQ ID NO: 13; CDRH3 including or consisting of a sequence shown in SEQ ID NO: 21; CDRL1 including or consisting of the sequence shown in SEQ ID NO: 27; CDRL2 including or consisting of the sequence shown in SEQ ID NO: 31; and CDRL3 including or consisting of a sequence shown in SEQ ID NO: 38;
(g) CDRH1 including or consisting of a sequence shown in SEQ ID NO: 7; CDRH2 including or consisting of a sequence shown in SEQ ID NO: 15; CDRH3 including or consisting of a sequence shown in SEQ ID NO: 23; CDRL1 including or consisting of a sequence shown in SEQ ID NO: 29; CDRL2 including or consisting of the sequence shown in SEQ ID NO: 30; and CDRL3 including or consisting of a sequence shown in SEQ ID NO: 40;
(h) CDRH1 including or consisting of the sequence shown in SEQ ID NO: 5; CDRH2 including or consisting of the sequence shown in SEQ ID NO: 12; CDRH3 including or consisting of the sequence shown in SEQ ID NO: 20; CDRL1 including or consisting of the sequence shown in SEQ ID NO: 27; CDRL2 including or consisting of the sequence shown in SEQ ID NO: 31; and CDRL3 including or consisting of the sequence shown in SEQ ID NO: 38;
(i) CDRH1 including or consisting of the sequence shown in SEQ ID NO: 7; CDRH2 including or consisting of the sequence shown in SEQ ID NO: 24; CDRH3 including or consisting of the sequence shown in SEQ ID NO: 23; CDRL1 including or consisting of the sequence shown in SEQ ID NO: 29; CDRL2 including or consisting of the sequence shown in SEQ ID NO: 30; and CDRL3 including or consisting of the sequence shown in SEQ ID NO: 40;
(j) CDRH1 including or consisting of the sequence shown in SEQ ID NO: 5; CDRH2 including or consisting of a sequence shown in SEQ ID NO: 75; CDRH3 including or consisting of the sequence shown in SEQ ID NO: 20; CDRL1 including or consisting of the sequence shown in SEQ ID NO: 27; CDRL2 including or consisting of the sequence shown in SEQ ID NO: 31; and CDRL3 including or consisting of the sequence shown in SEQ ID NO: 37;
(k) CDRH1 including or consisting of the sequence shown in SEQ ID NO: 5; CDRH2 including or consisting of a sequence shown in SEQ ID NO: 76; CDRH3 including or consisting of the sequence shown in SEQ ID NO: 20; CDRL1 including or consisting of the sequence shown in SEQ ID NO: 27; CDRL2 including or consisting of the sequence shown in SEQ ID NO: 31; and CDRL3 including or consisting of the sequence shown in SEQ ID NO: 37;
(l) CDRH1 including or consisting of the sequence shown in SEQ ID NO: 5; CDRH2 including or consisting of the sequence shown in SEQ ID NO: 12; CDRH3 including or consisting of a sequence shown in SEQ ID NO: 77; CDRL1 including or consisting of the sequence shown in SEQ ID NO: 27; CDRL2 including or consisting of the sequence shown in SEQ ID NO: 31; and CDRL3 including or consisting of the sequence shown in SEQ ID NO: 37; or
(m) CDRH1 including or consisting of the sequence shown in SEQ ID NO: 5; CDRH2 including or consisting of the sequence shown in SEQ ID NO: 12; CDRH3 including or consisting of a sequence shown in SEQ ID NO: 78; CDRL1 including or consisting of the sequence shown in SEQ ID NO: 27; CDRL2 including or consisting of the sequence shown in SEQ ID NO: 31; and CDRL3 including or consisting of the sequence shown in SEQ ID NO: 37.

In some embodiments, the heavy-chain variable region includes or consists of any one of amino acid sequences shown in SEQ ID NOs: 41-46, 48-60, and 79-83, or includes or consists of a sequence having a sequence identity of at least 85%, 90%, 91%, 92%, 93%, 94%, 95%, 96%, 97%, 98%, or 99% with any one of the amino acid sequences shown in SEQ ID NOs: 41-46, 48-60, and 79-83; and the light-chain variable region includes or consists of any one of amino acid sequences shown in SEQ ID NOs: 61-66 and 68-73, or includes or consists of a sequence having a sequence identity of at least 85%, 90%, 91%, 92%, 93%, 94%, 95%, 96%, 97%, 98%, or 99% with any one of the amino acid sequences shown in SEQ ID NOs: 61-66 and 68-73.

In some embodiments, the isolated anti-CLDN6 antibody or the antigen-binding fragment thereof includes:
(a) a heavy-chain variable region including or consisting of an amino acid sequence shown in SEQ ID NO: 41 and a light-chain variable region including or consisting of an amino acid sequence shown in SEQ ID NO: 61;
(b) a heavy-chain variable region including or consisting of an amino acid sequence shown in SEQ ID NO: 42 and a light-chain variable region including or consisting of an amino acid sequence shown in SEQ ID NO: 62;
(c) a heavy-chain variable region including or consisting of an amino acid sequence shown in SEQ ID NO: 43 and a light-chain variable region including or consisting of an amino acid sequence shown in SEQ ID NO: 63;
(d) a heavy-chain variable region including or consisting of an amino acid sequence shown in SEQ ID NO: 44 and a light-chain variable region including or consisting of an amino acid sequence shown in SEQ ID NO: 64;
(e) a heavy-chain variable region including or consisting of any one of amino acid sequences shown in SEQ ID NOs: 45 and 80-83 and a light-chain variable region including or consisting of an amino acid sequence shown in SEQ ID NO: 65;
(f) a heavy-chain variable region including or consisting of an amino acid sequence shown in SEQ ID NO: 46 or 45 and a light-chain variable region including or consisting of an amino acid sequence shown in SEQ ID NO: 66;
(g) a heavy-chain variable region including or consisting of an amino acid sequence shown in SEQ ID NO: 48 or 79 and a light-chain variable region including or consisting of an amino acid sequence shown in SEQ ID NO: 68;
(h) a heavy-chain variable region including or consisting of any one of amino acid sequences shown in SEQ ID NOs: 49-52 and a light-chain variable region including or consisting of an amino acid sequence shown in SEQ ID NO: 69;
(i) a heavy-chain variable region including or consisting of any one of the amino acid sequences shown in SEQ ID NOs: 49-52 and a light-chain variable region including or consisting of an amino acid sequence shown in SEQ ID NO: 70;
(j) a heavy-chain variable region including or consisting of an amino acid sequence shown in SEQ ID NO: 53 or 54 and a light-chain variable region including or consisting of an amino acid sequence shown in SEQ ID NO: 71;
(k) a heavy-chain variable region including or consisting of any one of amino acid sequences shown in SEQ ID NOs: 53-56 and a light-chain variable region including or consisting of an amino acid sequence shown in SEQ ID NO: 72; or
(l) a heavy-chain variable region including or consisting of any one of amino acid sequences shown in SEQ ID NOs: 57-60 and a light-chain variable region including or consisting of an amino acid sequence shown in SEQ ID NO: 73.

In some embodiments, the isolated anti-CLDN6 antibody or the antigen-binding fragment thereof has one or more of the following properties:
(a) binding to human or monkey CLDN6 with EC50 of 10 µg/mL or less;
(b) cross-reactivity: demonstrating a lower binding property to human CLDN3 and/or human CLDN4 and/or human CLDN9 than to CLDN6; and
(c) killing and/or inhibiting a cell expressing the human CLDN6 (including a tumor cell, such as a CLDN6-expressing tumor cell).

In some embodiments, the binding property of the isolated anti-CLDN6 antibody or the antigen-binding fragment thereof reflected by EC50 described in the (a) can be as follows: the isolated anti-CLDN6 antibody or the antigen-binding fragment thereof binds to human CLDN6 or monkey CLDN6 with EC50 of 5 µg/mL or less; binds to human CLDN6 or monkey CLDN6 with EC50 of 3 µg/mL or less; binds to human CLDN6 or monkey CLDN6 with EC50 of 2 µg/mL or less; binds to human CLDN6 or monkey CLDN6 with EC50 of 1 µg/mL or less; binds to human CLDN6 or monkey CLDN6 with EC50 of 0.5 µg/mL or less; binds to human CLDN6 or monkey CLDN6 with EC50 of 0.3 µg/mL or less; or binds to human CLDN6 or monkey CLDN6 with EC50 of 0.1 µg/mL or less.

In some embodiments, the cross-reactivity of the isolated anti-CLDN6 antibody or the antigen-binding fragment thereof described in the (b) can be as follows: the isolated anti-CLDN6 antibody or the antigen-binding fragment thereof does not bind or binds negligibly to CLDNs other than CLDN6; and/or the isolated anti-CLDN6 antibody or the antigen-binding fragment thereof does not bind or binds negligibly to CLDN3; and/or the isolated anti-CLDN6 antibody or the antigen-binding fragment thereof does not bind or binds negligibly to CLDN9; and/or the isolated anti-CLDN6 antibody or the antigen-binding fragment thereof does not bind or binds negligibly to CLDN4.

In some embodiments of the present disclosure, the killing and/or inhibiting a cell expressing the human CLDN6 (including a tumor cell, such as a CLDN6-expressing tumor cell) by the isolated anti-CLDN6 antibody or the antigen-binding fragment thereof described in the (c) may be as follows: the isolated anti-CLDN6 antibody or the antigen-binding fragment thereof induces killing and/or inhibition of the cell expressing the human CLDN6 (including a tumor cell, such as a CLDN6-expressing tumor cell) through antibody-dependent cell-mediated cytotoxicity (ADCC); and/or the isolated anti-CLDN6 antibody or the antigen-binding fragment thereof induces killing of the cell expressing the human CLDN6 (including a tumor cell, such as a CLDN6-expressing tumor cell) through complement-dependent cytotoxicity (CDC).

In an aspect, the present disclosure provides an isolated anti-CLDN6 antibody or an antigen-binding fragment thereof that competitively binds to CLDN6 expressed on a surface of a cell with the isolated anti-CLDN6 antibody or the antigen-binding fragment thereof described above.

In an aspect, the present disclosure provides an isolated anti-CLDN6 antibody or an antigen-binding fragment thereof, including complementarity determining region (CDR) sequences selected from a group consisting of sequences shown in SEQ ID NOs: 41-46, 48-60, and 79-83 and CDR sequences selected from a group consisting of sequences shown in SEQ ID NOs: 61-66 and 68-73.

In another aspect, the present disclosure provides an isolated anti-CLDN6 antibody or an antigen-binding fragment thereof, including a heavy-chain variable region and a light-chain variable region,
where the isolated anti-CLDN6 antibody includes:
   (1) CDRH1 including or consisting of a sequence shown in SEQ ID NO: 5;
   (2) CDRH2 including or consisting of a sequence shown in SEQ ID NO: 12, or including or consisting of a sequence YX₁NPNNGAX₂X₃YNQKFKG, where X₁ is selected from a group consisting of A, I, L, and V, X₂ is selected from a group consisting of S and T, and X₃ is selected from a group consisting of S and N; and preferably, CDRH2 including or consisting of a sequence shown in SEQ ID NO: 12, 91, 92, 93, 94, 119, 120, 121, 122, 123, 124, or 125;
   (3) CDRH3 including or consisting of a sequence shown in SEQ ID NO: 20, or including or consisting of a sequence WDGYX₁YYYGX₂DX₃, where X₁ is selected from a group consisting of V and S, X₂ is selected from a group consisting of V, L, and M, and X₃ is selected from a group consisting of Y and F; and preferably, CDRH3 including or consisting of a sequence shown in SEQ ID NO: 20, 95, 96, 97, 126, 127, 128, 129, 130, or 131;
   (4) CDRL1 including or consisting of a sequence shown in SEQ ID NO: 27, or including or consisting of a sequence KASX₁HX₂NX₃WLA, where X₁ is selected from a group consisting of E and D, X₂ is selected from a group consisting of I and V, and X₃ is selected from a group consisting of H and Q; and preferably, CDRL1 including or consisting of a sequence shown in SEQ ID NO: 27, 98, 99, 100, 132, 133, 134, or 135;
   (5) CDRL2 including or consisting of a sequence shown in SEQ ID NO: 31, or including or consisting of a sequence shown in SEQ ID NO: 101; and
   (6) CDRL3 including or consisting of a sequence shown in SEQ ID NO: 37, or including or consisting of a sequence QQYX₁X₂TPYX₃, where X₁ is selected from a group consisting of W and G, X₂ is selected from a group consisting of S, H, and N, and X₃ is selected from a group consisting of S and T; and preferably, CDRL3 including or consisting of a sequence shown in SEQ ID NO: 37, 102, 103, 104, 136, 137, 138, 139, 140, 141, or 142;
preferably, the isolated anti-CLDN6 antibody includes:
   (1) CDRH1 including or consisting of the sequence shown in SEQ ID NO: 5;
   (2) CDRH2 including or consisting of the sequence shown in SEQ ID NO: 12, or including or consisting of the sequence YX₁NPNNGAX₂X₃YNQKFKG, where X₁ is selected from a group consisting of A, I, L, and V, X₂ is selected from a group consisting of S and T, and X₃ is selected from a group consisting of S and N; and preferably, CDRH2 including or consisting of the sequence shown in SEQ ID NO: 12, 91, 92, 93, 94, 119, 120, 121, 122, 123, 124, or 125;
   (3) CDRH3 including or consisting of the sequence shown in SEQ ID NO: 20;
   (4) CDRL1 including or consisting of the sequence shown in SEQ ID NO: 27;
   (5) CDRL2 including or consisting of the sequence shown in SEQ ID NO: 31; and
   (6) CDRL3 including or consisting of the sequence shown in SEQ ID NO: 37; or
the isolated anti-CLDN6 antibody includes:
   (1) CDRH1 including or consisting of the sequence shown in SEQ ID NO: 5;
   (2) CDRH2 including or consisting of the sequence shown in SEQ ID NO: 12;
   (3) CDRH3 including or consisting of the sequence shown in SEQ ID NO: 20, or including or consisting of the sequence WDGYX₁YYYGX₂DX₃, where X₁ is selected from a group consisting of V and S, X₂ is selected from a group consisting of V, L, and M, and X₃ is selected from a group consisting of Y and F; and preferably, CDRH3 including or consisting of the sequence shown in SEQ ID NO: 20, 95, 96, 97, 126, 127, 128, 129, 130, or 131; (4) CDRL1 including or consisting of the sequence shown in SEQ ID NO: 27;
   (5) CDRL2 including or consisting of the sequence shown in SEQ ID NO: 31; and
   (6) CDRL3 including or consisting of the sequence shown in SEQ ID NO: 37; or
the isolated anti-CLDN6 antibody includes:
   (1) CDRH1 including or consisting of the sequence shown in SEQ ID NO: 5;
   (2) CDRH2 including or consisting of the sequence shown in SEQ ID NO: 12;
   (3) CDRH3 including or consisting of the sequence shown in SEQ ID NO: 20;
   (4) CDRL1 including or consisting of the sequence shown in SEQ ID NO: 27, or including or consisting of the sequence KASX₁HX₂NX₃WLA, where X₁ is selected from a group consisting of E and D, X₂ is selected from a group consisting of I and V, and X₃ is selected from a group consisting of H and Q; and preferably, CDRL1 including or consisting of the sequence shown in SEQ ID NO: 27, 98, 99, 100, 132, 133, 134, or 135;
   (5) CDRL2 including or consisting of the sequence shown in SEQ ID NO: 31; and
   (6) CDRL3 including or consisting of the sequence shown in SEQ ID NO: 37; or
the isolated anti-CLDN6 antibody includes:
   (1) CDRH1 including or consisting of the sequence shown in SEQ ID NO: 5;
   (2) CDRH2 including or consisting of the sequence shown in SEQ ID NO: 12;
   (3) CDRH3 including or consisting of the sequence shown in SEQ ID NO: 20;
   (4) CDRL1 including or consisting of the sequence shown in SEQ ID NO: 27;
   (5) CDRL2 including or consisting of the sequence shown in SEQ ID NO: 31, or including or consisting of the sequence shown in SEQ ID NO: 101; and
   (6) CDRL3 including or consisting of the sequence shown in SEQ ID NO: 37; or
the isolated anti-CLDN6 antibody includes:
   (1) CDRH1 including or consisting of the sequence shown in SEQ ID NO: 5;
   (2) CDRH2 including or consisting of the sequence shown in SEQ ID NO: 12;
   (3) CDRH3 including or consisting of the sequence shown in SEQ ID NO: 20;
   (4) CDRL1 including or consisting of the sequence shown in SEQ ID NO: 27;
   (5) CDRL2 including or consisting of the sequence shown in SEQ ID NO: 31; and
   (6) CDRL3 including or consisting of the sequence shown in SEQ ID NO: 37, or including or consisting of the sequence QQYX₁X₂TPYX₃, where X₁ is selected from a group consisting of W and G, X₂ is selected from a group consisting of S, H, and N, and X₃ is selected from a group consisting of S and T; and preferably, CDRL3 including or consisting of the sequence shown in SEQ ID NO: 37, 102, 103, 104, 136, 137, 138, 139, 140, 141, or 142; or
the isolated anti-CLDN6 antibody includes:
   (1) CDRH1 including or consisting of the sequence shown in SEQ ID NO: 5;
   (2) CDRH2 including or consisting of any one of sequences shown in SEQ ID NOs: 12, 91-94, and 119-125;
   (3) CDRH3 including or consisting of any one of sequences shown in SEQ ID NOs: 20, 95-97, and 126-131;
   (4) CDRL1 including or consisting of any one of sequences shown in SEQ ID NOs: 27, 98-99, and 132-135;
   (5) CDRL2 including or consisting of a sequence shown in SEQ ID NO: 31 or 101; and
   (6) CDRL3 including or consisting of any one of sequences shown in SEQ ID NOs: 37, 102-104, and 136-142.

Preferably, the isolated anti-CLDN6 antibody includes or consists of CDRs shown in Table I and/or Table J. For example, the isolated anti-CLDN6 antibody includes or consists of CDRH1, CDRH2, and CDRH3 from Table I and CDRL1, CDRL2, and CDRL3 from Table J.

Preferably, the isolated anti-CLDN6 antibody includes or consists of CDRs shown in Table G.

Preferably, the isolated anti-CLDN6 antibody includes or consists of CDRs shown in Table K.

The present disclosure further discloses an isolated anti-CLDN6 antibody or an antigen-binding fragment thereof, including a heavy-chain variable region and a light-chain variable region,
where the heavy-chain variable region includes or consists of any one of amino acid sequences shown in SEQ ID NOs: 105-111, or includes or consists of a sequence having a sequence identity of at least 80% including at least 85%, 90%, 91%, 92%, 93%, 94%, 95%, 96%, 97%, 98%, or 99% with any one of the amino acid sequences shown in SEQ ID NOs: 105-111; and the light-chain variable region includes or consists of an amino acid sequence shown in SEQ ID NO: 69, or includes or consists of a sequence having a sequence identity of at least 85% including at least 90%, 91%, 92%, 93%, 94%, 95%, 96%, 97%, 98%, or 99% with the amino acid sequence shown in SEQ ID NO: 69; or
the heavy-chain variable region includes or consists of an amino acid sequence shown in SEQ ID NO: 52, or includes or consists of a sequence having a sequence identity of at least 80% including at least 85%, 90%, 91%, 92%, 93%, 94%, 95%, 96%, 97%, 98%, or 99% with the amino acid sequence shown in SEQ ID NO: 52; and the light-chain variable region includes or consists of any one of amino acid sequences shown in SEQ ID NOs: 112-118, or includes or consists of a sequence having a sequence identity of at least 80% including at least 85%, 90%, 91%, 92%, 93%, 94%, 95%, 96%, 97%, 98%, or 99% with any one of the amino acid sequences shown in SEQ ID NOs: 112-118.

In some embodiments, the isolated anti-CLDN6 antibody is a monoclonal antibody.

In some embodiments, the isolated anti-CLDN6 antibody is a chimeric antibody or a humanized antibody.

In some embodiments, the isolated anti-CLDN6 antibody is an improved antibody, such as an improved chimeric monoclonal antibody, or an antibody constructed through an amino acid substitution.

In some embodiments, the isolated anti-CLDN6 antibody or the antigen-binding fragment thereof includes a constant region of immunoglobulin G (IgG), such as a constant region of IgG1, IgG2, IgG3, or IgG4.

In a specific embodiment, the constant region of IgG is a constant region of IgG1.

In some embodiments, the isolated anti-CLDN6 antibody or the antigen-binding fragment thereof includes the constant region of IgG1.

In some embodiments, the isolated anti-CLDN6 antibody or the antigen-binding fragment thereof includes a constant region of a κ or λ light chain.

In some embodiments, the isolated anti-CLDN6 antibody or the antigen-binding fragment thereof includes: a heavy chain including or consisting of any one selected from a group consisting of polypeptides having a sequence identity of at least 90%, 91%, 92%, 93%, 94%, 95%, 96%, 97%, 98%, 99%, or 100% with a sequence shown in any of SEQ ID NOs: 143-167 or SEQ ID NO: 87 or SEQ ID NO: 89; and a light chain including or consisting of any one selected from a group consisting of polypeptides having a sequence identity of at least 90%, 91%, 92%, 93%, 94%, 95%, 96%, 97%, 98%, 99%, or 100% with a sequence shown in any of SEQ ID NOs: 168-178 or SEQ ID NO: 88 or SEQ ID NO: 90.

In an aspect, the present disclosure provides an isolated nucleic acid including a nucleic acid sequence encoding the isolated anti-CLDN6 antibody or the antigen-binding fragment thereof described above.

In an aspect, the present disclosure provides a vector including the nucleic acid described above.

In an aspect, the present disclosure provides a host cell including the nucleic acid described above or the vector described above.

In an aspect, the present disclosure provides a conjugate including the isolated anti-CLDN6 antibody or the antigen-binding fragment thereof described above that is conjugated to at least one detectable label.

In an aspect, the present disclosure provides an antibody-drug conjugate including an antibody, where the antibody-drug conjugate includes one or more drug moieties, and the one or more drug moieties are linked to the isolated anti-CLDN6 antibody or the antigen-binding fragment thereof described above directly or indirectly through a linker (for example, preferably covalent linking).

In an aspect, the present disclosure provides a multispecific molecule including the isolated anti-CLDN6 antibody or the antigen-binding fragment thereof described above.

In some embodiments, the multispecific molecule specifically binds to CLDN6 and additionally specifically binds to one or more other targets.

In some embodiments, the multispecific molecule further includes at least one molecule (a second antibody) with second binding specificity for a second target.

In some embodiments, the multispecific molecule is a bispecific antibody.

In an aspect, the present disclosure provides a chimeric antigen receptor (CAR), including the isolated anti-CLDN6 antibody or the antigen-binding fragment thereof described above and an immune cell activation moiety, where the immune cell activation moiety includes a transmembrane domain of an immune cell receptor and/or an intracellular signaling domain of the immune cell receptor.

In an aspect, the present disclosure provides a targeted drug conjugate including the isolated anti-CLDN6 antibody or the antigen-binding fragment thereof described above, where the targeted drug conjugate is a radionuclide drug conjugate (RDC), and the RDC can be used for diagnosing or treating a disease.

In an aspect, the present disclosure provides an RNA drug including the isolated anti-CLDN6 antibody or the antigen-binding fragment thereof described above, where the RNA drug is a nucleic acid-drug conjugate, and the nucleic acid-drug conjugate can be used for diagnosing or treating a disease. The nucleic acid-drug conjugate is produced by conjugating RNA to the isolated anti-CLDN6 antibody or the antigen-binding fragment thereof.

In an aspect, the present disclosure provides a targeted protein degrader-drug conjugate including the anti-CLDN6 antibody or the antigen-binding fragment thereof described above, where the targeted protein degrader-drug conjugate is produced by conjugating a protein degrader to the anti-CLDN6 antibody or the antigen-binding fragment thereof through, for example, a chemical bond.

In another aspect, the present disclosure provides a pharmaceutical composition or kit, including the isolated anti-CLDN6 antibody or the antigen-binding fragment thereof described above, the nucleic acid described above, the vector described above, the host cell described above, the conjugate described above, the antibody-drug conjugate described above, the multispecific molecule described above, or the CAR described above; and a pharmaceutically acceptable carrier.

In another aspect, the present disclosure provides a method for preparing the isolated anti-CLDN6 antibody or the antigen-binding fragment thereof described above, including the following steps:
expressing the isolated anti-CLDN6 antibody or the antigen-binding fragment thereof described above in the host cell described above; and optionally (ii) isolating the isolated anti-CLDN6 antibody or the antigen-binding fragment thereof from the host cell.

In yet another aspect, the present disclosure provides a method for detecting CLDN6 in a sample or determining a content of the CLDN6 in the sample, including the following steps:
allowing the sample to be in contact with the isolated anti-CLDN6 antibody or the antigen-binding fragment thereof described above or the conjugate described above; and
detecting formation of a complex of the isolated anti-CLDN6 antibody, the antigen-binding fragment thereof, or the conjugate with the CLDN6, or determining an amount of the complex.

In an embodiment, the sample includes a cell.

In yet another aspect, the present disclosure provides a method for diagnosing, detecting, or monitoring a disease associated with expression of CLDN6 or binding of CLDN6 to a surface of a cell, including: administering an effective dose of the isolated anti-CLDN6 antibody or the antigen-binding fragment thereof described above, the nucleic acid described above, the vector described above, the host cell described above, the conjugate described above, the antibody-drug conjugate described above, the multispecific molecule described above, the CAR described above, or the pharmaceutical composition or kit described above to a subject in need.

In yet another aspect, the present disclosure provides a use of the isolated anti-CLDN6 antibody or the antigen-binding fragment thereof described above, the nucleic acid described above, the vector described above, the host cell described above, the conjugate described above, the antibody-drug conjugate described above, the multispecific molecule described above, the CAR described above, or the pharmaceutical composition or kit described above for diagnosing, detecting, or monitoring a disease associated with expression of CLDN6 or binding of CLDN6 to a surface of a cell in a subject.

In yet another aspect, the present disclosure provides a use of the isolated anti-CLDN6 antibody or the antigen-binding fragment thereof described above, the nucleic acid described above, the vector described above, the host cell described above, the conjugate described above, the antibody-drug conjugate described above, the multispecific molecule described above, the CAR described above, or the pharmaceutical composition or kit described above in preparation of a reagent for diagnosing, detecting, or monitoring a disease associated with expression of CLDN6 or binding of CLDN6 to a surface of a cell.

In yet another aspect, the present disclosure provides a method for treating a disease associated with expression of CLDN6 or binding of CLDN6 to a surface of a cell or determining prognosis of the disease in a subject, including: administering an effective dose of the isolated anti-CLDN6 antibody or the antigen-binding fragment thereof described above, the nucleic acid described above, the vector described above, the host cell described above, the conjugate described above, the antibody-drug conjugate described above, the multispecific molecule described above, the CAR described above, or the pharmaceutical composition or kit described above to the subject in need.

In yet another aspect, the present disclosure provides a use of the isolated anti-CLDN6 antibody or the antigen-binding fragment thereof described above, the nucleic acid described above, the vector described above, the host cell described above, the conjugate described above, the antibody-drug conjugate described above, the multispecific molecule described above, the CAR described above, or the pharmaceutical composition or kit described above for treating a disease associated with expression of CLDN6 or binding of CLDN6 to a surface of a cell or determining prognosis of the disease in a subject.

In yet another aspect, the present disclosure provides a use of the isolated anti-CLDN6 antibody or the antigen-binding fragment thereof described above, the nucleic acid described above, the vector described above, the host cell described above, the conjugate described above, the antibody-drug conjugate described above, the multispecific molecule described above, the CAR described above, or the pharmaceutical composition or kit described above in preparation of a reagent for treating a disease associated with CLDN6 expression or for determining prognosis of the disease.

In some embodiments, the disease associated with expression of CLDN6 or binding of CLDN6 to a surface of a cell is a cancer, and the cancer is preferably selected from a group consisting of ovarian cancer, lung cancer, gastric cancer, breast cancer, liver cancer, pancreatic cancer, skin cancer, malignant melanoma, head and neck cancer, sarcoma, cholangiocarcinoma, bladder cancer, kidney cancer, colon cancer, placental choriocarcinoma, cervical cancer, testicular cancer, and uterine cancer.

In some embodiments, the subject is a mammal and preferably a human.

### BRIEF DESCRIPTION OF THE DRAWINGS

FIG. 1 shows binding affinities of chimeric antibodies to HEK293T-CLDN6;
FIG. 2 shows a binding affinity of 20A3-2A7 to HEK293T-CLDN6;
FIG. 3 shows a binding affinity of an improved chimeric monoclonal antibody RecA101 to HEK293T-CLDN6;
FIG. 4 shows a binding affinity of a humanized antibody engineered based on 51C10H4 to HEK293T-CLDN6;
FIG. 5 shows a binding affinity of a humanized antibody engineered based on 54B5A7 to HEK293T-CLDN6;
FIG. 6 shows binding affinities of chimeric antibodies to HEK293T-CLDN9;
FIG. 7 shows a binding affinity of 20A3-2A7 to HEK293T-CLDN9;
FIG. 8 shows a binding affinity of an improved chimeric monoclonal antibody RecA101 to HEK293T-CLDN9;
FIG. 9 shows binding affinities of chimeric antibodies to HEK293T-CLDN3;
FIG. 10 shows a binding affinity of 20A3-2A7 to HEK293T-CLDN3;
FIG. 11 shows binding affinities of chimeric antibodies to HEK293T-CLDN4;
FIG. 12 shows a binding affinity of 20A3-2A7 to HEK293T-CLDN4;
FIG. 13 shows binding results of 51C10H4 and 54B5A7 chimeric antibodies at a concentration of 5 µg/mL to cell lines HEK293T-CLDN6, HEK293T-CLDN9, HEK293T-CLDN3, and HEK293T-CLDN4;
FIG. 14 shows binding results of chimeric antibodies to CHO-K1-mouse CLDN6 cells;
FIG. 15 shows binding results of 20A3-2A7 to CHO-K1-mouse CLDN6 cells;
FIG. 16 shows binding results of chimeric antibodies to CHO-K1-Cyno CLDN6 cells;
FIG. 17 shows binding results of 20A3-2A7 to CHO-K1-Cyno CLDN6 cells;
FIG. 18 shows binding of a humanized antibody engineered based on 51C10H4 to cells overexpressing mouse CLDN6;
FIG. 19 shows binding of a humanized antibody engineered based on 51C10H4 to cells overexpressing monkey CLDN6;
FIG. 20 shows binding of a humanized antibody engineered based on 54B5A7 to cells overexpressing mouse CLDN6;
FIG. 21 shows binding of a humanized antibody engineered based on 54B5A7 to cells overexpressing monkey CLDN6;
FIG. 22 shows binding affinities of chimeric antibodies to HepG2;
FIG. 23 shows a binding affinity of 20A3-2A7 to HepG2;
FIG. 24 shows a binding affinity of an improved chimeric monoclonal antibody RecA101 to HepG2;
FIG. 25 shows a binding affinity of a humanized antibody engineered based on 51C10H4 to HepG2;
FIG. 26 shows a binding affinity of a humanized antibody engineered based on 54B5A7 to HepG2;
FIG. 27 shows a binding affinity of a second humanized antibody engineered based on 54B5A7 to HepG2;
FIG. 28 shows binding affinities of chimeric antibodies to PA-1;
FIG. 29 shows a binding affinity of 20A3-2A7 to PA-1;
FIG. 30 shows a binding affinity of a humanized antibody engineered based on 51C10H4 to PA-1;
FIG. 31 shows a binding affinity of a humanized antibody engineered based on 54B5A7 to PA-1;
FIG. 32 shows a binding affinity of an improved antibody 20A3-M1 to a human lung adenocarcinoma cell line H1975;
FIG. 33 shows a binding affinity of an improved antibody 20A3-M1 to a human colon adenocarcinoma cell line LS174T;
FIG. 34 shows a binding affinity of an improved antibody 20A3-M1 to a human ovarian cancer cell line OVCAR3;
FIG. 35 shows a binding affinity of an improved antibody 20A3-M1 to a human prostate cancer cell line PC-3;
FIG. 36 shows a binding affinity of an improved antibody 20A3-M1 to a human pancreatic cancer cell line Capan2;
FIG. 37 shows a binding affinity of an improved antibody 20A3-M1 to HepG2;
FIG. 38 shows a binding affinity of an improved antibody 20A3-M1 to PA-1;
FIG. 39 shows ADCC;
FIG. 40 shows CDC;
FIG. 41 shows results of an endocytosis test;
FIG. 42 shows binding affinities of improved antibodies to CHO-K1-CLDN6;
FIG. 43 shows binding affinities of antibodies PiH2012, PiH1004, AB3-7, and IgG1 to a human ovarian cancer cell line OVCA429;
FIG. 44 shows binding affinities of antibodies PiH2012, PiH1004, AB3-7, and IgG1 to a human gastric cancer cell line MKN7;
FIG. 45 shows changes in body weights of mice after being administered with an anti-CLDN6 antibody;
FIG. 46 shows changes in jaundice rates of mice after being administered with an anti-CLDN6 antibody;
FIG. 47 shows expression of a gene *Abcd1* in mice after being administered with an anti-CLDN6 antibody;
FIG. 48 shows expression of a gene *II13* in mice after being administered with an anti-CLDN6 antibody;
FIG. 49 shows expression of a gene *II33* in mice after being administered with an anti-CLDN6 antibody;
FIG. 50 shows expression of a gene *Occludin* in mice after being administered with an anti-CLDN6 antibody;
FIG. 51 shows expression of a gene *Zo1* in mice after being administered with an anti-CLDN6 antibody;
FIG. 52 shows expression of a gene *Mmp7* in mice after being administered with an anti-CLDN6 antibody;
FIG. 53 shows expression of a gene *Col1a1* in mice after being administered with an anti-CLDN6 antibody;
FIG. 54 shows expression of a gene *Col1a2* in mice after being administered with an anti-CLDN6 antibody;
FIG. 55 shows expression of a gene *Col3a1* in mice after being administered with an anti-CLDN6 antibody;
FIG. 56 shows expression of a gene *α-SMA* in mice after being administered with an anti-CLDN6 antibody; and
FIG. 57 shows tissue sections of mice after being administered with an anti-CLDN6 antibody.

### DETAILED DESCRIPTION OF THE EMBODIMENTS

To enable those skilled in the art to well understand the technical solutions in the present disclosure, the technical solutions in the embodiments of the present disclosure will be clearly and completely described below. Obviously, the described embodiments are merely some rather than all of the embodiments of the present disclosure. The specific exemplary embodiments disclosed herein are intended to verify the principles of the present disclosure. It should be emphasized that the present disclosure is not limited to the specific embodiments illustrated. Further, any section heading used herein should not be construed as limiting the described subject matter.

Unless otherwise specified, all scientific and technical terms used in the present disclosure have the meanings commonly understood by those of ordinary skill in the art. Moreover, unless otherwise indicated clearly in the context, a term mentioned in a singular form shall include a plural form of the term, and a term mentioned in a plural form shall include a singular form of the term. More specifically, in this specification and the appended claims, unless otherwise indicated clearly in the context, singular forms such as "a", "an", and "the" include plural referents. In the present disclosure, unless otherwise specified, the use of "or" means "and/or". Further, the use of the term "comprising" and other forms (such as "including" and "containing") is not restrictive. Further, any range mentioned in the specification and the appended claims includes endpoints and all values between the endpoints.

### Definitions

To facilitate the comprehension of the present disclosure, definitions and explanations of the relevant terms are provided as follows.

As used herein, the term "antibody" or "Ab" generally refers to a Y-shaped tetrameric protein including two heavy (H) chains and two light (L) chains held together by covalent disulfide bonds and non-covalent interactions. A light chain of an antibody can be a κ or λ light chain. A heavy chain of an antibody can be a µ, δ, γ, α, or ε heavy chain, which define an antibody isotype as IgM, IgD, IgG, IgA, or IgE, respectively. In light and heavy chains, a variable region is linked to a constant region through a "J" region of approximately 12 or more amino acids. A heavy chain additionally includes a "D" region of about 3 or more amino acids. Each heavy chain includes a heavy-chain variable region (VH) and a heavy-chain constant region (CH). The heavy-chain constant region includes three domains (CH1, CH2, and CH3). Each light chain includes a light-chain variable region (VL) and a light-chain constant region (CL). VH and VL regions can each be further divided into hypervariable regions (known as CDRs) that are spaced with relatively conserved regions (framework regions (FRs)). Each VH or VL includes three CDRs and four FRs, which are arranged sequentially from an N terminus to a C terminus as follows: FR1, CDR1, FR2, CDR2, FR3, CDR3, and FR4. Variable regions (VH and VL) of each heavy chain/light chain pair collectively constitute an antigen-binding site/fragment. The distribution of amino acids across various regions or domains follows numbering definitions in common systems such as Kabat, IMGT, or Chothia. In a specific embodiment of the present disclosure, the determination of CDR sequences is based on numbering definitions in the Kabat system. There can be different antibody isotypes, for example, an IgG (such as subtype IgG1, IgG2, IgG3, or IgG4), IgA1, IgA2, IgD, IgE, or IgM antibody. The antibody in the present disclosure also includes an antigen-binding fragment. The antigen-binding fragment refers to a polypeptide including a portion/fragment of a full-length antibody, which retains the ability to specifically bind to an antigen specifically recognized by the full-length antibody, and/or competitively binds to a same antigen with the full-length antibody. Under some conditions, the antigen-binding fragment includes Fab, Fab', F(ab')2, Fd, Fv, dAb, and CDR fragments, a single-chain antibody (such as scFv), a chimeric antibody, a double antibody, and a polypeptide including at least a portion of the antibody sufficient to confer a specific antigen-binding capability to the polypeptide. The antigen-binding fragment of the antibody can be obtained from a given antibody by conventional techniques known to those skilled in the art (such as recombinant DNA technology or enzymatic or chemical cleavage method), and can be screened for specificity in the same manner as the full-length antibody.

As used herein, the term "isotype" refers to an antibody type (such as IgM or IgG1) encoded by a gene of a heavy-chain constant region.

As used herein, the term "monoclonal antibody" or "mAb" refers to an antibody molecule/preparation composed of a single type of molecules. The monoclonal antibody exhibits single binding specificity and affinity for a particular epitope. The antibody of the present disclosure may be derived from various species, including, but not limited to, a mouse, a rat, a rabbit, a guinea pig, and a human.

As used herein, the term "epitope" refers to an antigenic determinant in a molecule, which is a portion in the molecule that is recognized by an immune system (for example, by an antibody), such as a discontinuous three-dimensional site on an antigen that is recognized by the immune system. In the present disclosure, the epitope is derived from preferably CLDN and more preferably CLDN6.

As used herein, the term "chimeric antibody" refers to an antibody in which variable region sequences are derived from a species and constant region sequences are derived from another species, such as an antibody in which variable region sequences are derived from a mouse antibody and constant region sequences are derived from a human antibody.

The term "humanized antibody" refers to an antibody in which a CDR sequence/antigen-binding fragment or site derived from another mammalian species such as a mouse has been grafted onto a human framework sequence. An additional modification can also be made within the human framework sequence.

As used herein, the term "CLDN" refers to claudin, including CLDN6, CLDN9, CLDN4, and CLDN3, and preferably refers to human CLDN.

As used herein, the term "anti-CLDN6 antibody" or "CLDN6 antibody" refers to an antibody as defined herein that can bind to CLDN6 or to a cell expressing CLDN6.

As used herein, the term "specific binding" refers to a non-random binding reaction between two molecules, such as a reaction between an antibody and an antigen targeted thereby. In some embodiments, an antibody that specifically binds to an antigen (or an antibody specific for an antigen) refers to an antibody that binds to the antigen with a KD value of less than approximately 10⁻⁵ M, for example, less than approximately 10⁻⁶ M, 10⁻⁷ M, 10⁻⁸ M, 10⁻⁹ M, 10⁻¹⁰ M, or lower.

As used herein, the term "isolated" refers to a state acquired from a natural state through an artificial means. If an "isolated" substance or component occurs naturally, it may be due to a change in a natural environment of the substance or component, or the separation of the substance or component from the natural environment, or both. For example, if a non-isolated polynucleotide or polypeptide naturally exists in a living organism, the same polynucleotide or polypeptide with a high purity isolated form the natural state is referred to as an isolated polynucleotide or polypeptide. The term "isolated" does not preclude the presence of admixed artificial or synthetic substances, and also does not preclude the presence of other impurities that do not affect an activity of the isolated substance.

As used herein, the term "isolated antibody" refers to an antibody that is substantially free of other antibodies having different antigenic specificities (for example, an isolated antibody that specifically binds to CLDN6 is substantially free of antibodies that specifically bind to antigens other than CLDN6). However, an isolated antibody that specifically binds to human CLDN6 may exhibit cross-reactivity with other antigens, such as CLDN6 from other species, or other members of the CLDN family. Further, the isolated antibody may be substantially free of other cellular materials and/or chemical substances.

As used herein, the term "vector" refers to a nucleic acid vehicle into which a polynucleotide can be inserted. When the vector allows the expression of a protein encoded by the polynucleotide inserted into the vector, the vector is referred to as an expression vector. The vector can be introduced into a host cell through transformation, transduction, or transfection, such that a genetic material element carried by the vector can be expressed in the host cell. The vector is well known to those skilled in the art, and includes, but is not limited to, a plasmid; a phage; a cosmid; an artificial chromosome, such as a yeast artificial chromosome (YAC), a bacterial artificial chromosome (BAC), or a P1-derived artificial chromosome (PAC); a phage, such as a λ phage or an M13 phage; and an animal virus. Animal viruses that can be used as vectors include, but are not limited to, a retrovirus (including a lentivirus), an adenovirus, an adeno-associated virus, a herpesvirus (such as herpes simplex virus), a poxvirus, a baculovirus, a papillomavirus, and a papovavirus (such as SV40). The vector may include a variety of elements to control expression, including, but not limited to, a promoter sequence, a transcription initiation sequence, an enhancer sequence, a selection element, and a reporter gene. Additionally, the vector may include an origin of replication.

As used herein, the term "host cell" refers to a cell system that can be engineered to produce a target protein, protein fragment, or peptide. The host cell includes, but is not limited to, a cultured cell, including a cultured mammalian cell derived from a rodent (a rat, a mouse, a guinea pig, or a hamster), such as CHO, BHK, NSO, SP2/0, and YB2/0; or a human tissue or hybridoma cell, a yeast cell, and an insect cell, and a cell in a transgenic animal or a cultured tissue. This term encompasses not only a specific subject cell but also a progeny of the specific subject cell. Since some modifications may occur in subsequent generations due to mutations or environmental impacts, such progenies may differ from a parental cell, but is still included within the scope of the term "host cell".

As used herein, the term "identity" refers to a relationship between sequences of two or more polypeptide molecules or two or more nucleic acid molecules that is determined by aligning and comparing the sequences. "Percent identity" refers to a percentage of identical amino acids or nucleotides in the compared molecules, and is calculated based on a size of the smallest molecule among the compared molecules. For such a calculation, a gap (if present) in the alignment is preferably addressed by a specific mathematical model or computer program (namely, "algorithm"). Methods that can be used to calculate an identity of aligned nucleic acids or polypeptides include those described in Computational Molecular Biology, (Lesk, A.M., ed.), 1988, New York: Oxford University Press; Biocomputing Informatics and Genome Projects, (Smith, D.W., ed.), 1993, New York: AcademicPress; Computer Analysis of Sequence Data, Part I, (Griffin, A.M., and Griffin, H.G., eds.), 1994, New Jersey: Humana Press; von Heinje, G., 1987, Sequence Analysisin Molecular Biology, New York: Academic Press; Sequence Analysis Primer, (Gribskov, M. and Devereux, J., eds.), 1991, New York: M. Stockton Press; and Carillo etal, 1988, SIAMJ. Applied Math.48:1073.

As used herein, the term "immunogenicity" refers to the ability to stimulate the generation of a specific antibody or a sensitized lymphocyte in an organism. The immunogenicity refers not only to the ability of an antigen to stimulate the activation, proliferation, and differentiation of a specific immune cell to ultimately produce an immune effector substance such as an antibody and a sensitized lymphocyte, but also to the fact that a specific immune response of an antibody or a sensitized T lymphocyte can be elicited within an immune system of an organism following an antigenic stimulation. The immunogenicity is the most critical property of an antigen. The ability of an antigen to successfully induce an immune response in a host depends on the following three factors: the nature of the antigen, the responsiveness of the host, and the approach of immunization.

As used herein, the term "transfection" refers to a process of introducing a nucleic acid into a eukaryotic cell, particularly a mammalian cell. Solutions and techniques for the transfection include, but are not limited to, lipofection, and chemical/physical methods such as electroporation. Many transfection techniques are well known in the art and are disclosed herein, see, for example, Graham et al., 1973, Virology 52: 456; Sambrook et al., 2001, Molecular Cloning: A Laboratory Manual, ibid.; Davis et al., 1986, Basic Methods in Molecular Biology, Elsevier; Chu et al., 1981, Gene 13: 197. In a specific embodiment of the present disclosure, a human XO40 gene is transfected into 293F cells.

As used herein, the terms "hybridoma" and "hybridoma cell line" can be used interchangeably. When the terms "hybridoma" and "hybridoma cell line" are mentioned, subclones and progeny cells of hybridomas are also included.

As used herein, unless otherwise specified, the term "expression" encompasses the production of DNA, RNA, or a protein/peptide, including transient or stable expression, and "aberrant expression" or "abnormal expression" or "partial expression".

For vectors expressing antibodies, a vector type in which heavy and light chains of an antibody are present in separate vectors or a vector type in which heavy and light chains of an antibody are present in a same vector may be adopted.

As used herein, the term "surface plasmon resonance" or "SPR" refers to and includes an optical phenomenon that allows the analysis of a real-time biospecific interaction by detecting a change in a protein concentration within a biosensor matrix, for example, using the BIAcore system (Pharmacia Biosensor AB, Uppsala, Sweden, and Piscataway, NJ).

As used herein, the term "fluorescence-activated cell sorting" or "FACS" refers to a specialized type of flow cytometry. The FACS provides a method for sorting a heterogeneous mixture of biological cells into two or more containers, with one cell at a time, based on the specific light scattering and fluorescent characteristics of each cell (FlowMetric. "Sorting Out Fluorescence Activated Cell Sorting". 2017-11-09). An instrument for FACS is known to those skilled in the art and is commercially available to the public. Examples of such an instrument include FACS Star Plus, FACScan, and FACSort from Becton Dickinson (Foster City, Calif.), Epics C from Coulter Epics Division (Hialeah, FLa), and MoFlo from Cytomation (Colorado Springs, Colo.).

As used herein, the term "immune effector function" encompasses any function mediated by components of an immune system, which results in the inhibition of tumor growth and/or tumorigenesis, including the inhibition of tumor dissemination and metastasis. Preferably, the immune effector function leads to the killing of tumor cells. Preferably, the immune effector function in the present disclosure is an antibody-mediated effector function. Such a function includes CDC, ADCC, induction of apoptosis in cells bearing a tumor-associated antigen (for example, through the binding of an antibody to a surface antigen), and/or inhibition of proliferation of cells bearing a tumor-associated antigen, and is preferably ADCC and/or CDC. The antibody can also function simply by binding to a tumor-associated antigen on a surface of a tumor cell. For example, the antibody may block a function of a tumor-associated antigen or induce apoptosis solely by binding to the tumor-associated antigen on a surface of a tumor cell.

As used herein, the term "antibody-dependent cell-mediated cytotoxicity" or "ADCC" refers to a cytotoxicity form in which the secreted Ig binding to an Fc receptor (FcR) on some cytotoxic cells (such as, natural killer (NK) cells, neutrophils, and macrophages) enables these cytotoxic effector cells to specifically bind to antigen-bearing target cells and subsequently kill the target cells with cytotoxins. The "arming" of the cytotoxic cells with an antibody is absolutely required for such killing. NK cells, the primary cells mediating ADCC, express only FcγRIII, while monocytes express FcγRI, FcγRII, and FcγRIII. The expression of FcR on hematopoietic cells is summarized in Table 3 on page 464 of Ravetch and Kinet, Annu. Rev. Immunol 9: 457-92 (1991). To assess an ADCC activity of a molecule of interest, an *in vitro* ADCC assay may be conducted, such as the assay described in the U.S. Patent No. US5,500,362 or US5,821,337. Effector cells that can be used in such an assay include peripheral blood mononuclear cells (PBMCs) and NK cells. Optionally or additionally, an ADCC activity of a molecule of interest may be assessed *in vivo,* for example, in the animal model disclosed in Clynes et al., PNAS (USA) 95: 652-656 (1998).

The term "complement-dependent cytotoxicity" or "CDC" refers to the lysis of a target cell in the presence of a complement. The activation of the classical complement pathway is initiated through the binding of a first component (C1q) of a complement system to an antibody (of an appropriate subtype) binding to a homologous antigen of the antibody. To evaluate the activation of a complement, a CDC assay may be conducted, as described in Gazzano-Santoro et al., J. Immunol. Methods 202: 163 (1996).

The term "subject" includes any mammal, such as a human or non-human animal, preferably a human.

As used herein, the term "cancer" refers to any tumor or malignant cell growth, proliferation, or metastasis-mediated solid tumors and non-solid tumors such as leukemia that cause medical conditions.

The terms "treatment" and "therapy" as used herein in the context of managing a medical condition generally refer to the care and management of a human or animal patient, where a desired therapeutic effect is achieved, such as inhibiting progression of the medical condition, including slowing the progression of the medical condition, halting the progression of the medical condition, regressing the medical condition, improving the medical condition, and curing the medical condition. The terms "treatment" and "therapy" also encompass a treatment as a preventive measure (namely, prophylaxis). The "treatment" for a cancer may refer to the inhibition or slowing of tumor or malignant cell growth, proliferation, or metastasis, or any combination thereof. The "treatment" for a tumor includes removal of all or part of the tumor, inhibition or slowing of tumor growth and metastasis, prevention or delay of tumor development, or any combination thereof.

As used herein, the term "therapeutically effective amount" refers to an amount of an active compound or a material, composition, or formulation including the active compound that is effective to produce a desired therapeutic effect commensurate with a reasonable benefit/risk ratio when administered according to a desired therapeutic plan. For example, when used in connection with treating or detecting a relevant disease or disorder, the term "effective amount" or "effective dose" refers to an amount or a concentration of the antibody or the antigen-binding fragment thereof that is effective in treating or detecting the disease or disorder.

As used herein, the term "prevention", "prophylaxis", or "preventing" in relation to a disease condition in a mammal refers to preventing or delaying the onset of the disease condition or preventing the manifestation of clinical or subclinical symptoms of the disease condition.

As used herein, the term "inhibition" means the ability to cause a reduction, such as a reduction of a cell proliferation level, including a reduction of 5% or more, 10% or more, 20% or more, 50% or more, or 75% or more, and also including complete or basically complete inhibition, namely, a reduction to 0 or substantially to 0.

As used herein, the term "pharmaceutically acceptable" means that a carrier, a diluent, an excipient, and/or a salt thereof is chemically and/or physically compatible with other ingredients in a formulation and is physiologically compatible with a recipient.

As used herein, the term "pharmaceutically acceptable carrier and/or excipient" refers to a carrier and/or excipient that is pharmacologically and/or physiologically compatible with a subject and an active agent, and is well known in the art (see, for example, Remington's Pharmaceutical Sciences. Edited by Gennaro AR, 19th ed. Pennsylvania: Mack Publishing Company, 1995). The pharmaceutically acceptable carrier and/or excipient includes, but is not limited to, a pH adjusting agent, a surfactant, an adjuvant, and an ionic strength enhancer. For example, the pH adjusting agent includes, but is not limited to, phosphate buffered saline (PBS). The surfactant includes, but is not limited to, a cationic, anionic, or non-ionic surfactant, such as Tween-80. The ionic strength enhancer includes, but is not limited to, sodium chloride.

As used herein, the term "adjuvant" refers to a non-specific immunopotentiator. When delivered together with an antigen or in advance to an organism, the adjuvant can enhance an immune response to the antigen or alter a type of the immune response in the organism. There is a variety of adjuvants, including, but not limited to, an aluminum adjuvant (such as aluminum hydroxide), Freund's adjuvant (such as Freund's complete adjuvant and Freund's incomplete adjuvant), *Corynebacterium parvum,* a lipopolysaccharide, and a cytokine. Freund's adjuvant is currently the most common adjuvant in animal experiments. Aluminum hydroxide is more likely to be used as an adjuvant in clinical trials.

### Anti-CLDN6 antibody

In some aspects, the present disclosure includes an isolated antibody or an antigen-binding fragment thereof.

In the context of the present disclosure, the "antibody" may include a polyclonal antibody, a monoclonal antibody, a chimeric antibody, humanized and primatized antibodies, a CDR-grafted antibody, a human antibody, a recombinant antibody, an intracellular antibody, a multispecific antibody, a bispecific antibody, a monovalent antibody, a multivalent antibody, an anti-idiotypic antibody, a synthetic antibody including a mutant protein and variant thereof, and an improved antibody; and a derivative thereof (including a Fc fusion protein and other modifications), and any other immunoreactive molecules, provided that the antibody exhibits preferential association with or binding to CLDN6. Further, unless otherwise indicated in the context, this term also encompasses all antibody types (namely, IgA, IgD, IgE, IgG, and IgM) and subtypes (namely, IgG1, IgG2, IgG3, IgG4, IgA1, and IgA2). In a preferred embodiment, the antibody is a monoclonal antibody. In a more preferred embodiment, the antibody is a chimeric monoclonal antibody, a humanized monoclonal antibody, or an improved chimeric monoclonal antibody.

In some embodiments, the antibody includes:
(1) CDRH 1 including or consisting of a sequence X₁YNMH, where X₁ is selected from a group consisting of D and S;
(2) CDRH2 including or consisting of a sequence YX₁X₂PX₃X₄X₅X₆TX₇YNQKFKG, where X₁ is selected from a group consisting of V and I, X₂ is selected from a group consisting of N and Y, X₃ is selected from a group consisting of N and G, X₄ is selected from a group consisting of N, Q, S, and A, X₅ is selected from a group consisting of A, G, D, Q, and S, X₆ is selected from a group consisting of A and G, and X₇ is selected from a group consisting of S and N;
(3) CDRH3 including or consisting of a sequence WX₁X₂YVYYYGX₃DY or a sequence shown in SEQ ID NO: 23, where X₁ is selected from a group consisting of D, E, S, and G, X₂ is selected from a group consisting of A, G, S, D, and Q, and X₃ is selected from a group consisting of L and M;
(4) CDRL1 including or consisting of a sequence shown in SEQ ID NO: 27 or a sequence shown in SEQ ID NO: 29;
(5) CDRL2 including or consisting of a sequence shown in SEQ ID NO: 30 or a sequence shown in SEQ ID NO: 31; and
(6) CDRL3 including or consisting of a sequence QQYWX₁TPYT or a sequence shown in SEQ ID NO: 40, where X₁ is selected from a group consisting of S and N.

Alternatively, the antibody includes:
(1) CDRH1 including or consisting of a sequence SYX₁IX₂, where X₁ is selected from a group consisting of N and G and X₂ is selected from a group consisting of H and S;
(2) CDRH2 including or consisting of a sequence X₁IYPX₂X₃GX₄TX₅X₆NEX₇FKG, where X₁ is selected from a group consisting of Y and E, X₂ is selected from a group consisting of G and R, X₃ is selected from a group consisting of N and S, X₄ is selected from a group consisting of G and N, X₅ is selected from a group consisting of K and Y, X₆ is selected from a group consisting of Y and H, and X₇ is selected from a group consisting of R and K;
(3) CDRH3 including or consisting of a sequence shown in SEQ ID NO: 16 or a sequence shown in SEQ ID NO: 19;
(4) CDRL1 including or consisting of a sequence shown in SEQ ID NO: 24 or a sequence shown in SEQ ID NO: 26;
(5) CDRL2 including or consisting of a sequence X₁X₂SNLX₃S, where X₁ is selected from a group consisting of L and G, X₂ is selected from a group consisting of A and T, and X₃ is selected from a group consisting of E and A; and
(6) CDRL3 including or consisting of a sequence shown in SEQ ID NO: 34 or a sequence shown in SEQ ID NO: 179.

Alternatively, the antibody includes:
(1) CDRH1 including or consisting of a sequence X₁YX₂MS, where X₁ is selected from a group consisting of S and N and X₂ is selected from a group consisting of G and A;
(2) CDRH2 including or consisting of a sequence TISX₁GGSYTX₂YPDX₃X₄KG, where X₁ is S or D, X₂ is selected from a group consisting of Y and S, X₃ is selected from a group consisting of S and N, and X₄ is selected from a group consisting of V and I;
(3) CDRH3 including or consisting of a sequence shown in SEQ ID NO: 17 or a sequence shown in SEQ ID NO: 18;
(4) CDRL1 including or consisting of a sequence shown in SEQ ID NO: 25;
(5) CDRL2 including or consisting of a sequence X₁X₂TSLET, where X₁ is selected from a group consisting of G and A and X₂ is selected from a group consisting of A and T; and
(6) CDRL3 including or consisting of a sequence QQYWS X₁PX₂T, where X₁ is selected from a group consisting of T and F and X₂ is selected from a group consisting of P and R.

In some embodiments, the antibody includes:
(1) CDRH1 including or consisting of a sequence shown in SEQ ID NO: 5;
(2) CDRH2 including or consisting of a sequence YX₁NPNX₂X₃ATX₄YNQKFKG, where X₁ is selected from a group consisting of V and I, X₂ is selected from a group consisting of N, Q, S, and A, X₃ is selected from a group consisting of A, G, D, Q, and S, and X₄ is selected from a group consisting of S and N; and
(3) CDRH3 including or consisting of a sequence WDGYVYYYGX₁DY, where X₁ is selected from a group consisting of L and M;
(4) CDRL1 including or consisting of a sequence shown in SEQ ID NO: 27;
(5) CDRL2 including or consisting of a sequence shown in SEQ ID NO: 31; and
(6) CDRL3 including or consisting of a sequence QQYWX₁TPYT, where X₁ is selected from a group consisting of S and N.

In some embodiments, the antibody includes:
(1) CDRH1 including or consisting of a sequence shown in SEQ ID NO: 5;
(2) CDRH2 including or consisting of a sequence YX₁NPNNGATX₂YNQKFKG, where X₁ is selected from a group consisting of V and I and X₂ is selected from a group consisting of S and N; and
(3) CDRH3 including or consisting of a sequence WDGYVYYYGX₁DY, where X₁ is selected from a group consisting of L and M;
(4) CDRL1 including or consisting of a sequence shown in SEQ ID NO: 27;
(5) CDRL2 including or consisting of a sequence shown in SEQ ID NO: 31; and
(6) CDRL3 including or consisting of a sequence QQYWX₁TPYT, where X₁ is selected from a group consisting of S and N.

In some embodiments, an isolated anti-CLDN6 antibody or an antigen-binding fragment thereof is provided, including a heavy-chain variable region and a light-chain variable region. The heavy-chain variable region includes:
(i) CDRH1 including or consisting of any one of sequences shown in SEQ ID NOs: 1-5 and 7 or a sequence having a sequence identity of at least 90%, 91%, 92%, 93%, 94%, 95%, 96%, 97%, 98%, or 99% with any one of the sequences shown in SEQ ID NOs: 1-5 and 7;
(ii) CDRH2 including or consisting of any one of sequences shown in SEQ ID NOs: 8-13, 15, and 74-76 or a sequence having a sequence identity of at least 90%, 91%, 92%, 93%, 94%, 95%, 96%, 97%, 98%, or 99% with any one of the sequences shown in SEQ ID NOs: 8-13, 15, and 74-76; and
(iii) CDRH3 including or consisting of any one of sequences shown in SEQ ID NOs: 16-21, 23, and 77-78 or a sequence having a sequence identity of at least 90%, 91%, 92%, 93%, 94%, 95%, 96%, 97%, 98%, or 99% with any one of the sequences shown in SEQ ID NOs: 16-21, 23, and 77-78.

The light-chain variable region includes:
(i) CDRL1 including or consisting of any one of sequences shown in SEQ ID NOs: 24-27 and 29 or a sequence having a sequence identity of at least 90%, 91%, 92%, 93%, 94%, 95%, 96%, 97%, 98%, or 99% with any one of the sequences shown in SEQ ID NOs: 24-27 and 29;
(ii) CDRL2 including or consisting of any one of sequences shown in SEQ ID NOs: 30-33 or a sequence having a sequence identity of at least 90%, 91%, 92%, 93%, 94%, 95%, 96%, 97%, 98%, or 99% with any one of the sequences shown in SEQ ID NOs: 30-33; and
(iii) CDRL3 including or consisting of any one of sequences shown in SEQ ID NOs: 34-38 and 40 or a sequence having a sequence identity of at least 90%, 91%, 92%, 93%, 94%, 95%, 96%, 97%, 98%, or 99% with any one of the sequences shown in SEQ ID NOs: 34-38, 40, and 179.

Variable regions and CDRs in an antibody sequence can be identified according to general rules established in the art (as described above, the Kabat numbering system, for example) or by aligning the sequence with a database of known variable regions.

In other embodiments, an amino acid sequence of CDR may have an identity of at least 90%, 91%, 92%, 93%, 94%, 95%, 96%, 97%, 98%, or 99% with the corresponding sequence described above. As an illustrative example, the antibody may include CDRH1 that has a sequence identity of at least 90%, 91%, 92%, 93%, 94%, 95%, 96%, 97%, 98%, or 99% with CDRH1 shown in any of SEQ ID NOs: 1-5 and 7.

In some embodiments, the isolated anti-CLDN6 antibody or the antigen-binding fragment thereof includes:
CDRH1 including or consisting of a sequence shown in SEQ ID NO: 1; CDRH2 including or consisting of a sequence shown in SEQ ID NO: 8; CDRH3 including or consisting of a sequence shown in SEQ ID NO: 16; CDRL1 including or consisting of a sequence shown in SEQ ID NO: 24; CDRL2 including or consisting of a sequence shown in SEQ ID NO: 30; and CDRL3 including or consisting of a sequence shown in SEQ ID NO: 34.

In some embodiments, the isolated anti-CLDN6 antibody or the antigen-binding fragment thereof includes:
CDRH1 including or consisting of a sequence shown in SEQ ID NO: 2; CDRH2 including or consisting of a sequence shown in SEQ ID NO: 9; CDRH3 including or consisting of a sequence shown in SEQ ID NO: 17; CDRL1 including or consisting of a sequence shown in SEQ ID NO: 25; CDRL2 including or consisting of a sequence shown in SEQ ID NO: 31; and CDRL3 including or consisting of a sequence shown in SEQ ID NO: 35.

In some embodiments, the isolated anti-CLDN6 antibody or the antigen-binding fragment thereof includes:
CDRH1 including or consisting of a sequence shown in SEQ ID NO: 3; CDRH2 including or consisting of a sequence shown in SEQ ID NO: 10; CDRH3 including or consisting of a sequence shown in SEQ ID NO: 18; CDRL1 including or consisting of a sequence shown in SEQ ID NO: 25; CDRL2 including or consisting of a sequence shown in SEQ ID NO: 32; and CDRL3 including or consisting of a sequence shown in SEQ ID NO: 36.

In some embodiments, the isolated anti-CLDN6 antibody or the antigen-binding fragment thereof includes:
CDRH1 including or consisting of a sequence shown in SEQ ID NO: 4; CDRH2 including or consisting of a sequence shown in SEQ ID NO: 11; CDRH3 including or consisting of a sequence shown in SEQ ID NO: 19; CDRL1 including or consisting of a sequence shown in SEQ ID NO: 26; CDRL2 including or consisting of a sequence shown in SEQ ID NO: 33; and CDRL3 including or consisting of a sequence shown in SEQ ID NO: 179.

In some embodiments, the isolated anti-CLDN6 antibody or the antigen-binding fragment thereof includes:
CDRH1 including or consisting of a sequence shown in SEQ ID NO: 5; CDRH2 including or consisting of a sequence shown in SEQ ID NO: 12; CDRH3 including or consisting of a sequence shown in SEQ ID NO: 20; CDRL1 including or consisting of a sequence shown in SEQ ID NO: 27; CDRL2 including or consisting of a sequence shown in SEQ ID NO: 31; and CDRL3 including or consisting of a sequence shown in SEQ ID NO: 37.

In some embodiments, the isolated anti-CLDN6 antibody or the antigen-binding fragment thereof includes:
CDRH1 including or consisting of a sequence shown in SEQ ID NO: 5; CDRH2 including or consisting of a sequence shown in SEQ ID NO: 13; CDRH3 including or consisting of a sequence shown in SEQ ID NO: 21; CDRL1 including or consisting of a sequence shown in SEQ ID NO: 27; CDRL2 including or consisting of a sequence shown in SEQ ID NO: 31; and CDRL3 including or consisting of a sequence shown in SEQ ID NO: 38.

In some embodiments, the isolated anti-CLDN6 antibody or the antigen-binding fragment thereof includes:
CDRH1 including or consisting of a sequence shown in SEQ ID NO: 7; CDRH2 including or consisting of a sequence shown in SEQ ID NO: 15; CDRH3 including or consisting of a sequence shown in SEQ ID NO: 23; CDRL1 including or consisting of a sequence shown in SEQ ID NO: 29; CDRL2 including or consisting of a sequence shown in SEQ ID NO: 30; and CDRL3 including or consisting of a sequence shown in SEQ ID NO: 40.

In some embodiments, the isolated anti-CLDN6 antibody or the antigen-binding fragment thereof includes:
CDRH1 including or consisting of a sequence shown in SEQ ID NO: 5; CDRH2 including or consisting of a sequence shown in SEQ ID NO: 12; CDRH3 including or consisting of a sequence shown in SEQ ID NO: 20; CDRL1 including or consisting of a sequence shown in SEQ ID NO: 27; CDRL2 including or consisting of a sequence shown in SEQ ID NO: 31; and CDRL3 including or consisting of a sequence shown in SEQ ID NO: 38.

In some embodiments, the isolated anti-CLDN6 antibody or the antigen-binding fragment thereof includes:
CDRH1 including or consisting of a sequence shown in SEQ ID NO: 7; CDRH2 including or consisting of a sequence shown in SEQ ID NO: 24; CDRH3 including or consisting of a sequence shown in SEQ ID NO: 23; CDRL1 including or consisting of a sequence shown in SEQ ID NO: 29; CDRL2 including or consisting of a sequence shown in SEQ ID NO: 30; and CDRL3 including or consisting of a sequence shown in SEQ ID NO: 40.

In some embodiments, the isolated anti-CLDN6 antibody or the antigen-binding fragment thereof includes:
CDRH1 including or consisting of a sequence shown in SEQ ID NO: 5; CDRH2 including or consisting of a sequence shown in SEQ ID NO: 75; CDRH3 including or consisting of a sequence shown in SEQ ID NO: 20; CDRL1 including or consisting of a sequence shown in SEQ ID NO: 27; CDRL2 including or consisting of a sequence shown in SEQ ID NO: 31; and CDRL3 including or consisting of a sequence shown in SEQ ID NO: 37.

In some embodiments, the isolated anti-CLDN6 antibody or the antigen-binding fragment thereof includes:
CDRH1 including or consisting of a sequence shown in SEQ ID NO: 5; CDRH2 including or consisting of a sequence shown in SEQ ID NO: 76; CDRH3 including or consisting of a sequence shown in SEQ ID NO: 20; CDRL1 including or consisting of a sequence shown in SEQ ID NO: 27; CDRL2 including or consisting of a sequence shown in SEQ ID NO: 31; and CDRL3 including or consisting of a sequence shown in SEQ ID NO: 37.

In some embodiments, the isolated anti-CLDN6 antibody or the antigen-binding fragment thereof includes:
CDRH1 including or consisting of a sequence shown in SEQ ID NO: 5; CDRH2 including or consisting of a sequence shown in SEQ ID NO: 12; CDRH3 including or consisting of a sequence shown in SEQ ID NO: 77; CDRL1 including or consisting of a sequence shown in SEQ ID NO: 27; CDRL2 including or consisting of a sequence shown in SEQ ID NO: 31; and CDRL3 including or consisting of a sequence shown in SEQ ID NO: 37.

In some embodiments, the isolated anti-CLDN6 antibody or the antigen-binding fragment thereof includes:
CDRH1 including or consisting of a sequence shown in SEQ ID NO: 5; CDRH2 including or consisting of a sequence shown in SEQ ID NO: 12; CDRH3 including or consisting of a sequence shown in SEQ ID NO: 78; CDRL1 including or consisting of a sequence shown in SEQ ID NO: 27; CDRL2 including or consisting of a sequence shown in SEQ ID NO: 31; and CDRL3 including or consisting of a sequence shown in SEQ ID NO: 37.

An antibody chain including a specific CDR sequence (such as, a specific CDR3 sequence) mentioned in the present disclosure means that the specific CDR sequence constitutes a CDR region (such as a CDR3 region) of the antibody chain. That is, the CDR region consists of the specific CDR sequence, or the specific CDR sequence serves as a part of the CDR region (such as the CDR3 region of the antibody chain). That is, the CDR region includes the specific CDR sequence.

In some embodiments, in the isolated anti-CLDN6 antibody or the antigen-binding fragment thereof, the heavy-chain variable region includes or consists of any one of amino acid sequences shown in SEQ ID NOs: 41-46, 48-60, and 79-83, or includes or consists of a sequence having a sequence identity of at least 85%, 90%, 91%, 92%, 93%, 94%, 95%, 96%, 97%, 98%, or 99% with any one of the amino acid sequences shown in SEQ ID NOs: 41-46, 48-60, and 79-83; and the light-chain variable region includes or consists of any one of amino acid sequences shown in SEQ ID NOs: 61-66 and 68-73, or includes or consists of a sequence having a sequence identity of at least 85%, 90%, 91%, 92%, 93%, 94%, 95%, 96%, 97%, 98%, or 99% with any one of the amino acid sequences shown in SEQ ID NOs: 61-66 and 68-73.

In some embodiments, the isolated anti-CLDN6 antibody or the antigen-binding fragment thereof includes:
(a) a heavy-chain variable region including or consisting of an amino acid sequence shown in SEQ ID NO: 41 and a light-chain variable region including or consisting of an amino acid sequence shown in SEQ ID NO: 61;
(b) a heavy-chain variable region including or consisting of an amino acid sequence shown in SEQ ID NO: 42 and a light-chain variable region including or consisting of an amino acid sequence shown in SEQ ID NO: 62;
(c) a heavy-chain variable region including or consisting of an amino acid sequence shown in SEQ ID NO: 43 and a light-chain variable region including or consisting of an amino acid sequence shown in SEQ ID NO: 63;
(d) a heavy-chain variable region including or consisting of an amino acid sequence shown in SEQ ID NO: 44 and a light-chain variable region including or consisting of an amino acid sequence shown in SEQ ID NO: 64;
(e) a heavy-chain variable region including or consisting of any one of amino acid sequences shown in SEQ ID NOs: 45 and 80-83 and a light-chain variable region including or consisting of an amino acid sequence shown in SEQ ID NO: 65;
(f) a heavy-chain variable region including or consisting of an amino acid sequence shown in SEQ ID NO: 46 or 45 and a light-chain variable region including or consisting of an amino acid sequence shown in SEQ ID NO: 66;
(g) a heavy-chain variable region including or consisting of an amino acid sequence shown in SEQ ID NO: 48 or 79 and a light-chain variable region including or consisting of an amino acid sequence shown in SEQ ID NO: 68;
(h) a heavy-chain variable region including or consisting of any one of amino acid sequences shown in SEQ ID NOs: 49-52 and a light-chain variable region including or consisting of an amino acid sequence shown in SEQ ID NO: 69;
(i) a heavy-chain variable region including or consisting of any one of the amino acid sequences shown in SEQ ID NOs: 49-52 and a light-chain variable region including or consisting of an amino acid sequence shown in SEQ ID NO: 70;
(j) a heavy-chain variable region including or consisting of an amino acid sequence shown in SEQ ID NO: 53 or 54 and a light-chain variable region including or consisting of an amino acid sequence shown in SEQ ID NO: 71;
(k) a heavy-chain variable region including or consisting of any one of amino acid sequences shown in SEQ ID NOs: 53-56 and a light-chain variable region including or consisting of an amino acid sequence shown in SEQ ID NO: 72; or
(l) a heavy-chain variable region including or consisting of any one of amino acid sequences shown in SEQ ID NOs: 57-60 and a light-chain variable region including or consisting of an amino acid sequence shown in SEQ ID NO: 73.

In some embodiments, the isolated anti-CLDN6 antibody or the antigen-binding fragment thereof may include a conservative substitution or modification or replacement (such as a conservative substitution), a deletion, or an addition of an amino acid in a variable region of a heavy chain and/or a light chain (for example, a substitution, a deletion, or an addition of 1, 2, 3, 4, or 5 amino acids; or a conservative substitution of at most 20, at most 15, at most 10, or at most 5 amino acids), or may have a sequence identity of at least 85%, at least 90%, at least 95%, at least 96%, at least 97%, at least 98%, at least 99%, or at least 100% with an antibody or an antigen-binding fragment from which the isolated anti-CLDN6 antibody or the antigen-binding fragment thereof is derived. An amino acid deletion variant resulting from a deletion at an N terminus and/or a C terminus of a protein is also referred to as an N-terminus and/or C-terminus truncation variant. It should be understood in the art that some conservative sequence modifications may be conducted without eliminating antigen binding, for example, as described in Brummell et al. (1993) Biochem 32: 1180-8; de Wildt et al. (1997) Prot. Eng. 10: 835-41; Komissarov et al. (1997) J. Biol. Chem. 272: 26864-26870; Hall et al. (1992) J. Immunol. 149: 1605-12; Kelley and O'Connell (1993) Biochem. 32: 6862-35; Adib-Conquy et al. (1998) Int. Immunol. 10: 341-6; and Beers et al. (2000) Clin. Can. Res. 6: 2835-43.

In some embodiments, the isolated anti-CLDN6 antibody includes:
(1) CDRH1 including or consisting of a sequence shown in SEQ ID NO: 5;
(2) CDRH2 including or consisting of a sequence shown in SEQ ID NO: 12, or including or consisting of a sequence YX₁NPNNGAX₂X₃YNQKFKG, where X₁ is selected from a group consisting of A, I, L, and V, X₂ is selected from a group consisting of S and T, and X₃ is selected from a group consisting of S and N; and preferably, CDRH2 including or consisting of a sequence shown in SEQ ID NO: 12, 91, 92, 93, 94, 119, 120, 121, 122, 123, 124, or 125;
(3) CDRH3 including or consisting of a sequence shown in SEQ ID NO: 20, or including or consisting of a sequence WDGYX₁YYYGX₂DX₃, where X₁ is selected from a group consisting of V and S, X₂ is selected from a group consisting of V, L, and M, and X₃ is selected from a group consisting of Y and F; and preferably, CDRH3 including or consisting of a sequence shown in SEQ ID NO: 20, 95, 96, 97, 126, 127, 128, 129, 130, or 131;
(4) CDRL1 including or consisting of a sequence shown in SEQ ID NO: 27, or including or consisting of a sequence KASX₁HX₂NX₃WLA, where X₁ is selected from a group consisting of E and D, X₂ is selected from a group consisting of I and V, and X₃ is selected from a group consisting of H and Q; and preferably, CDRL1 including or consisting of a sequence shown in SEQ ID NO: 27, 98, 99, 100, 132, 133, 134, or 135;
(5) CDRL2 including or consisting of a sequence shown in SEQ ID NO: 31, or including or consisting of a sequence shown in SEQ ID NO: 101; and
(6) CDRL3 including or consisting of a sequence shown in SEQ ID NO: 37, or including or consisting of a sequence QQYX₁X₂TPYX₃, where X₁ is selected from a group consisting of W and G, X₂ is selected from a group consisting of S, H, and N, and X₃ is selected from a group consisting of S and T; and preferably, CDRL3 including or consisting of a sequence shown in SEQ ID NO: 37, 102, 103, 104, 136, 137, 138, 139, 140, 141, or 142.

Preferably, the isolated anti-CLDN6 antibody includes:
(1) CDRH1 including or consisting of the sequence shown in SEQ ID NO: 5;
(2) CDRH2 including or consisting of the sequence shown in SEQ ID NO: 12, or including or consisting of the sequence YX₁NPNNGAX₂X₃YNQKFKG, where X₁ is selected from a group consisting of A, I, L, and V, X₂ is selected from a group consisting of S and T, and X₃ is selected from a group consisting of S and N; and preferably, CDRH2 including or consisting of the sequence shown in SEQ ID NO: 12, 91, 92, 93, 94, 119, 120, 121, 122, 123, 124, or 125;
(3) CDRH3 including or consisting of the sequence shown in SEQ ID NO: 20;
(4) CDRL1 including or consisting of the sequence shown in SEQ ID NO: 27;
(5) CDRL2 including or consisting of the sequence shown in SEQ ID NO: 31; and
(6) CDRL3 including or consisting of the sequence shown in SEQ ID NO: 37.

Alternatively, the isolated anti-CLDN6 antibody includes:
(1) CDRH1 including or consisting of the sequence shown in SEQ ID NO: 5.
(2) CDRH2 including or consisting of the sequence shown in SEQ ID NO: 12.
(3) CDRH3 including or consisting of the sequence shown in SEQ ID NO: 20, or including or consisting of the sequence WDGYX₁YYYGX₂DX₃, where X₁ is selected from a group consisting of V and S, X₂ is selected from a group consisting of V, L, and M, and X₃ is selected from a group consisting of Y and F; and preferably, CDRH3 including or consisting of the sequence shown in SEQ ID NO: 20, 95, 96, 97, 126, 127, 128, 129, 130, or 131; (4) CDRL1 including or consisting of the sequence shown in SEQ ID NO: 27;
(5) CDRL2 including or consisting of the sequence shown in SEQ ID NO: 31; and
(6) CDRL3 including or consisting of the sequence shown in SEQ ID NO: 37.

Alternatively, the isolated anti-CLDN6 antibody includes:
(1) CDRH1 including or consisting of the sequence shown in SEQ ID NO: 5;
(2) CDRH2 including or consisting of the sequence shown in SEQ ID NO: 12;
(3) CDRH3 including or consisting of the sequence shown in SEQ ID NO: 20;
(4) CDRL1 including or consisting of the sequence shown in SEQ ID NO: 27, or including or consisting of the sequence KASX₁HX₂NX₃WLA, where X₁ is selected from a group consisting of E and D, X₂ is selected from a group consisting of I and V, and X₃ is selected from a group consisting of H and Q; and preferably, CDRL1 including or consisting of the sequence shown in SEQ ID NO: 27, 98, 99, 100, 132, 133, 134, or 135;
(5) CDRL2 including or consisting of the sequence shown in SEQ ID NO: 31; and
(6) CDRL3 including or consisting of the sequence shown in SEQ ID NO: 37.

Alternatively, the isolated anti-CLDN6 antibody includes:
(1) CDRH1 including or consisting of the sequence shown in SEQ ID NO: 5;
(2) CDRH2 including or consisting of the sequence shown in SEQ ID NO: 12;
(3) CDRH3 including or consisting of the sequence shown in SEQ ID NO: 20;
(4) CDRL1 including or consisting of the sequence shown in SEQ ID NO: 27;
(5) CDRL2 including or consisting of a sequence shown in SEQ ID NO: 31, or including or consisting of the sequence shown in SEQ ID NO: 101; and
(6) CDRL3 including or consisting of the sequence shown in SEQ ID NO: 37.

Alternatively, the isolated anti-CLDN6 antibody includes:
(1) CDRH1 including or consisting of the sequence shown in SEQ ID NO: 5;
(2) CDRH2 including or consisting of the sequence shown in SEQ ID NO: 12;
(3) CDRH3 including or consisting of the sequence shown in SEQ ID NO: 20;
(4) CDRL1 including or consisting of the sequence shown in SEQ ID NO: 27;
(5) CDRL2 including or consisting of the sequence shown in SEQ ID NO: 31; and
(6) CDRL3 including or consisting of the sequence shown in SEQ ID NO: 37, or including or consisting of the sequence QQYX₁X₂TPYX₃, where X₁ is selected from a group consisting of W and G, X₂ is selected from a group consisting of S, H, and N, and X₃ is selected from a group consisting of S and T; and preferably, CDRL3 including or consisting of the sequence shown in SEQ ID NO: 37, 102, 103, 104, 136, 137, 138, 139, 140, 141, or 142.

Alternatively, the isolated anti-CLDN6 antibody includes:
(1) CDRH1 including or consisting of the sequence shown in SEQ ID NO: 5;
(2) CDRH2 including or consisting of any one of sequences shown in SEQ ID NOs: 12, 91-94, and 119-125;
(3) CDRH3 including or consisting of any one of sequences shown in SEQ ID NOs: 20, 95-97, and 126-131;
(4) CDRL1 including or consisting of any one of sequences shown in SEQ ID NOs: 27, 98-99, and 132-135;
(5) CDRL2 including or consisting of a sequence shown in SEQ ID NO: 31 or 101; and
(6) CDRL3 including or consisting of any one of sequences shown in SEQ ID NOs: 37, 102-104, and 136-142.

Preferably, the isolated anti-CLDN6 antibody includes or consists of CDRs shown in Table I and/or Table J. For example, the isolated anti-CLDN6 antibody includes or consists of CDRH1, CDRH2, and CDRH3 from Table I and CDRL1, CDRL2, and CDRL3 from Table J.

Preferably, the isolated anti-CLDN6 antibody includes or consists of CDRs shown in Table G.

Preferably, the isolated anti-CLDN6 antibody includes or consists of CDRs shown in Table K.

The present disclosure further discloses an isolated anti-CLDN6 antibody or an antigen-binding fragment thereof, including a heavy-chain variable region and a light-chain variable region.

The heavy-chain variable region includes or consists of any one of amino acid sequences shown in SEQ ID NOs: 105-111, or includes or consists of a sequence having a sequence identity of at least 80% including at least 85%, 90%, 91%, 92%, 93%, 94%, 95%, 96%, 97%, 98%, or 99% with any one of the amino acid sequences shown in SEQ ID NOs: 105-111, and the light-chain variable region includes or consists of an amino acid sequence shown in SEQ ID NO: 69, or includes or consists of a sequence having a sequence identity of at least 85% including at least 90%, 91%, 92%, 93%, 94%, 95%, 96%, 97%, 98%, or 99% with the amino acid sequence shown in SEQ ID NO: 69.

Alternatively, the heavy-chain variable region includes or consists of an amino acid sequence shown in SEQ ID NO: 52, or includes or consists of a sequence having a sequence identity of at least 80% including at least 85%, 90%, 91%, 92%, 93%, 94%, 95%, 96%, 97%, 98%, or 99% with the amino acid sequence shown in SEQ ID NO: 52, and the light-chain variable region includes or consists of any one of amino acid sequences shown in SEQ ID NOs: 112-118, or includes or consists of a sequence having a sequence identity of at least 80% including at least 85%, 90%, 91%, 92%, 93%, 94%, 95%, 96%, 97%, 98%, or 99% with any one of the amino acid sequences shown in SEQ ID NOs: 112-118.

As mentioned above, the term "conservative substitution/replacement" used herein refers to an amino acid substitution/replacement that does not adversely affect or alter the basic properties of a protein/polypeptide including an amino acid sequence. For example, the conservative substitution/replacement can be introduced using a standard technique known in the art (such as site-directed mutagenesis and polymerase chain reaction (PCR)-mediated mutagenesis). A conservative amino acid substitution includes a substitution in which an amino acid residue is substituted by another amino acid residue with a similar side chain to the former amino acid residue. For example, a corresponding amino acid residue is substituted with a residue that is physically or functionally similar to the former amino acid residue (such as, similar in a size, a shape, a charge, and chemical properties including an ability to form covalent or hydrogen bonding). Families of amino acid residues with similar side chains have been defined in the art. These families include amino acids with basic side chains (such as lysine, arginine, and histidine), amino acids with acidic side chains (such as aspartic acid and glutamic acid), amino acids with uncharged polar side chains (such as glycine, asparagine, glutamine, serine, threonine, tyrosine, cysteine, and tryptophan), amino acids with nonpolar side chains (such as alanine, valine, leucine, isoleucine, proline, phenylalanine, and methionine), amino acids with β-branched side chains (such as threonine, valine, and isoleucine), and amino acids with aromatic side chains (such as tyrosine, phenylalanine, tryptophan, and histidine). Therefore, a corresponding amino acid residue is preferably substituted by another amino acid residue from a family with the same side chain as the former amino acid residue. Methods for identifying conservative amino acid substitutions are well known in the art (see, for example, Brummell et al., Biochem. 32: 1180-1187 (1993); Kobayashi et al., Protein Eng. 12 (10): 879-884 (1999); and Burks et al., Proc. Natl. Acad. Sci. USA 94: 412-417 (1997), which are incorporated herein by reference).

According to the present disclosure, if it is mentioned that a specific heavy chain and/or a specific light chain (such as, a chain including a specific CDR sequence) is included, the two heavy chains and/or the two light chains of the antibody preferably include the specific heavy chain and/or the specific light chain, respectively.

In some embodiments, the isolated anti-CLDN6 antibody or the antigen-binding fragment thereof has one or more of the following properties:
binding to human or monkey CLDN6 with EC50 of 5 µg/mL or less;
cross-reactivity: demonstrating a lower binding affinity to human CLDN3 and/or human CLDN4 and/or human CLDN9 than to human CLDN6; and
killing and/or inhibiting a cell expressing the human CLDN6 (including a tumor cell, such as a CLDN6-expressing tumor cell).

A unique feature of the antibody of the present disclosure is the ability to bind to CLDN6 on a cell surface. This has been proved by the flow cytometry analysis of CLDN6-expressing cells.

Regardless of how the antibody is produced, a method for testing the ability of the antibody to bind to a CLDN6 epitope is known in the art and includes any antibody-antigen binding assay, such as radioimmunoassay (RIA), enzyme-linked immunosorbent assay (ELISA), Western blot, immunoprecipitation, SPR, and competitive inhibition assay (see, for example, Janeway et al., infra, and U.S. Patent Application Publication No. 2002/0197266, and the preceding sections regarding competitive assays).

According to the present disclosure, in a standard assay (such as the assay described in the present disclosure), if an antibody demonstrates a significant affinity for a predetermined target (such as CLDN6 or a CLDN6-expressing cell), the antibody can bind to the predetermined target. To test the binding of a monoclonal antibody to a CLDN-expressing live cell, flow cytometry may be adopted. Preferably, the binding of the antibody to a target expressed on a cell surface is determined through FACS analysis. If the antibody detectably binds to the target (CLDN6 or a CLDN6-expressing cell), the antibody can bind to the target with an affinity. Preferably, if the antibody in the present disclosure is present at a concentration of 10 µg/mL or less, 5 µg/mL or less, 3 µg/mL or less, 2 µg/mL or less, 1 µg/mL or less, or 0.5 µg/mL or less, the antibody detectably binds to the target.

In some embodiments, the isolated anti-CLDN6 antibody or the antigen-binding fragment thereof may have the following binding property: the isolated anti-CLDN6 antibody or the antigen-binding fragment thereof binds to human or monkey CLDN6 with KD of 5 × 10⁻⁸ M or lower; binds to human or monkey CLDN6 with KD of 1 × 10⁻⁹ M or lower; binds to human or monkey CLDN6 with KD of 5 × 10⁻⁹ M or lower; or binds to human or monkey CLDN6 with KD of 1 × 10⁻¹⁰ M or lower.

In some embodiments, the binding property of the isolated anti-CLDN6 antibody or the antigen-binding fragment thereof reflected by EC50 can be as follows: the isolated anti-CLDN6 antibody or the antigen-binding fragment thereof binds to human CLDN6 or monkey CLDN6 with EC50 of 5 µg/mL or less; binds to human CLDN6 or monkey CLDN6 with EC50 of 3 µg/mL or less; binds to human CLDN6 or monkey CLDN6 with EC50 of 2 µg/mL or less; binds to human CLDN6 or monkey CLDN6 with EC50 of 1 µg/mL or less; binds to human CLDN6 or monkey CLDN6 with EC50 of 0.5 µg/mL or less; binds to human CLDN6 or monkey CLDN6 with EC50 of 0.3 µg/mL or less; or binds to human CLDN6 or monkey CLDN6 with EC50 of 0.1 µg/mL or less.

In a standard assay, if the antibody does not exhibit a significant affinity for the target and does not bind significantly to the target, the antibody is incapable of or substantially incapable of binding to the target. Preferably, the binding of the antibody to the target (such as CLDN6) expressed on a cell surface is determined through FACS analysis. If the antibody does not detectably bind to the target, the antibody is (substantially) incapable of binding to the target. Preferably, when the antibody of the present disclosure is present at a concentration of up to 1 µg/mL, up to 2 µg/mL, preferably up to 5 µg/mL, preferably up to 10 µg/mL, preferably up to 20 µg/mL, more preferably up to 50 µg/mL, particularly up to 100 µg/mL, up to 150 µg/mL, up to 200 µg/mL, or higher, the antibody does not detectably bind to the target (CLDN).

If the antibody can bind to a predetermined target, but cannot bind to other targets (that is, the antibody does not exhibit a significant affinity for other targets or does not bind significantly to other targets in a standard assay), then the antibody is specific for the predetermined target. According to the present disclosure, if the antibody can bind to CLDN6, but cannot bind to or cannot bind significantly to other targets (particularly, CLDNs other than CLDN6, such as CLDN9, CLDN4, and CLDN3), then the antibody is specific for CLDN6.

The CLDN6 specificity described in the present disclosure refers to the ability to bind to one or more CLDN6 epitopes, particularly a CLDN6 epitope with a native conformation, and specifically refers to human CLDN6 specificity.

In some embodiments, the cross-reactivity of the isolated anti-CLDN6 antibody or the antigen-binding fragment thereof described in the (b) can be as follows: the isolated anti-CLDN6 antibody or the antigen-binding fragment thereof does not bind or binds negligibly to CLDNs other than CLDN6; and/or the isolated anti-CLDN6 antibody or the antigen-binding fragment thereof does not bind or binds negligibly to CLDN3; and/or the isolated anti-CLDN6 antibody or the antigen-binding fragment thereof does not bind or binds negligibly to CLDN9; and/or the isolated anti-CLDN6 antibody or the antigen-binding fragment thereof does not bind or binds negligibly to CLDN4.

TCGA data shows that CLDN9 is expressed at low levels in the pancreas (4.23 TPM, n = 4) and kidney (1.99 TPM, n = 25), and is almost not expressed in other normal tissues (with the highest expression level in the bile duct among the normal tissues: 0.82 TPM in, n = 9). The expression of CLDN9 is up-regulated in ovarian cancer (5.68 TPM, n = 426) and cholangiocarcinoma (8.44 TPM, n = 36). Therefore, an antibody with binding specificity for both CLDN6 and CLDN9 can enhance the binding to/inhibition of tumor cells without causing significant toxic and side effects.

In some embodiments, the isolated anti-CLDN6 antibody or the antigen-binding fragment thereof exhibits a high binding affinity for CLDN6 (for example, satisfying the binding property described in (a)) and a specified binding affinity for CLDN9 (for example, a binding affinity for CLDN9 is 1/10 or less, 1/50 or less, 1/100 or less, 1/120 or less, 1/150 or less, 1/200 or less, or 1/250 or less of a binding affinity for CLDN6). Antibodies or antigen-binding fragments thereof with such a binding property can demonstrate prominent and superior efficacy against tumors broadly expressing both CLDN6 and CLDN9, such as chimeric antibodies 58G3C7, 47D5D6, 51C10H4, 20A3-2A7, and 54B5A7, and improved or humanized antibodies.

In some embodiments, the cross-reactivity of the isolated anti-CLDN6 antibody or the antigen-binding fragment thereof described in the (b) can be as follows: the isolated anti-CLDN6 antibody or the antigen-binding fragment thereof does not bind or binds negligibly to CLDN3; and/or the isolated anti-CLDN6 antibody or the antigen-binding fragment thereof binds to CLDN9 (for example, a binding affinity for CLDN9 is 1/10 or less, 1/50 or less, 1/100 or less, 1/120 or less, 1/150 or less, 1/200 or less, or 1/250 or less of a binding affinity for CLDN6); and/or the isolated anti-CLDN6 antibody or the antigen-binding fragment thereof does not bind or binds negligibly to CLDN4.

In some embodiments, the cross-reactivity of the isolated anti-CLDN6 antibody or the antigen-binding fragment thereof described in the (b) can be as follows: the isolated anti-CLDN6 antibody or the antigen-binding fragment thereof does not bind or binds negligibly to CLDN3; and the isolated anti-CLDN6 antibody or the antigen-binding fragment thereof binds to CLDN9 (for example, a binding affinity for CLDN9 is 1/10 or less, 1/50 or less, 1/100 or less, 1/120 or less, 1/150 or less, 1/200 or less, or 1/250 or less of a binding affinity for CLDN6). Antibodies or antigen-binding fragments thereof with such a binding property can demonstrate prominent and superior efficacy against tumors broadly expressing both CLDN6 and CLDN9, and avoid cross-reactivity with tissues widely expressing CLDN3 to reduce toxic and side effects, such as chimeric antibodies 47D5D6, 51C10H4, 20A3-2A7, and 54B5A7, and improved or humanized antibodies.

In some embodiments, the cross-reactivity of the isolated anti-CLDN6 antibody or the antigen-binding fragment thereof described in the (b) can be as follows: the isolated anti-CLDN6 antibody or the antigen-binding fragment thereof does not bind or binds negligibly to CLDN4; and the isolated anti-CLDN6 antibody or the antigen-binding fragment thereof binds to CLDN9 (for example, a binding affinity for CLDN9 is 1/10 or less, 1/50 or less, 1/100 or less, 1/120 or less, 1/150 or less, 1/200 or less, or 1/250 or less of a binding affinity for CLDN6). Antibodies or antigen-binding fragments thereof with such a binding property can demonstrate prominent and superior efficacy against tumors broadly expressing both CLDN6 and CLDN9, and avoid cross-reactivity with tissues widely expressing CLDN4 to reduce toxic and side effects, such as chimeric antibodies 51C10H4 and 54B5A7, and improved or humanized antibodies.

In some embodiments, the cross-reactivity of the isolated anti-CLDN6 antibody or the antigen-binding fragment thereof described in the (b) can be as follows: the isolated anti-CLDN6 antibody or the antigen-binding fragment thereof does not bind or binds negligibly to CLDN4; the isolated anti-CLDN6 antibody or the antigen-binding fragment thereof does not bind or binds negligibly to CLDN3; and the isolated anti-CLDN6 antibody or the antigen-binding fragment thereof binds to CLDN9 (for example, a binding affinity for CLDN9 is 1/10 or less, 1/50 or less, 1/100 or less, 1/120 or less, 1/150 or less, 1/200 or less, or 1/250 or less of a binding affinity for CLDN6). Antibodies or antigen-binding fragments thereof with such a binding property can demonstrate prominent and superior efficacy against tumors broadly expressing both CLDN6 and CLDN9, and avoid cross-reactivity with tissues widely expressing CLDN4 and CLDN3 to avoid/reduce toxic and side effects, such as chimeric antibodies 51C10H4 and 54B5A7, and improved or humanized antibodies. As a result, the therapeutic efficacy can be maximized, and the systemic toxicity can be minimized.

FACS analysis reveals that the antibodies of the present disclosure, particularly the antibodies with the aforementioned properties, demonstrate high C6 reactivity (selective binding to a surface of a CLDN6-expressing cell), but low cross-reactivity.

Although the antibodies of the present disclosure possess significant CLDN6-specific binding properties, the cross-reactivity of some antibodies with CLDN3/CLDN4 unexpectedly endows these antibodies with strong and extensive tumor cell-killing effects.

For example, studies have shown that expression levels of CLDN3 and CLDN4 in several ovarian cancer cases are 83 to 109 times higher than those in normal epithelial cells. Further, DNA microarray analysis has revealed that CLDN3 and CLDN4 are two of the five most differentially expressed genes in ovarian cancer, and the expression levels of CLDN3 and CLDN4 are abnormally elevated in many ovarian cancer cases. Therefore, antibodies targeting CLDN3, CLDN4, or both have the potential to serve as therapeutic agents. Some antibodies have been proved to demonstrate an antitumor activity in preclinical animal models, and there are antibodies having advanced to clinical stages. For example, in the prior art, a murine antibody KM3900 that can recognize and bind to the second extracellular loop of CLDN4 has been selected and humanized to produce a humanized antibody KM3934. The humanized antibody KM3934 exhibits ADCC and CDC in *in vitro* experiments, and can significantly inhibit the tumor growth in immunodeficient mice transplanted with a human ovarian cancer cell line MCAS or a human pancreatic cancer cell line CFPAC-1.

In some embodiments, the isolated anti-CLDN6 antibody or the antigen-binding fragment thereof exhibits a high binding affinity for CLDN6 (for example, satisfying the binding property described in (a)) and a specified binding affinity for CLDN4 (for example, a binding affinity for CLDN4 is 1/4 or less, 1/10 or less, 1/50 or less, 1/100 or less, 1/120 or less, 1/150 or less, 1/200 or less, or 1/250 or less of a binding affinity for CLDN6). Antibodies or antigen-binding fragments thereof with such a binding property can demonstrate prominent and superior efficacy against tumors or tissues broadly expressing both CLDN6 and CLDN4. For example, the antibodies or antigen-binding fragments thereof with such a binding property exhibit an outstanding inhibitory or killing effect for ovarian cancer cells. Examples of the antibodies or antigen-binding fragments thereof with such a binding property include chimeric antibodies 20A3-2A7, 47D5D6, 58G3C7, and 15H2D4, and improved or humanized antibodies.

In some embodiments, the isolated anti-CLDN6 antibody or the antigen-binding fragment thereof exhibits a high binding affinity for CLDN6 (for example, satisfying the binding property described in (a)) and a specified binding affinity for CLDN3 (for example, a binding affinity for CLDN3 is 1/20 or less, 1/50 or less, 1/100 or less, 1/120 or less, 1/150 or less, 1/200 or less, or 1/250 or less of a binding affinity for CLDN6). Antibodies or antigen-binding fragments thereof with such a binding property can demonstrate prominent and superior efficacy against tumors or tissues broadly expressing both CLDN6 and CLDN3. For example, the antibodies or antigen-binding fragments thereof with such a binding property exhibit an outstanding inhibitory or killing effect for ovarian cancer cells. Examples of the antibodies or antigen-binding fragments thereof with such a binding property include chimeric antibodies 47A5B10 and 58G3C7, and improved or humanized antibodies.

In some embodiments, the isolated anti-CLDN6 antibody or the antigen-binding fragment thereof exhibits a high binding affinity for CLDN6 (for example, satisfying the binding property described in (a)), a specified binding affinity for CLDN3 (for example, a binding affinity for CLDN3 is 1/20 or less, 1/50 or less, 1/100 or less, 1/120 or less, 1/150 or less, 1/200 or less, or 1/250 or less of a binding affinity for CLDN6), and a specified binding affinity for CLDN4 (for example, a binding affinity for CLDN4 is 1/4 or less, 1/10 or less, 1/50 or less, 1/100 or less, 1/120 or less, 1/150 or less, 1/200 or less, or 1/250 or less of a binding affinity for CLDN6). Antibodies or antigen-binding fragments thereof with such a binding property can demonstrate prominent and superior efficacy against tumors or tissues broadly expressing CLDN6, CLDN3, and CLDN4. For example, the antibodies or antigen-binding fragments thereof with such a binding property exhibit an outstanding inhibitory or killing effect for ovarian cancer cells. Examples of the antibodies or antigen-binding fragments thereof with such a binding property include chimeric antibodies 47A5B10 and 58G3C7, and improved or humanized antibodies.

In some embodiments of the present disclosure, the killing and/or inhibiting a cell expressing the human CLDN6 (including a tumor cell, such as a CLDN6-expressing tumor cell) by the isolated anti-CLDN6 antibody or the antigen-binding fragment thereof described in the (c) may be as follows: the isolated anti-CLDN6 antibody or the antigen-binding fragment thereof induces killing and/or inhibition of the cell expressing the human CLDN6 (including a tumor cell, such as a CLDN6-expressing tumor cell) through ADCC; and/or the isolated anti-CLDN6 antibody or the antigen-binding fragment thereof induces killing of the cell expressing the human CLDN6 (including a tumor cell, such as a CLDN6-expressing tumor cell) through CDC.

In an aspect, the present disclosure provides an isolated anti-CLDN6 antibody or an antigen-binding fragment thereof that competitively binds to CLDN6 expressed on a surface of a cell with the isolated anti-CLDN6 antibody or the antigen-binding fragment thereof described above.

In an aspect, the present disclosure provides an isolated anti-CLDN6 antibody or an antigen-binding fragment thereof, including: a CDR sequence including or consisting of a sequence shown in any of SEQ ID NOs: 41-46, 48-60, and 79-83 and a CDR sequence including or consisting of a sequence shown in any of SEQ ID NOs: 61-66 and 68-73.

In some embodiments, the isolated anti-CLDN6 antibody is a monoclonal antibody.

In some embodiments, the isolated anti-CLDN6 antibody is a chimeric antibody, a humanized antibody, or an improved antibody such as an improved chimeric antibody.

In some embodiments, the isolated anti-CLDN6 antibody or the antigen-binding fragment thereof includes a constant region of IgG. The constant region of IgG is preferably a constant region of IgG1, IgG2, IgG3, or IgG4. The constant region of IgG is more preferably a constant region of IgG1.

In some embodiments, the isolated anti-CLDN6 antibody or the antigen-binding fragment thereof includes: a heavy chain including or consisting of any one selected from a group consisting of polypeptides having a sequence identity of at least 90%, 91%, 92%, 93%, 94%, 95%, 96%, 97%, 98%, 99%, or 100% with a sequence shown in any of SEQ ID NOs: 143-167 or SEQ ID NO: 87 or SEQ ID NO: 89; and a light chain including or consisting of any one selected from a group consisting of polypeptides having a sequence identity of at least 90%, 91%, 92%, 93%, 94%, 95%, 96%, 97%, 98%, 99%, or 100% with a sequence shown in any of SEQ ID NOs: 168-178 or SEQ ID NO: 88 or SEQ ID NO: 90.

Results show that, although the antibodies of the present disclosure, such as 51C10H4 (including a heavy chain shown in SEQ ID NO: 87 and a light chain shown in SEQ ID NO: 88) and 54B5A7 (including a heavy chain shown in SEQ ID NO: 95 and a light chain shown in SEQ ID NO: 120), exhibit an excellent binding affinity to CLDN6 and superior efficacy in tumor therapy, a recombination is conducted based on the generated and validated antibodies (such as, heavy chains, light chains, or CDRs of the antibodies) to maintain the desired properties. For example, heavy and light chains of the antibodies 51C10H4 and 54B5A7 are recombined to produce a novel improved chimeric monoclonal antibody RecA101. It has been validated that this improved chimeric monoclonal antibody RecA101 (including a heavy chain shown in SEQ ID NO: 87 and a light chain shown in SEQ ID NO: 120) also exhibits an excellent binding affinity to CLDN6 and superior efficacy in tumor therapy.

In the art, there are diversified strategies for modifying antibodies without altering the desired properties of the antibodies. In addition to the recombination described above, amino acid substitutions have been proven feasible in the present disclosure. For example, based on the antibody 51C10H4, M at a position 5 of light-chain CDR3 is substituted with N, V at a position 2 of heavy-chain CDR2 is substituted with I, S at a position 10 of heavy-chain CDR2 is substituted with N, and L at a position 10 of heavy-chain CDR3 is substituted with M, so as to produce an antibody named 54B5A7 in the present disclosure. It has been proved that both of the two antibodies retain the desired properties, including excellent binding affinity to CLDN6 and superior efficacy in tumor therapy.

For example, the sequences in the present disclosure, including chimeric or humanized antibody sequences, are subjected to conservative amino acid substitutions. For instance, when CDRs of the sequences in the present disclosure include a deamidation site NG (such as in CDR2 of 20A3-2A7 or CDR2 of 51C10H4), this site is mutated in various ways using diverse strategies. For example, G in NG can be mutated to A/S/D/Q, or N in NG can be mutated to S/Q/A, thereby removing an amino acid posing a deamidation risk. For instance, when CDRs of the sequences in the present disclosure include DG (such as in CDR3 of 51C10H4), this site is mutated in various ways using diverse strategies. For example, D in DG can be mutated to E/S/G, or G can be mutated to A/S/D/Q, thereby reducing the risk of an antigen-binding affinity decline caused by isomerization. The sequences in the present disclosure, including chimeric or humanized antibody sequences, are subjected to conservative amino acid substitutions. For instance, when CDRs of the sequences in the present disclosure include V (such as in CDR2 of PiH1004), this site is mutated in various ways using diverse strategies. For example, V can be mutated to A/I/L.

An antibody primarily interacts with a target antigen through amino acid residues located in six CDRs of heavy and light chains. Due to this reason, amino acid sequences within CDRs are more diverse than sequences outside the CDRs among different antibodies. Since CDR sequences are responsible for the majority of antibody-antigen interactions, it is possible to construct an expression vector for expressing a recombinant antibody that mimics the properties of a specific naturally occurring antibody. The expression vector includes CDR sequences derived from the specific naturally occurring antibody, which are grafted onto framework sequences derived from a different antibody with different properties (see, for example, Riechmann, L. et al. (1998) Nature 332: 323-327; Jones, P. et al. (1986) Nature 321: 522-525; and Queen, C. et al. (1989) Proc. Natl. Acad. Sci. U.S.A. 86: 10029-10033). Such framework sequences can be acquired from public DNA databases including germline antibody gene sequences. These germline antibody gene sequences differ from mature antibody gene sequences in that these germline antibody gene sequences do not include fully assembled variable genes and are produced through V(D)J recombination during maturation of B cells. These germline antibody gene sequences will also differ from sequences of high-affinity secondary repertoire antibodies at a specific site throughout a variable region.

Mouse antibodies demonstrate high immunogenicity in humans. When mouse antibodies are administered repeatedly, the therapeutic efficacy is reduced. The immunogenicity is primarily mediated by heavy-chain constant regions. If an individual mouse antibody is chimerized or humanized, the immunogenicity of the mouse antibody in humans can be reduced or entirely avoided.

A chimeric antibody refers to an antibody in which different parts are derived from different animal species, for example, an antibody with variable regions from a mouse antibody and constant regions from human Ig. The chimerization of an antibody is achieved by linking variable regions of heavy and light chains from a mouse antibody to constant regions of heavy and light chains from a human antibody (for example, as described by Kraus et al. in Methods in Molecular Biology series, Recombinant Antibodies for Cancer Therapy, ISBN-0-89603-918-8). In a preferred embodiment, a chimeric antibody is produced by linking constant regions of a human κ light chain to variable regions of a mouse light chain. In another preferred embodiment, a chimeric antibody is produced by linking constant regions of a human λ light chain to variable regions of a mouse light chain.

A humanized antibody refers to an antibody in which a CDR sequence/antigen-binding fragment or site derived from another mammalian species such as a mouse is grafted onto a human framework sequence.

To reduce the immunogenicity of the antibody in humans, a humanized anti-CLDN6 antibody is produced using sequences of the anti-CLDN6 antibody in the present disclosure. CDRs of a murine anti-CLDN6 antibody are combined with a human-derived framework region (for example, from human Ig) to produce the humanized anti-CLDN6 antibody in the present disclosure. The humanized anti-CLDN6 antibody is expected to retain the function to bind to human CLDN6 and monkey CLDN6.

In the present disclosure, through humanization and screening, 18 humanized antibody sequences with desired properties are obtained (each including a heavy chain shown in any of SEQ ID NOs: 149-160 and a light chain shown in any of SEQ ID NOs: 174-178). Specifically, PiH1001, PiH1002, PiH1003, PiH1004, PiH1005, PiH1006, PiH1007, and PiH1008 are desired humanized antibodies derived from the humanization of the chimeric antibody 51C10H4. CDRs of these desired humanized antibodies are identical to CDRs of 51C10H4. The humanized antibodies PiH1001, PiH1002, PiH1003, and PiH1004 are produced from the combination of a same light chain L3 with different heavy chains H1 to H4. The humanized antibodies PiH1005, PiH1006, PiH1007, and PiH1008 are produced from the combination of a same light chain L4 with different heavy chains H1 to H4. Specifically, PiH2001, PiH2004, PiH2005, PiH2006, PiH2007, PiH2008, PiH2009, PiH2010, PiH2011, and PiH2012 are desired humanized antibodies produced from the humanization of the chimeric antibody 54B5A7. CDRs of these desired humanized antibodies are identical to CDRs of 54B5A7. PiH2001 and PiH2004 are produced from the combination of a same light chain L3' with different heavy chains H1' and H4'. PiH2005, PiH2006, PiH2007, and PiH2008 are produced from the combination of a same light chain L4' with heavy chains H1', H2', H3', and H4'. PiH2009, PiH2010, PiH2011, and PiH2012 are produced from the combination of a same light chain L5 with different heavy chains H5, H6, H7, and H8. Results show that the humanized antibodies of the present disclosure exhibit reduced binding affinities to human CLDN6 and/or monkey CLDN6 compared with the corresponding chimeric antibodies prior to humanization, but still exhibit satisfactory binding affinities (such as binding affinity reflected by KD or binding affinity reflected by EC50), binding affinities to tumor cells, ADCC, CDC, and endocytosis that meet the requirements of the anti-CLDN6 antibody in the present disclosure.

### Antibody preparation or production

The antibody of the present disclosure can be produced through various techniques, including conventional monoclonal antibody techniques, such as the standard somatic hybridization technique of Kohler and Milstein, Nature 256: 495 (1975). Although the hybridoma technique is preferred, other methods for producing monoclonal antibodies may be adopted in principle, including transformation of B lymphocytes with a virus or an oncogene, phage display technology using an antibody gene library, somatic hybridization technique, and genetic engineering and recombination technique. For example, DNA molecules of genes encoding heavy and light chains of the antibody of the present disclosure are produced through chemical synthesis or PCR amplification and then inserted into an expression vector, a resulting vector is then transfected into a host cell, and a transfected host cell is cultured under specific conditions to express the antibody of the present disclosure.

Other preferred animal systems for preparing hybridomas secreting monoclonal antibodies are rat and rabbit systems (for example, as described in Spieker-Polet et al., Proc. Natl. Acad. Sci. U.S.A. 92: 9348 (1995); and Rossi et al., Am. J. Clin. Pathol. 124: 295 (2005)). Hybridoma production in mice is a highly established method. Immunization protocols and techniques for isolating immunized splenocytes for fusion are known in the art. Fusion partners (such as murine myeloma cells) and fusion methods are also known in the art.

Monoclonal antibodies can be prepared using a variety of techniques known in the art, including hybridoma technology, recombination technology, phage display technology, transgenic animals, or some combinations thereof. For example, monoclonal antibodies can be produced using hybridomas in combination with biochemical and genetic engineering techniques established in the art, as detailed in An, Zhigiang (ed.) Therapeutic Monoclonal Antibodies: From Bench to Clinic, John Wiley and Sons, 1st ed. 2009; Shire et al. (eds.) Current Trends in Monoclonal Antibody Development and Manufacturing, Springer Science + Business Media LLC, 1st ed. 2010; Harlow et al., Antibodies: A Laboratory Manual, Cold Spring Harbor Laboratory Press, 2nd ed. 1988; Hammerling et al., in: Monoclonal Antibodies and T-Cell Hybridomas 563-681 (Elsevier, N.Y., 1981), each of which is incorporated herein by reference in its entirety.

It should be understood that the selected binding sequences may be further modified, for example, to enhance an affinity for a target, humanize a target-binding sequence, improve the production in a cell culture, reduce the immunogenicity *in vivo,* generate a multispecific antibody, etc. Antibodies including such modified target-binding sequences also fall within the scope of the present disclosure. In a preferred embodiment, an anti-CLDN6 monoclonal antibody is prepared using a hybridoma.

To acquire a hybridoma producing the antibody of the present disclosure, such as the human monoclonal antibody of the present disclosure, a splenocyte and/or a lymph node cell may be isolated from an immunized mouse and fused to an appropriate immortalized cell line, such as a mouse myeloma cell line. The produced hybridomas are screened for the production of an antigen-specific antibody. The production of hybridomas is well known in the art, as shown, for example, in Harlow and Lane (1988) Antibodies, A Laboratory Manual, Cold Spring Harbor Publications, New York.

The antibody of the present disclosure may also be produced in a transfected host cell using, for example, a combination of a recombinant DNA technology and a gene transfection method well known in the art (for example, Morrison, S. (1985) Science 229: 1202). In some embodiments, DNAs encoding partial or full-length light and heavy chains that are produced by a standard molecular biology technique are inserted into one or more expression vectors, such that the genes are operably linked to transcriptional and translational regulatory sequences. In this context, the term "operably linked" means that genes for an antibody are inserted into a vector such that transcriptional and translational control sequences in the vector perform the intended functions of regulating the transcription and translation of the genes for the antibody.

A gene for a light chain of an antibody and a gene for a heavy chain of the antibody may be inserted into the same or different expression vectors. In some embodiments, variable regions are inserted into an expression vector that has already encoded a heavy-chain constant region and a light-chain constant region of a desired isotype to produce a full-length gene for an antibody of any isotype, such that VH is operably linked to CH in the vector and VL is operably linked to CL in the vector. Additionally or alternatively, a recombinant expression vector may encode a signal peptide that facilitates the secretion of an antibody chain by a host cell. A gene for the antibody chain may be cloned into the vector such that the signal peptide is linked to an amino terminus of the gene for the antibody chain. The signal peptide may be an Ig signal peptide or a heterologous signal peptide (namely, a signal peptide derived from a non-Ig protein).

To express light and heavy chains, an expression vector encoding the light and heavy chains is transfected into a host cell using a standard technique. Various forms of the term "transfection" are intended to encompass various techniques commonly used to introduce exogenous DNA into prokaryotic or eukaryotic host cells, including electroporation, calcium phosphate precipitation, DEAE-dextran-mediated transfection, etc. The antibody of the present disclosure can be expressed in prokaryotic or eukaryotic host cells such as mammalian host cells (which can assemble and secrete properly folded and immunologically active antibodies).

Mammalian host cells for expressing the recombinant antibody of the present disclosure include Chinese Hamster Ovary (CHO) cells (including dhfr-CHO cells described in Urlaub and Chasin, (1980) Proc. Natl. Acad. Sci. USA 77: 4216-4220) used with a dihydrofolate reductase (DHFR) selection marker (for example, as described in R. J. Kaufman and P. A. Sharp (1982) J. Mol. Biol. 159: 601-621), NSO myeloma cells, COS cells, and SP2 cells. In particular, an expression system adopted for NSO myeloma cells is the GS gene expression system disclosed in WO 87/04462, WO 89/01036, and EP 338,841. When a recombinant expression vector including a gene encoding an antibody is introduced into a mammalian host cell, the antibody is produced by culturing the host cell for a period of time sufficient to allow the expression of the antibody in the host cell or the secretion of the antibody into a medium in which the host cell grows. The antibody can be recovered from the medium using a standard protein purification method.

In another preferred embodiment, transgenic or transchromosomal mice carrying a partial human immune system (rather than a mouse system) may be used to produce a human anti-CLDN6 monoclonal antibody.

Another strategy for producing the monoclonal antibody involves the direct isolation of an antibody-encoding gene from an antibody-producing lymphocyte of a defined strategy, see, for example, Babcock et al., 1996; A novel strategy for generating monoclonal antibodies from single, isolated lymphocytes producing antibodies of defined strategy. Details on recombinant antibody engineering can also be seen in Welschof and Krau, Recombinant antibodies for cancer therapy ISBN-0-89603-918-8 and Benny K.C.Lo Antibody Engineering ISBN 1-58829-092-1.

To prepare a chimeric antibody, a variable region of murine Ig can be linked to a constant region of human Ig using a method known in the art (see, for example, U.S. Patent No. US4,816,567 of Cabilly et al.). A nucleic acid encoding VH can be operably linked to another DNA molecule encoding a heavy-chain constant region (CH1, CH2, and CH3) to convert the isolated nucleic acid encoding the VH into a full-length heavy-chain gene. Sequences of genes for human heavy-chain constant regions are known in the art (see, for example, Kabat et al. (1991), Sequences of Proteins of Immunological Interest, Fifth Edition, U.S. Department of Health and Human Services, NIH Publication No. 91-3242). A heavy-chain constant region may be a constant region of IgG1, IgG2, IgG3, IgG4, IgA, IgE, IgM, or IgD, and is preferably a constant region of IgG1 or IgG4. DNA encoding VL can be operably linked to another DNA molecule encoding a light-chain constant region CL to convert the isolated nucleic acid encoding the VL into a full-length light-chain gene (and a Fab light-chain gene). Sequences of genes for human light-chain constant regions are known in the art (see, for example, Kabat et al., ibid.). DNA fragments including these regions can be produced through standard PCR amplification. In a preferred embodiment, the light-chain constant region may be a κ or λ constant region, but is generally preferably a κ constant region. Once DNA fragments encoding VH and VL regions are obtained, these DNA fragments can be further manipulated by a standard recombinant DNA technique. For example, a gene for a variable region can be converted into a gene for a full-length antibody chain, a gene for an Fab fragment, or a gene for scFv. In such manipulations, a DNA fragment encoding VL or VH is operably linked to another DNA fragment encoding another protein, such as a constant region of an antibody or a flexible linker. As used herein, the term "operably linked" means that two DNA fragments are linked to make amino acid sequences encoded by the two DNA fragments remain in-frame.

To prepare a humanized antibody, murine CDR can be inserted into a human framework sequence by a method known in the art (see U.S. Patent No. US5,225,539 of Winter; U.S. Patent Nos. US5,530,101, US5,585,089, and US5,693,762 of Queen et al.; and Lo, Benny K.C., editor, in Antibody Engineering: Methods and Protocols, volume 248, Humana Press, New Jersey, 2004). Alternatively, transgenic animals may be utilized, which can generate a complete human antibody repertoire without producing endogenous Igs upon immunization. For example, it has been reported that the production of an endogenous antibody can be completely suppressed in chimeric and germline mutant mice through the homozygous deletion of a gene for a joining region of a heavy chain (JH) of the antibody, and then a human germline Ig gene array is introduced into such germline mutant mice, such that a human antibody will be generated in these mice upon an antigenic stimulation (see, for example, Jakobovits et al., 1993, Proc. Natl. Acad. Sci. USA 90: 2551; Jakobovits et al., 1993, Nature 362: 255-258; Bruggermann et al., 1993, Year in Immunology 7: 33; and Duchosal et al., 1992, Nature 355: 258). Non-limiting examples of the transgenic animals include HuMAb mouse (Medarex, Inc.), which carries human Ig miniloci encoding unrearranged human Ig heavy-chain (µ and γ) and κ light-chain sequences, and a targeted mutation inactivating endogenous loci for µ and κ chains (see, for example, Lonberg et al. (1994) Nature 368 (6474): 856-859); or "KM Mouse^{™}", which carries a human heavy-chain transgene and a human light-chain transchromosome (as shown in the patent application WO02/43478). Other methods for antibody humanization include phage display technology (Hoogenboom et al., 1991, J. Mol. Biol. 227: 381; Marks et al., J. Mol. Biol. 1991, 222: 581-597; and Vaughan et al., 1996, Nature Biotech 14: 309).

### Nucleic acid encoding the antibody of the present disclosure

In some aspects, the present disclosure relates to an isolated nucleic acid including a nucleic acid sequence encoding a heavy-chain variable region and/or a light-chain variable region of the isolated antibody disclosed herein.

The nucleic acid of the present disclosure can be produced using a standard molecular biology technique. For antibodies expressed by hybridomas (such as hybridomas generated from transgenic mice carrying human Ig genes further described below), cDNAs encoding light and heavy chains of an antibody produced by a hybridoma can be produced by a standard PCR amplification or cDNA cloning technique. Nucleic acids encoding antibodies obtained from an Ig gene library (for example, using phage display technology) can be recovered from the gene library.

To prepare a chimeric antibody, a variable region of murine Ig can be linked to a constant region of human Ig using a method known in the art (see, for example, U.S. Patent No. 4,816,567 of Cabilly et al.). A nucleic acid encoding VH can be operably linked to another DNA molecule encoding a heavy-chain constant region (CH1, CH2, and CH3) to convert the isolated nucleic acid encoding the VH into a full-length heavy-chain gene. DNA fragments including these regions can be produced through standard PCR amplification. DNA encoding VL can be operably linked to another DNA molecule encoding a light-chain constant region CL to convert the isolated nucleic acid encoding the VL into a full-length light-chain gene (and a Fab light-chain gene). Once DNA fragments encoding VH and VL regions are obtained, these DNA fragments can be further manipulated by a standard recombinant DNA technique. For example, a gene for a variable region can be converted into a gene for a full-length antibody chain, a gene for an Fab fragment, or a gene for scFv. In such manipulations, a DNA fragment encoding VL or VH is operably linked to another DNA fragment encoding another protein, such as a constant region of an antibody or a flexible linker.

In some embodiments, the present disclosure relates to an isolated nucleic acid including a nucleic acid sequence encoding a heavy-chain variable region of the isolated antibody disclosed herein.

In some embodiments, the present disclosure relates to an isolated nucleic acid including a nucleic acid sequence encoding a light-chain variable region of the isolated antibody disclosed herein.

In an aspect, the present disclosure provides a vector including the nucleic acid described above.

In an aspect, the present disclosure provides a host cell including the nucleic acid described above or the vector described above.

### Conjugate

In an aspect, the present disclosure provides a conjugate including the isolated anti-CLDN6 antibody or the antigen-binding fragment thereof described above that is conjugated to at least one detectable label. The detectable label includes, but is not limited to: (i) a label providing a detectable signal; (ii) a label interacting with a second label to modify a detectable signal provided by a first or second label, such as fluorescence resonance energy transfer (FRET); (iii) a label affecting mobility (such as electrophoretic mobility) through charge, hydrophobicity, shape, or other physical parameters; or (iv) a label providing a capture moiety, such as affinity and antibody/antigen or ionic complexation. A structure suitable as the label includes, for example, a fluorescent label, a luminescent label, a chromogenic label, a radioisotope label, an isotopic label (preferably a stable isotopic label), an isobaric label, an enzyme label (such as horseradish peroxidase, alkaline phosphatase, β-galactosidase, urease, and glucose oxidase), a particulate label (particularly a metal particle label, a magnetic particle label, and a polymer particle label), an organic small molecule (such as biotin and a ligand or binding molecule for a receptor (e.g., a cell adhesion protein or a lectin)), a label sequence that includes a nucleic acid and/or an amino acid residue and can be detected using a binding agent, etc. The label includes, but is not limited to, barium sulfate, iocetamic acid, iopanoic acid, calcium iopodate, sodium diatrizoate, meglumine diatrizoate, metrizamide, tyropanoate sodium, a radiodiagnostic agent (including a positron emitter (e.g., fluorine-18 and carbon-11), a γ emitter (e.g., iodine-123, iodine-125, technetium-99m, iodine-131, and indium-111), and a magnetic resonance nucleus (e.g., fluorine and gadolinium)), a luminescent substance (such as isoluminol and an acridinium ester), a fluorescent substance (such as fluorescein and rhodamine), and a colored substance (such as a latex particle and colloidal gold).

The detectable label can be detected by a method known in the art. For example, a radioactive label can be detected using a photographic film or a scintillation counter. A fluorescent label can be detected using a photodetector to measure emitted light. An enzyme label is typically detected by providing the enzyme with a substrate and measuring a product resulting from an interaction of the enzyme with the substrate. A thermal label is detected through simple visualization of the colored label. In some embodiments, the label is suitable for immunodetection (such as ELISA, RIA, fluoroimmunoassay, and chemiluminescence immunoassay). In some embodiments, the detectable label may be linked to the antibody or the antigen-binding fragment thereof of the present disclosure through linkers of varying lengths to reduce potential steric hindrance.

### Antibody-drug conjugate/immunoconjugate

In an aspect, the present disclosure provides an antibody-drug conjugate including an antibody. The antibody-drug conjugate includes one or more drug moieties, and the one or more drug moieties are linked to the isolated anti-CLDN6 antibody or the antigen-binding fragment thereof described above directly or indirectly through a linker (for example, covalent linking). In the antibody-drug conjugate of the present disclosure, a structure of the linker for conjugating the anti-CLDN6 antibody to a drug is not particularly limited, provided that the antibody-drug conjugate can be utilized.

The antibody-drug conjugate has the ability to selectively deliver one or more drugs to a target tissue (such as, a tumor-associated antigen, or a CLDN6- and/or CLDN9-expressing tumor). Thus, the antibody-drug conjugate can enhance the therapeutic efficacy of the antibody or the antigen-binding fragment thereof of the present disclosure in treating a disease (such as cancer).

### Multispecific molecule

The antibody or the antigen-binding fragment thereof of the present disclosure can be used to produce a multispecific molecule (such as a bispecific molecule). The antibody or the antigen-binding fragment thereof of the present disclosure may be a part of a multispecific molecule (such as a bispecific molecule). The bispecific or multispecific molecule includes a second functional module (such as a second antibody) or a third functional module (such as a third antibody) with different binding specificity from the antibody or the antigen-binding fragment thereof of the present disclosure, thereby enabling binding to at least two different binding sites and/or target molecules. For example, the antibody or the antigen-binding fragment thereof of the present disclosure may be linked to a second antibody or an antigen-binding fragment thereof capable of specifically binding to any protein that can serve as a potential target for combination therapy. To produce the bispecific or multispecific molecule, the antibody or the antigen-binding fragment thereof of the present disclosure may be linked (for example, through chemical conjugation, gene fusion, non-covalent association, or other modes) to one or more other binding molecules (such as an additional antibody, antibody fragment, peptide, or binding mimetic).

Therefore, in some aspects, the present disclosure provides a multispecific molecule including the antibody or the antigen-binding fragment thereof of the present disclosure.

In some preferred embodiments, the multispecific molecule specifically binds to CLDN6 (such as human CLDN6 or monkey CLDN6), and specifically binds to one or more additional targets.

In some preferred embodiments, the multispecific molecule further includes at least one molecule (such as a second antibody) with second binding specificity for a second target.

In some preferred embodiments, the multispecific molecule is a bispecific antibody.

### CAR

In an aspect, the present disclosure provides CAR including the isolated anti-CLDN6 antibody or the antigen-binding fragment thereof described above and an immune cell activation moiety. The immune cell activation moiety includes a transmembrane domain of an immune cell receptor and/or an intracellular signaling domain of the immune cell receptor.

### Drug conjugates

In an aspect, the present disclosure provides a targeted drug conjugate including the isolated anti-CLDN6 antibody or the antigen-binding fragment thereof described above. The targeted drug conjugate is RDC, and the RDC can be used for diagnosing or treating a disease.

In an aspect, the present disclosure provides an RNA drug including the isolated anti-CLDN6 antibody or the antigen-binding fragment thereof described above. The RNA drug is a nucleic acid-drug conjugate, and the nucleic acid-drug conjugate can be used for diagnosing or treating a disease. The nucleic acid-drug conjugate is produced by conjugating RNA to the isolated anti-CLDN6 antibody or the antigen-binding fragment thereof.

In an aspect, the present disclosure provides a targeted protein degrader-drug conjugate including the anti-CLDN6 antibody or the antigen-binding fragment thereof described above. The targeted protein degrader-drug conjugate is produced by conjugating a protein degrader to the anti-CLDN6 antibody or the antigen-binding fragment thereof through a chemical bond.

### Pharmaceutical composition

In some aspects, the present disclosure provides a pharmaceutical composition or kit, including the isolated anti-CLDN6 antibody or the antigen-binding fragment thereof described above, the nucleic acid described above, the vector described above, the host cell described above, the conjugate described above, the antibody-drug conjugate described above, the multispecific molecule described above, or the CAR described above; and a pharmaceutically acceptable carrier.

The pharmaceutical composition may optionally include one or more additional active pharmaceutical ingredients, such as another antibody or drug. The pharmaceutical composition of the present disclosure may also be administered in combination with, for example, an immunostimulant, an anti-cancer agent, an antiviral agent, or a vaccine, such that the anti-CLDN6 antibody enhances an immune response to the vaccine. The pharmaceutically acceptable carrier may include, for example, a pharmaceutically acceptable liquid, gel, or solid carrier; an aqueous medium and a non-aqueous medium; an antimicrobial agent, an isotonic agent, a buffer, an antioxidant, an anesthetic, a suspending/dispersing agent, a chelating agent, a diluent, an adjuvant, an excipient, or a non-toxic auxiliary substance; and a combination of various components known in the art.

Suitable components may include, for example, an antioxidant, a filler, a binder, a disintegrant, a buffer, a preservative, a lubricant, a flavoring agent, a thickening agent, a coloring agent, an emulsifier, or a stabilizer such as a sugar and a cyclodextrin. Suitable antioxidants may include, for example, methionine, ascorbic acid, ethylenediaminetetraacetic acid (EDTA), sodium thiosulfate, platinum, catalase, citric acid, cysteine, thioglycerol, thioglycolic acid, thiosorbitol, butyl methyl anisole, butylated hydroxytoluene, and/or propyl arsenate. As disclosed in the present disclosure, in a solvent including the pharmaceutical composition of the present disclosure that includes the antibody or the antigen-binding fragment thereof and one or more antioxidants such as methionine, the antibody or the antigen-binding fragment thereof may still be oxidized. The redox control can prevent or reduce the decline in binding affinity, thereby enhancing the stability and extending the shelf life for the antibody. Therefore, in some embodiments, the present disclosure provides a composition including one or more antibodies or antigen-binding fragments thereof and one or more antioxidants such as methionine. The present disclosure further provides a plurality of methods for mixing the antibody or the antigen-binding fragment thereof with one or more antioxidants such as methionine. Thus, the antibody or the antigen-binding fragment thereof can be protected from oxidation to extend the shelf life and/or enhance the activity.

For further illustration, the pharmaceutically acceptable carrier may include, for example, aqueous vehicles, such as sodium chloride injection, Ringer's injection, isotonic dextrose injection, sterile water injection, or dextrose and lactated Ringer's injection; non-aqueous vehicles, such as plant-derived non-volatile oils, cottonseed oil, corn oil, sesame oil, or peanut oil; antimicrobial agents in bacteriostatic or fungistatic concentrations; isotonic agents, such as sodium chloride or glucose; buffers, such as phosphate or citrate buffers; antioxidants, such as sodium bisulfate; local anesthetics, such as procaine hydrochloride; suspending and dispersing agents, such as sodium carboxymethylcellulose, hydroxypropyl methylcellulose, or polyvinylpyrrolidone; emulsifiers, such as polysorbate 80 (TWEEN-80); and sequestering or chelating agents, such as EDTA or ethylene glycol tetraacetic acid (EGTA), ethanol, polyethylene glycol, propylene glycol, sodium hydroxide, hydrochloric acid, citric acid, or lactic acid. An antimicrobial agent that serves as a carrier may be added to a pharmaceutical composition in a multi-dose container including a phenol or a cresol, a mercurial agent, benzyl alcohol, chlorobutanol, methylparaben and propylparaben, thimerosal, benzalkonium chloride, and benzethonium chloride. A suitable excipient may include, for example, water, saline, dextrose, glycerol, or ethanol. A suitable non-toxic auxiliary substance may include, for example, a wetting agent or an emulsifier, a pH buffer, a stabilizer, a solubility enhancer, or an agent such as sodium acetate, sorbitan monolaurate, triethanolamine oleate, or cyclodextrin.

### Administration, formulation, and dosage

The pharmaceutical composition of the present disclosure can be administered *in vivo* to a subject in need through various routes, including, but not limited to, oral, intravenous, intra-arterial, subcutaneous, parenteral, intranasal, intramuscular, intracranial, intracardiac, intraventricular, intratracheal, buccal, rectal, intraperitoneal, intradermal, topical, transdermal, and intrathecal administration routes, or implantation or inhalation. The pharmaceutical composition of the present disclosure may be prepared into a formulation in a solid, semi-solid, liquid, or gas form, including, but not limited to, a tablet, a capsule, a powder, a granule, an ointment, a solution, a suppository, an enema, an injection, an inhalant, and an aerosol. An appropriate formulation and administration route may be selected based on an intended application and therapeutic plan.

A proper formulation for enteral administration includes a hard or soft gelatin capsule, a pill, a tablet (including a coated tablet), an elixir, a suspension, a syrup, or an inhalant, and a controlled-release dosage form.

A formulation suitable for parenteral administration (such as injection) includes an aqueous or non-aqueous, isotonic, pyrogen-free, sterile liquid (such as a solution or a suspension) in which an active ingredient is dissolved, suspended, or present in other forms (for example, in a liposome or other microparticles). Such a liquid may additionally include other pharmaceutically acceptable ingredients, such as an antioxidant, a buffer, a preservative, a stabilizer, a bacteriostatic agent, a suspending agent, a thickening agent, and a solute that makes the formulation isotonic with blood (or other relevant body fluids) of an intended recipient. The excipient includes, for example, water, an alcohol, a polyol, glycerol, a vegetable oil, etc. Examples of an isotonic carrier suitable for such a formulation include sodium chloride injection, Ringer's solution, or lactated Ringer's injection. Similarly, a specific dosing regimen (including a dose, timing, and a frequency) depends on a specific individual, a medical history of the individual, and empirical considerations such as pharmacokinetics (such as half-life and clearance).

Requirements for an effective pharmaceutical carrier of an injectable formulation/composition are well known to those of ordinary skill in the art (see, for example, Pharmaceutics and Pharmacy Practice, J. B. Lippincott Company, Philadelphia, PA, edited by Banker and Chalmers, pp. 238-250 (1982), and ASHP Handbook on Injectable Drugs, Toissel, 4th ed., pp. 622-630 (1986)).

During a therapeutic process, an administration frequency may be determined and adjusted based on reducing the number of proliferating or tumorigenic cells, maintaining the reduction in tumor cells, inhibiting the proliferation of tumor cells, or delaying the development of metastases. In some embodiments, an administered dose may be adjusted or reduced to control potential side effects and/or toxicity. Alternatively, a sustained-release formulation may be suitable for the therapeutic composition of the present disclosure.

Those skilled in the art will appreciate that an appropriate dose may vary from patient to patient. Determining the optimal dose typically involves balancing a therapeutic benefit level against any risks or detrimental side effects. The selection of a dose level will depend on a variety of factors, including, but not limited to, an activity of a specific compound, administration route, administration time, clearance of the specific compound, treatment duration, other drugs, compounds, and/or materials that are co-administered with the specific compound, severity of a condition, a species, and a gender, an age, a body weight, a condition, a general health status, and a prior medical history of a patient. A dose and route of administration of the compound are ultimately determined by a physician, a veterinarian, or a clinician. The dose is typically selected to achieve a local concentration for a desired effect at an action site without causing substantial harmful or adverse side effects.

Generally, the antibody or the antigen-binding fragment thereof of the present disclosure may be administered within various ranges. These ranges include approximately 5 µg/kg of body weight to approximately 100 mg/kg of body weight per dose, approximately 50 µg/kg of body weight to approximately 5 mg/kg of body weight per dose, and approximately 100 µg/kg of body weight to approximately 10 mg/kg of body weight per dose. Other ranges include approximately 100 µg/kg of body weight to approximately 20 mg/kg of body weight per dose and approximately 0.5 mg/kg of body weight to approximately 20 mg/kg of body weight per dose. In some embodiments, the dose is at least approximately 100 µg/kg of body weight, at least approximately 250 µg/kg of body weight, at least approximately 750 µg/kg of body weight, at least approximately 3 mg/kg of body weight, at least approximately 5 mg/kg of body weight, and at least approximately 10 mg/kg of body weight.

In any case, the antibody or the antigen-binding fragment thereof of the present disclosure is preferably administered as required to a subject in need. Those skilled in the art can determine an administration frequency, for example, by a physician based on considerations such as a condition to be treated, an age of a subject to be treated, severity of the condition, and a general health status of the subject.

In some preferred embodiments, a therapeutic process involving the antibody or the antigen-binding fragment thereof of the present disclosure will include administering multiple doses of a selected drug product over a period of weeks or months. More specifically, the antibody or the antigen-binding fragment thereof of the present disclosure may be administered daily, every two days, every four days, weekly, every ten days, every two weeks, every three weeks, monthly, every six weeks, every two months, every ten weeks, or every three months. In this regard, it can be understood that the dose or interval for administration may be adjusted based on patient response and clinical practice.

A dose and regimen for the therapeutic composition of the present disclosure may also be determined empirically for an individual receiving one or more administrations. For example, the therapeutic composition produced herein may be administered to an individual at an incremental dose. In a selected embodiment, the dose may be gradually increased or decreased or mitigated based on experiences or observed side effects or toxicity. To evaluate the efficacy of a selected composition, a marker for a specific disease, disorder, or condition may be monitored as described previously. For cancers, evaluation methods include direct measurement of a tumor size by palpation or visual observation, or indirect measurement of a tumor size by X-ray imaging or other imaging techniques; evaluation of an improvement by direct tumor biopsy and microscopic examination of a tumor sample; measurement of an indirect tumor marker (such as prostate-specific antigen (PSA) for prostate cancer) or a tumorigenic antigen identified by the method described herein or alleviation in pain or paralysis; measurement of an improvement in tumor-associated speech, vision, respiration, or other disabilities; measurement of an increased appetite; or measurement of an improvement in a life quality or extension of survival by an accepted test. Those skilled in the art will appreciate that the dose will vary depending on an individual, a type of a tumor condition, a stage of the tumor condition, whether the tumor condition has begun to metastasize to other sites in the individual, and past and concurrent treatments.

A compatible formulation for parenteral administration (such as intravenous injection) includes the antibody or the antigen-binding fragment thereof disclosed herein at a concentration of approximately 10 µg/mL to approximately 100 mg/mL. In some selected embodiments, a concentration of the antibody or the antigen-binding fragment thereof includes 20 µg/mL, 40 µg/mL, 60 µg/mL, 80 µg/mL, 100 µg/mL, 200 µg/mL, 300 µg/mL, 400 µg/mL, 500 µg/mL, 600 µg/mL, 700 µg/mL, 800 µg/mL, 900 µg/mL, or 1 mg/mL. In other preferred embodiments, a concentration of the antibody-drug conjugate (ADC) includes 2 mg/mL, 3 mg/mL, 4 mg/mL, 5 mg/mL, 6 mg/mL, 8 mg/mL, 10 mg/mL, 12 mg/mL, 14 mg/mL, 16 mg/mL, 18 mg/mL, 20 mg/mL, 25 mg/mL, 30 mg/mL, 35 mg/mL, 40 mg/mL, 45 mg/mL, 50 mg/mL, 60 mg/mL, 70 mg/mL, 80 mg/mL, 90 mg/mL, or 100 mg/mL.

The anti-CLDN6 antibody of the present disclosure may be co-administered with one or more other therapeutic agents (such as a cytotoxic agent, a radiotoxic agent, an anti-tumor agent, an anti-angiogenic agent, and/or an immunosuppressive agent) to reduce the induction of an immune response against the anti-CLDN6 antibody of the present disclosure. The anti-CLDN6 antibody may be linked to the therapeutic agent (as an immunocomplex) or may be administered separately from the therapeutic agent. In the case of separate administration, the anti-CLDN6 antibody may be administered before, after, or concurrently with the therapeutic agent, or may be co-administered with other known treatments (such as anti-cancer therapy (including radiotherapy and chemotherapy).

In the context of therapeutic administration, the term "combination" or "coadministration" used herein refers to the use of more than one therapies or therapeutic agents. The use of the term "combination" does not restrict a sequence in which therapies or therapeutic agents are administered to a subject. A therapy or therapeutic agent may be administered before, concurrently with, or after the administration of another therapy or therapeutic agent to a patient. Preferably, therapies or therapeutic agents are administered to a subject in a specific sequence, at specific doses, and/or at specific time intervals such that the therapies or therapeutic agents can act together. In a specific embodiment, therapies or therapeutic agents are administered to a subject in a specific sequence, at specific doses, and/or at specific time intervals such that the therapies or therapeutic agents provide an increased benefit compared with the case where the therapies or therapeutic agents are administered in other ways (particularly independently of one another). Preferably, the increased benefit is a synergistic effect.

### Pharmaceutical use

The antibody, the antibody composition, and the method of the present disclosure have numerous *in vitro* and *in vivo* uses, including, for example, detection of CLDN6 or enhancement of an immune response. For example, these molecules may be administered to cells cultured *in vitro* or *ex vivo,* or for example, administered to a human subject.

Preferred subjects include mammals, such as humans/patients in need. Mammals in the context of the present disclosure include humans, non-human primates, domesticated animals such as dogs, cats, sheep, cattle, goats, pigs, and horses, laboratory animals such as mice, rats, rabbits, and guinea pigs, and captive animals such as animals in zoos.

The present disclosure further provides a method for detecting presence of a human CLDN6 antigen in a sample or measuring a content of the human CLDN6 antigen in the sample, including: The sample and a control sample are allowed to be in contact with a monoclonal antibody or an antigen-binding fragment thereof that specifically binds to human CLDN6 under conditions enabling formation of a complex of the monoclonal antibody or the antigen-binding fragment thereof with the human CLDN6. The formation of the complex is then detected. Differential complex formation between the sample and the control sample indicates the presence of the human CLDN6 antigen in the sample. Further, the anti-CLDN6 antibody of the present disclosure can be used to purify human CLDN6 through immunoaffinity purification. In some embodiments, the sample includes a cell.

### Treating conditions including cancers

In some aspects, the present disclosure provides a method for treating a condition in a mammal, including: a therapeutically effective amount of the antibody or the antigen-binding fragment thereof as disclosed herein is administered to a subject in need (such as a human). For example, the condition is a cancer.

In some aspects, the antibody of the present disclosure possesses one or more of the following activities: (i) killing a CLDN6-expressing cell; (ii) inhibiting proliferation of a CLDN6-expressing cell; (iii) inhibiting colony formation of a CLDN6-expressing cell; (iv) mediating regression (such as volume reduction) of a tumor, and preferably complete regression (such as complete disappearance); (v) preventing formation or reformation of a tumor; and (vi) inhibiting a metastasis of a CLDN6-expressing cell. Therefore, the antibody can be used for one or more of the aforementioned purposes (particularly when administered to a patient).

As described herein, the antibody of the present disclosure possesses one or more activities for therapeutically killing and/or inhibiting a cell. In particular, the activity for killing a cell, inhibiting proliferation of a cell, and/or inhibiting colony formation of a cell can be used to treat or prevent a cancer (including a cancer metastasis). The inhibition on proliferation, colony formation, and/or metastasis of a cell can be applied particularly to treating or preventing a metastasis of a cancer and metastatic spreading of a cancer cell.

In some aspects, the antibody of the present disclosure mediates killing of a cell (for example, a human CLDN6-expressing cell, including a tumor cell or a CLDN6-expressing tumor cell) by inducing ADCC-mediated lysis, apoptosis, homotypic adhesion, and/or phagocytosis.

In some aspects, the antibody of the present disclosure mediates killing of a cell (for example, a human CLDN6-expressing cell, including a tumor cell or a CLDN6-expressing tumor cell) by inducing CDC-mediated lysis, apoptosis, homotypic adhesion, and/or phagocytosis.

However, the present disclosure also includes embodiments where the antibody exerts the activities described herein (for example, killing a cell and/or inhibiting one or more cellular activities (including cell proliferation and/or colony formation)) without inducing CDC or ADCC-mediated lysis, apoptosis, homotypic adhesion, and/or phagocytosis.

The antibody of the present disclosure can interact with components of an immune system, preferably through an ADCC or CDC interaction. The antibody of the present disclosure may also function by simply binding to CLDN6 on a surface of a cell (for example, to block proliferation of the cell).

In some aspects, ADCC-mediated cell lysis occurs in the presence of an effector cell. In some specific embodiments, the effector cell is selected from a group consisting of a monocyte, a mononuclear cell, an NK cell, and a neutrophil (polymorphonuclear neutrophil (PMN)).

In some specific embodiments, the phagocytosis is carried out by a macrophage.

In some aspects, the antibody of the present disclosure exhibits one or more immune effector functions against a cell carrying CLDN6, particularly CLDN6 with a native conformation. The one or more immune effector functions are preferably selected from a group consisting of CDC, ADCC, induction of apoptosis, and inhibition of proliferation.

The antibody of the present disclosure can interact with components of an immune system, preferably through an ADCC or CDC interaction. The antibody of the present disclosure may also function by simply binding to CLDN6 on a surface of a cell (for example, to block proliferation of the cell).

In some aspects, the present disclosure provides a method for treating a disease associated with expression of CLDN6 or binding of CLDN6 to a surface of a cell or determining prognosis of the disease in a subject, including: an effective dose of the isolated anti-CLDN6 antibody or the antigen-binding fragment thereof, the nucleic acid, the vector, the host cell, the conjugate, the antibody-drug conjugate, the multispecific molecule, the CAR, or the pharmaceutical composition or kit is administered to the subject in need.

In some aspects, the present disclosure provides a use of the isolated anti-CLDN6 antibody or the antigen-binding fragment thereof, the nucleic acid, the vector, the host cell, the conjugate, the antibody-drug conjugate, the multispecific molecule, the CAR described above, or the pharmaceutical composition or kit for treating a disease associated with expression of CLDN6 or binding of CLDN6 to a surface of a cell or determining prognosis of the disease in a subject.

In some aspects, the present disclosure provides a use of the isolated anti-CLDN6 antibody or the antigen-binding fragment thereof, the nucleic acid, the vector, the host cell, the conjugate, the antibody-drug conjugate, the multispecific molecule, the CAR, or the pharmaceutical composition or kit in preparation of a reagent for treating a disease associated with CLDN6 expression or for determining prognosis of the disease.

According to the present disclosure, the "disease associated with expression of CLDN6 or binding of CLDN6 to a surface of a cell" means that the expression and binding of CLDN6 in a cell of a diseased tissue or organ is preferably elevated compared to a state in a healthy tissue or organ. The "elevation" refers to an elevation of at least 10%, particularly at least 20%, at least 50%, at least 100%, at least 200%, at least 500%, at least 1,000%, at least 10,000%, or even higher. Alternatively, the "elevation" refers to the detection of this indicator from absent to present, and subsequently, may optionally also include an elevation in the above numerical range. In an embodiment, the expression of CLDN6 and the binding of CLDN6 to a surface of a cell are observed only in a diseased tissue, but the expression of CLDN6 is suppressed in a healthy tissue. According to the present disclosure, the disease associated with expression of CLDN6 or binding of CLDN6 to a surface of a cell includes a neoplastic disease, such as a cancer. Further, according to the present disclosure, the neoplastic disease (such as a cancer) is preferably a disease in which a tumor or cancer cell expresses CLDN6 and is characterized by binding of CLDN6 to a surface of the tumor or cancer cell.

Various cancers involving CLDN6, whether malignant or benign and whether primary or secondary, can be treated or prevented using the method provided in the present disclosure. The cancer may be a solid tumor or a hematologic malignancy. Examples of the cancer include: lung cancer, such as bronchial cancer (e.g., squamous cell carcinoma, small cell carcinoma, large cell carcinoma, and adenocarcinoma), alveolar cell carcinoma, bronchial adenoma, chondroid hamartoma (non-cancerous), and sarcoma (cancerous); heart cancer such as myxoma, fibroma, and rhabdomyoma; bone cancer such as osteochondroma, chondroma, chondroblastoma, chondromyxoid fibroma, osteoid osteoma, giant cell tumor, chondrosarcoma, multiple myeloma, osteosarcoma, fibrosarcoma, malignant fibrous histiocytoma, Ewing's tumor (Ewing's sarcoma), and reticulum cell sarcoma; brain cancer such as glioma (e.g., glioblastoma multiforme), anaplastic astrocytoma, astrocytoma, oligodendroglioma, medulloblastoma, chordoma, schwannoma, ependymoma, meningioma, pituitary adenoma, pinealoma, osteoma, hemangioblastoma, craniopharyngioma, chordoma, germinoma, teratoma, dermoid cyst, and hemangioma; cancers of the digestive system, such as colon cancer, leiomyoma, epidermoid carcinoma, adenocarcinoma, leiomyosarcoma, gastric adenocarcinoma, intestinal lipoma, intestinal neurofibroma, intestinal fibroma, colorectal polyps, and colorectal cancer; liver cancer, such as hepatocellular adenoma, hemangioma, hepatocellular carcinoma, fibrolamellar carcinoma, cholangiocarcinoma, hepatoblastoma, and hemangiosarcoma; kidney cancer, such as renal adenocarcinoma, renal cell carcinoma, hypernephroma, and transitional cell carcinoma of the renal pelvis; bladder cancer; blood cancers, such as acute lymphocytic (lymphoblastic) leukemia, acute myeloid (myelocytic, myelogenous, myeloblastic, or myelomonocytic) leukemia, chronic lymphocytic leukemia (e.g., Sézary syndrome and hairy cell leukemia), chronic myeloid (myelocytic, myelogenous, or granulocytic) lymphoma, Hodgkin lymphoma, non-Hodgkin lymphoma, B-cell lymphoma, mycosis fungoides, and myeloproliferative disorders (e.g., polycythemia vera, myelofibrosis, thrombocythemia, and chronic myeloid leukemia); skin cancer, such as basal cell carcinoma, squamous cell carcinoma, melanoma, Kaposi's sarcoma, and Paget's disease; head and neck cancer; eye-related cancers, such as retinoblastoma and intraocular melanoma; cancers of the male reproductive system, such as benign prostatic hyperplasia, prostate cancer, and testicular cancer (e.g., seminoma, teratoma, embryonal carcinoma, and choriocarcinoma); breast cancer; cancers of the female reproductive system, such as uterine cancer (endometrial cancer), cervical cancer (cervical carcinoma), ovarian cancer (ovarian carcinoma), vulvar cancer, vaginal cancer, fallopian tube cancer, and hydatidiform mole; thyroid cancer (including papillary, follicular, anaplastic, or medullary carcinoma); pheochromocytoma (adrenal); non-cancerous growth of the parathyroid gland; pancreatic cancer; and hematologic cancers, such as leukemia, myeloma, non-Hodgkin lymphoma, and Hodgkin lymphoma. In a specific embodiment, the cancer is colon cancer.

In some embodiments, the disease associated with expression of CLDN6 is a cancer, and the cancer is preferably selected from a group consisting of ovarian cancer (e.g., ovarian adenocarcinoma and ovarian teratocarcinoma), lung cancer (such as small cell lung cancer and non-small cell lung cancer), gastric cancer, breast cancer, liver cancer, pancreatic cancer, skin cancer, malignant melanoma, head and neck cancer, sarcoma, cholangiocarcinoma, bladder cancer, kidney cancer, colorectal cancer, small intestine cancer, placental choriocarcinoma, cervical cancer, testicular cancer (such as testicular teratoma and embryonal testicular carcinoma), uterine cancer, and metastases thereof.

In some embodiments, the disease associated with expression of CLDN6 is a fibrotic disease or a fibrosis-driven cancer. The fibrotic disease includes a disease caused by fibrosis, a disease manifested as fibrosis, or a complication resulting from fibrosis.

In some embodiments, the fibrotic disease is selected from a group consisting of pulmonary fibrosis, hepatic fibrosis, colonic fibrosis, biliary fibrosis, and gastric fibrosis.

In some embodiments, the fibrotic disease is selected from a group consisting of hepatitis, pulmonary fibrosis, hepatic fibrosis, cirrhosis, scleroderma, and biliary atresia.

In some embodiments, the hepatitis includes non-alcoholic steatohepatitis (NASH).

In some embodiments, the pulmonary fibrosis includes idiopathic pulmonary fibrosis.

In some embodiments, the hepatic fibrosis includes alcohol- or drug-induced hepatic fibrosis, and infection-induced hepatic fibrosis.

According to the present disclosure, the terms "fibrosis" and "fibrotic disease" refer to a pathological condition characterized by excessive deposition of fibrous connective tissue in an organ or a tissue. More specifically, fibrosis is a pathological process that involves persistent fibrotic scar formation and excessive production of extracellular matrix by connective tissue, as responses to tissue injuries. Physiologically, the deposition of connective tissue can destroy the structural system and function of an organ or a tissue.

The function and effect of the antibody are evaluated by the inventors in a hepatic fibrosis model (such as a long-term choline-deficient L-amino acid-defined (CDAA) dietinduced hepatic fibrosis model and a CCl₄-induced liver injury model). Immunohistochemical staining results show that the antibody of the present disclosure can reduce the fibrosis of liver.

The inventors have also found the beneficial effect of the antibody of the present disclosure for treating biliary atresia by evaluating indicators such as body weight, survival rate, damage and fibrosis extent of a liver tissue, and cytokines closely associated with fibrosis in a biliary atresia mouse model (such as a Rhesus Rotavirus (RRV)-induced biliary atresia mouse model).

The antibody or the antigen-binding fragment thereof may be used alone as a monotherapy or in combination with a chemotherapy or a radiotherapy.

The antibody or the antigen-binding fragment thereof may be used in combination with an anti-cancer agent, a cytotoxic agent, or a chemotherapeutic agent.

The term "metastasis" indicates the spread of a cancer cell from an original site to other sites of a body. The metastasis is an extremely complex process in which a malignant cell detaches from a primary tumor, invades the extracellular matrix, penetrates the endothelial basement membrane to enter body cavities and blood vessels, and subsequently is transported through the bloodstream to infiltrate a target organ. The growth of a new tumor at a target site depends on angiogenesis. Tumor metastasis often occurs even after the removal of a primary tumor. This is because a tumor cell or component may remain and develop metastatic potential. In an embodiment, the term "metastasis" according to the present disclosure refers to "distant metastasis" indicating a metastasis away from a primary tumor and a regional lymph node system.

A cell of a secondary or metastatic tumor is similar to a cell of a primary tumor. This means that, for example, if ovarian cancer metastasizes to the liver, a secondary tumor consists of an abnormal ovarian cell (rather than an abnormal liver cell), and a resulting tumor in the liver is referred to as metastatic ovarian cancer (rather than liver cancer).

The term "anti-cancer agent" or "anti-proliferative agent" refers to any agent that can be used for treating a cell proliferative disorder such as a cancer. The anti-cancer agent or anti-proliferative agent includes, but is not limited to, a cytotoxic agent, a cytostatic agent, an anti-angiogenic agent, a radiotherapy and a radiotherapeutic agent, a targeted anti-cancer agent, a biologic response modifier (BRM), a therapeutic antibody, a cancer vaccine, a cytokine, a hormone therapy, a radiotherapy, an anti-metastatic agent, and an immunotherapeutic agent. It should be understood that, in the selected embodiments as described above, such an anti-cancer agent may include a conjugate and can bind to the disclosed site-specific antibody prior to administration. More specifically, in some embodiments, a selected anti-cancer agent is linked to unpaired cysteine of an engineered antibody to provide the engineered conjugate as described herein. Therefore, such an engineered conjugate is explicitly contemplated within the scope of the present disclosure. In other embodiments, the disclosed anti-cancer agent is administered in combination with a site-specific conjugate including the different therapeutic agents described above.

The present disclosure also provides a combination of the antibody or the antigen-binding fragment thereof with a radiotherapy (namely, any mechanism for locally inducing a DNA damage in a tumor cell, such as γ-irradiation, X-rays, ultraviolet (UV)-irradiation, microwaves, and electron emission). Combination therapies utilizing the targeted delivery of a radioisotope to a tumor cell are also considered. The disclosed conjugate may be used in conjunction with a targeted anti-cancer agent or other targeting modalities. Typically, the radiotherapy is administered in pulses over a period of about 1 week to about 2 weeks. The radiotherapy may be administered to a subject with head and neck cancer for approximately 6 weeks to 7 weeks. Optionally, the radiotherapy may be administered at a single dose or at multiple sequential doses.

### Diagnosis

The present disclosure provides *in vitro* and *in vivo* methods for detecting, diagnosing, or monitoring a proliferative disorder, and a method for screening cells from a patient to identify a tumor cell including a tumorigenic cell. Such methods include identifying an individual with a cancer to be treated or monitoring progression of a cancer. Such methods specifically includes: a patient or a sample *(in vivo* or *in vitro)* collected from the patient is allowed to be in contact with the antibody described herein, and the presence of the antibody or a binding level of the antibody to a bound or free target molecule in the sample is determined. In some embodiments, the antibody includes the detectable label disclosed herein or a reported molecule.

In some aspects, the present disclosure provides a method for diagnosing, detecting, or monitoring a disease associated with CLDN6 expression, including: an effective dose of the isolated anti-CLDN6 antibody or the antigen-binding fragment thereof, the nucleic acid, the vector, the host cell, the conjugate, the antibody-drug conjugate, the multispecific molecule, the CAR, or the pharmaceutical composition or kit is administered to a subject in need.

In some aspects, the present disclosure provides a use of the isolated anti-CLDN6 antibody or the antigen-binding fragment thereof, the nucleic acid, the vector, the host cell, the conjugate, the antibody-drug conjugate, the multispecific molecule, the CAR, or the pharmaceutical composition or kit for diagnosing, detecting, or monitoring a disease associated with CLDN6 expression in a subject.

In yet another aspect, the present disclosure provides a use of the isolated anti-CLDN6 antibody or the antigen-binding fragment thereof, the nucleic acid, the vector, the host cell, the conjugate, the antibody-drug conjugate, the multispecific molecule, the CAR, or the pharmaceutical composition or kit in preparation of a reagent for diagnosing, detecting, or monitoring a disease associated with expression of CLDN6 or binding of CLDN6 to a surface of a cell.

In some embodiments, binding of the antibody to a specific cell in the sample may mean that the sample potentially includes a tumorigenic cell, which indicates that an individual with a cancer may be effectively treated with the antibody described herein.

The sample may be analyzed by a variety of assays, such as RIA, enzyme immunoassay (such as ELISA), competitive binding assay, fluorescence immunoassay, immunoblot assay, Western blot analysis, and flow cytometry assay. Compatible *in vivo* diagnosis or diagnostic assays may include imaging or monitoring techniques well known in the art, such as magnetic resonance imaging, computerized tomography (such as computed axial tomography (CAT)), positron emission tomography (PET), radiography, and ultrasonography that are known to those skilled in the art.

The methods for *in vitro* detection or monitoring of expression of CLDN6 or an expression level of CLDN6 in a cell according to the present disclosure can be further used for non-diagnostic purposes.

Preferred subjects include mammals, such as humans/patients in need.

A sample from a subject is blood, excreta (urine or feces), an oral or nasal secretion, an alveolar lavage fluid, a tissue fluid, or a sweat from the subject, or an extract thereof.

### Pharmaceutical package and kit

The present disclosure also provides a pharmaceutical package or kit that has one or more containers including one or more doses of the antibody or the antigen-binding fragment thereof. In some embodiments, a unit dose is provided. The unit dose includes a predetermined amount of a composition. The composition includes, for example, the antibody or the antigen-binding fragment thereof, and includes or does not include one or more other agents. In other embodiments, the unit dose is supplied by a single-use pre-filled syringe for injection. In other embodiments, the composition included in the unit dose may include saline, sucrose, or an analogue; and a buffer such as a phosphate buffer, and/or may be prepared within a stable and effective pH range. Alternatively, in some embodiments, the conjugate composition may be provided as a lyophilized powder, which can be reconstituted upon addition of a suitable liquid (such as sterile water or a salt solution). In some preferred embodiments, the composition includes one or more substances inhibiting protein aggregation, including, but not limited to, sucrose and arginine. Any label on or associated with a container indicates that the encapsulated conjugate composition is intended for treating a selected neoplastic disease condition.

The present disclosure also provides a kit for producing a single-dose or multi-dose administration unit of a site-specific conjugate and optionally one or more anti-cancer agents. The kit includes a container and a label or package insert on or associated with the container. Suitable containers include, for example, a bottle, a vial, a syringe, etc. The container may be made of a variety of materials, such as glass or plastic, and includes a pharmaceutically effective amount of the disclosed conjugate in a conjugated or non-conjugated form. In other preferred embodiments, the container includes a sterile access port (for example, the container may be an intravenous infusion bag or a vial with a stopper pierceable by a subcutaneous injection needle). Such a kit typically includes a pharmaceutically acceptable formulation of an engineered conjugate in a suitable container, and optionally includes one or more anti-cancer agents in the same or a different container. The kit may further include another pharmaceutically acceptable formulation for diagnosis or a combination therapy. For example, in addition to the antibody or the antigen-binding fragment thereof of the present disclosure, such a kit may include any one or more anti-cancer agents, such as chemotherapeutic agents or radiotherapeutic agents, anti-angiogenic agents, anti-metastatic agents, targeted anti-cancer agents, cytotoxic agents, and/or other anti-cancer agents.

More specifically, the kit may have a single container that includes the disclosed antibody or antigen-binding fragment thereof and includes or does not include an additional component, or may have different containers for desired agents, respectively. In the case of providing combined therapeutic agents for conjugation, the combined therapeutic agents may be pre-mixed in equal molar masses or with one therapeutic agent in excess of another therapeutic agent to produce a single solution. Alternatively, the conjugate and any optional anti-cancer agent of the kit may be stored separately in different containers before being administered to a patient. The kit may further include a second/third container device for accommodating a sterile pharmaceutically acceptable buffer or other diluents, such as bacteriostatic water for injection (BWFI), PBS, Ringer's solution, and dextrose solution.

When components of the kit are provided in one or more liquid solutions, the liquid solution is preferably an aqueous solution and more preferably a sterile aqueous solution or a saline solution. Components of the kit may be provided as dry powders. When a reagent or a component is provided as a dry powder, the dry powder can be reconstituted by adding a suitable solvent. It is contemplated that the solvent may also be provided in an additional container.

As briefly mentioned above, the kit may also include a means for administering the antibody or the antigen-binding fragment thereof and any optional component to a patient, such as one or more needles, I.V. bags, or syringes, or even eyedroppers, transfer pipettes, or other similar devices. A formulation can be injected or introduced into an animal or administered to a diseased area of a body by the means. The kit of the present disclosure typically further includes a device for accommodating vials or analogues, and other tightly sealed parts for commercial sale, such as injection-molded or blow-molded plastic containers in which the required vials and other units are placed and maintained.

The present application is accompanied by sequence lists including numerous nucleic acid and amino acid sequences. Tables A, B, C, D, E, and F below provide an overview of the sequences involved in the present disclosure. The illustrative antibodies disclosed herein are anti-CLDN6 monoclonal antibodies.

**Table A: Amino acid sequences of CDRs**

| Antibody | | CDR1 | CDR2 | CDR3 |
|---|---|---|---|---|
| 15H2D4 | CDR H | SEQ ID NO:1 | SEQ ID NO:8 | SEQ ID NO:16 |
| | | SYNIH | YIYPGNGGTKYNERFKG | WRYYDRPTHYAM DY |
| | CDRL | SEQ ID NO:24 | SEQ ID NO:30 | SEQ ID NO:34 |
| | | RASKSVSTSGFSYL H | LASNLES | QHSREFPFT |
| 47D5D6 | CDR H | SEQ ID NO:2 | SEQ ID NO:9 | SEQ ID NO:17 |
| | | SYGMS | TISSGGSYTYYPDSVKG | PHYVSSYAMDY |
| | CDRL | SEQ ID NO:25 | SEQ ID NO:31 | SEQ ID NO:35 |
| | | KASEDIYNRLA | GATSLET | QQYWSTPPT |
| 58G3C7 | CDR H | SEQ ID NO:3 | SEQ ID NO:10 | SEQ ID NO:18 |
| | | NYAMS | TISDGGSYTSYPDNIKG | DAILITTAGFAY |
| | CDRL | SEQ ID NO:25 | SEQ ID NO:32 | SEQ ID NO:36 |
| | | KASEDIYNRLA | ATTSLET | QQYWSFPRT |
| 47A5B10 | CDR H | SEQ ID NO:4 | SEQ ID NO:11 | SEQ ID NO:19 |
| | | SYGIS | EIYPRSGNTYHNEKFKG | SSYDYYAMDY |
| | CDRL | SEQ ID NO:26 | SEQ ID NO:33 | SEQ ID NO:179 |
| | | SVSSIISSSKLH | GTSNLAS | QQWSSYPLT |
| 51C10H4 | CDR H | SEQ ID NO:5 | SEQ ID NO:12 | SEQ ID NO:20 |
| | | DYNMH | YVNPNNGATSYNQKFK G | WDGYVYYYGLDY |
| | CDRL | SEQ ID NO:27 | SEQ ID NO:31 | SEQ ID NO:37 |
| | | KASDHINNWLA | GATSLET | QQYWSTPYT |
| 54B5A7 | CDR H | SEQ ID NO:5 | SEQ ID NO:13 | SEQ ID NO:21 |
| | | DYNMH | YINPNNGATNYNQKFK G | WDGYVYYYGMDY |
| | CDRL | SEQ ID NO:27 | SEQ ID NO:31 | SEQ ID NO:38 |
| | | KASDHINNWLA | GATSLET | QQYWNTPYT |
| IMAB02 7 | CDR H | SEQ ID NO:6 | SEQ ID NO:14 | SEQ ID NO:22 |
| | | GYSFTGYT | INPYNGGTI | ARDYGFVLDY |
| | CDRL | SEQ ID NO:28 | / | SEQ ID NO:39 |
| | | SSVSY | STS | QQRSIYPPWT |
| 20A3-2A7 | CDR H | SEQ ID NO:7 | SEQ ID NO:15 | SEQ ID NO:23 |
| | | SYNMH | YIYPGNGGTNYNQKFK G | WKYYDKTTHYALD Y |
| | CDRL | SEQ ID NO:29 | SEQ ID NO:30 | SEQ ID NO:40 |
| | | RASKSVSTSGYSYI H | LASNLES | HHSRELPFT |
| RecA101 | CDR H | SEQ ID NO:5 | SEQ ID NO:12 | SEQ ID NO:20 |
| | | DYNMH | YVNPNNGATSYNQKFK G | WDGYVYYYGLDY |
| | CDRL | SEQ ID NO:27 | SEQ ID NO:31 | SEQ ID NO:38 |
| | | KASDHINNWLA | GATSLET | QQYWNTPYT |

**Table B: Amino acid sequences of variable regions**

| Antibody | VH | VL |
|---|---|---|
| 15H2D4 | SEQ ID NO:41 | SEQ ID NO:61 |
| | QAYLQQSGAELVRSGASVKMSCKAS GYTFTSYNIHWVKQTPGQGLEWIGYI YPGNGGTKYNERFKGKATLTADASSS TAYMQISSLTSEDSAVYFCARWRYYD RPTHYAMDYWGQGTSVTVSS | DIVVTQSPASLAVSLGQRATISCRASK SVSTSGFSYLHWFQQKPGQPPKLLIY LASNLESGVPARFSGSGSGTDFTLNIH PVEEEDVAVYYCQHSREFPFTFGSGT KLEIK |
| 47D5D6 | SEQ ID NO:42 | SEQ ID NO:62 |
| | EVQLVESGGDLVKPGGSLKLSCAASG FTFSSYGMSWVRQTPDKRLEWVATIS SGGSYTYYPDSVKGRFTISRDNAKNT LYLQMSSLKSEDTAMYYCARPHYVS SYAMDYWGQGTSVTVSS | DIQMTQSSSSFSVSLGDRVTITCKASE DIYNRLAWYQQKPGNAPRLLISGATS LETGVPSRFSGSGSGKDYTLSITSLQT EDVATYYCQQYWSTPPTFGGGTKLEI K |
| 58G3C7 | SEQ ID NO:43 | SEQ ID NO:63 |
| | EVQLVESGGGLVKPGGSLKLSCAASG FTFSNYAMSWVRQTPEKRLEWVATIS DGGSYTSYPDNIKGRFTISRDNAKNN LYLQVTHLKSEDTAMYYCARDAILIT TAGFAYWGQGTLLTVSA | DIQMTQSSSSFSVSLGDRVTITCKASE DIYNRLAWYQQKPGNAPRLLISATTS LETGVPSRFSGSGSGKDYTLSITSLQT EDVATYYCQQYWSFPRTFGGGTKLE VK |
| 47A5B10 | SEQ ID NO:44 | SEQ ID NO:64 |
| | QGQLQQSGAELARPGASVKLSCKAS GYTFTSYGISWVKQRTGQGLEWIGEI YPRSGNTYHNEKFKGKATLTADKSSS TAYMELRSLTSEDSAVYFCARSSYDY YAMDYWGQGTSVTVSS | EIVVTQSPALMAASPGEKVTITCSVSS IISSSKLHWYQQKSETSPKPWIYGTSN LASGVPIRFSGSGSGTSYSLTISSMEAE DAATYYCQQWSSYPLTFGAGTKLEL K |
| 51C10H4 | SEQ ID NO:45 | SEQ ID NO:65 |
| | EVQLQQSGPELVKPGASVKMSCKAS GYTFTDYNMHWVKKSHGKSLEWIGY VNPNNGATSYNQKFKGKATLTVNKS SSTAYMELRSLTSEDSAVYNCARWD GYVYYYGLDYWGQGTSVTVSS | DIQMTQSSSYLSVSLGGRVTITCKASD HINNWLAWYQQKPGNAPRLLISGATS LETGVPSRFSGSGSGKDYTLSITSLQT EDVATYYCQQYWSTPYTFGGGTKLEI K |
| 54B5A7 | SEQ ID NO:46 | SEQ ID NO:66 |
| | EVQLQQSGPELVKPGASVKMSCKAS GYTFTDYNMHWVKKSHGKSLEWIGY INPNNGATNYNQKFKGKATLTVNKSS STAYMELRSLTSEDSAVYNCARWDG YVYYYGMDYWGQGTSVTVSS | DIQMTQSSSYLSVSLGGRVTITCKASD HINNWLAWYQQKPGNAPRLLISGATS LETGVPSRFSGSGSGKDYTLSITSLQT EDVATYYCQQYWNTPYTFGGGTKLE IK |
| IMAB02 7 | SEQ ID NO:47 | SEQ ID NO:67 |
| | EVQLQQSGPELVKPGASMKISCKASG YSFTGYTMNWVKQSHGKNLEWIGLI NPYNGGTIYNQKFKGKATLTVDKSSS TAYMELLSLTSEDSAVYYCARDYGF VLDYWGQGTTLTVSS | IVLTQSPAIMSASPGEKVTITCSASSSV SYLHWFQQKPGTSPKLWVYSTSNLPS GVPARFGGSGSGTSYSLTISRMEAED AATYYCQQRSIYPPWTFGGGTKLEIK |
| 20A3-2A7 | SEQ ID NO:48 | SEQ ID NO:68 |
| | QAYLQQSGAELVRSGASVKMSCKAS GYTFTSYNMHWVKQTPGQGLEWIGY IYPGNGGTNYNQKFKGKATLTADTSS STAYMQISSLTSEDSAVYFCARWKYY DKTTHYALDYWGQGTLVTVSS | DIVLTQSPASLAVSLGQWATISCRAS KSVSTSGYSYIHWHQQKPGQPPKLLI YLASNLESGVPARFSGSGSGTDFTLNI HPVEEEDAATYYCHHSRELPFTFGSG TKLEIK |
| RecA101 | SEQ ID NO:45 | SEQ ID NO:66 |
| | EVQLQQSGPELVKPGASVKMSCKAS GYTFTDYNMHWVKKSHGKSLEWIGY VNPNNGATSYNQKFKGKATLTVNKS SSTAYMELRSLTSEDSAVYNCARWD GYVYYYGLDYWGQGTSVTVSS | DIQMTQSSSYLSVSLGGRVTITCKASD HINNWLAWYQQKPGNAPRLLISGATS LETGVPSRFSGSGSGKDYTLSITSLQT EDVATYYCQQYWNTPYTFGGGTKLE IK |
| PiH1001 (51-VH1+VL 3) | SEQ ID NO:49 | SEQ ID NO:69 |
| | QVQLVQSGAEVKKPGASVKVSCKAS GYTFTDYNMHWVRQAPGQGLEWIG YVNPNNGATSYNQKFKGRATMTRDT STSTVYMELSSLRSEDTAVYYCARW DGYVYYYGLDYWGQGTLVTVSS | DIQMTQSPSSLSASVGDRVTITCKASD HINNWLAWYQQKPGKVPKLLISGAT SLETGVPSRFSGSGSGTDYTLTISSLQP EDVATYYCQQYWSTPYTFGGGTKLEI K |
| PiH1002 (51-VH2+VL 3) | SEQ ID NO:50 | SEQ ID NO:69 |
| | QVQLVQSGAEVKKPGASVKVSCKAS GYTFTDYNMHWVRQAPGQGLEWIG YVNPNNGATSYNQKFKGRVTMTVDT STSTVYMELSSLRSEDTAVYNCARW DGYVYYYGLDYWGQGTLVTVSS | DIQMTQSPSSLSASVGDRVTITCKASD HINNWLAWYQQKPGKVPKLLISGAT SLETGVPSRFSGSGSGTDYTLTISSLQP EDVATYYCQQYWSTPYTFGGGTKLEI K |
| PiH1003 (51-VH3+VL 3) | SEQ ID NO:51 | SEQ ID NO:69 |
| | QVQLVQSGAEVKKPGASVKVSCKAS GYTFTDYNMHWVRQAPGQGLEWIG YVNPNNGATSYNQKFKGRATMTVDT STSTVYMELSSLRSEDTAVYNCARW DGYVVYYYGLDYWGQGTLVTVSS | DIQMTQSPSSLSASVGDRVTITCKASD HINNWLAWYQQKPGKVPKLLISGAT SLETGVPSRFSGSGSGTDYTLTISSLQP EDVATYYCQQYWSTPYTFGGGTKLEI K |
| PiH1004 (51-VH4+VL 3) | SEQ ID NO:52 | SEQ ID NO:69 |
| | QVQLVQSGAEVKKPGASVKVSCKAS GYTFTDYNMHWVRQAPGQSLEWIGY VNPNNGATSYNQKFKGRATMTVDKS TSTVYMELSSLRSEDTAVYNCARWD GYVYYYGLDYWGQGTLVTVSS | DIQMTQSPSSLSASVGDRVTITCKASD HINNWLAWYQQKPGKVPKLLISGAT SLETGVPSRFSGSGSGTDYTLTISSLQP EDVATYYCQQYWSTPYTFGGGTKLEI K |
| PiH1005 (51-VH1+VL 4) | SEQ ID NO:49 | SEQ ID NO:70 |
| | QVQLVQSGAEVKKPGASVKVSCKAS GYTFTDYNMHWVRQAPGQGLEWIG YVNPNNGATSYNQKFKGRATMTRDT STSTVYMELSSLRSEDTAVYYCARW DGYVYYYGLDYWGQGTLVTVSS | DIQMTQSPSSLSASVGGRVTITCKASD HINNWLAWYQQKPGKVPRLLISGATS LETGVPSRFSGSGSGTDYTLTISSLQP EDVATYYCQQYWSTPYTFGGGTKLEI K |
| PiH1006 (51-VH2+VL 4) | SEQ ID NO:50 | SEQ ID NO:70 |
| | QVQLVQSGAEVKKPGASVKVSCKAS GYTFTDYNMHWVRQAPGQGLEWIG YVNPNNGATSYNQKFKGRVTMTVDT STSTVYMELSSLRSEDTAVYNCARW DGYVYYYGLDYWGQGTLVTVSS | DIQMTQSPSSLSASVGGRVTITCKASD HINNWLAWYQQKPGKVPRLLISGATS LETGVPSRFSGSGSGTDYTLTISSLQP EDVATYYCQQYWSTPYTFGGGTKLEI K |
| PiH1007 (51-VH3+VL 4) | SEQ ID NO:51 | SEQ ID NO:70 |
| | QVQLVQSGAEVKKPGASVKVSCKAS GYTFTDYNMHWVRQAPGQGLEWIG YVNPNNGATSYNQKFKGRATMTVDT STSTVYMELSSLRSEDTAVYNCARW DGYVYYYGLDYWGQGTLVTVSS | DIQMTQSPSSLSASVGGRVTITCKASD HINNWLAWYQQKPGKVPRLLISGATS LETGVPSRFSGSGSGTDYTLTISSLQP EDVATYYCQQYWSTPYTFGGGTKLEI K |
| PiH1008 (51-VH4+VL 4) | SEQ ID NO:52 | SEQ ID NO:70 |
| | QVQLVQSGAEVKKPGASVKVSCKAS GYTFTDYNMHWVRQAPGQSLEWIGY VNPNNGATSYNQKFKGRATMTVDKS TSTVYMELSSLRSEDTAVYNCARWD GYVYYYGLDYWGQGTLVTVSS | DIQMTQSPSSLSASVGGRVTITCKASD HINNWLAWYQQKPGKVPRLLISGATS LETGVPSRFSGSGSGTDYTLTISSLQP EDVATYYCQQYWSTPYTFGGGTKLEI K |
| PiH2001 (54-VH1+VL 3) | SEQ ID NO:53 | SEQ ID NO:71 |
| | QVQLVQSGAEVKKPGASVKVSCKAS GYTFTDYNMHWVRQAPGQRLEWIGY INPNNGATNYNQKFKGRATITVDTSA STAYMELSSLRSEDTAVYYCARWDG YVYYYGMDYWGQGTLVTVSS | DIQMTQSPSSLSASVGDRVTITCKASD HINNWLAWYQQKPGKAPKLLISGAT SLETGVPSRFSGSGSGTDYTFTISSLQP EDIATYYCQQYWNTPYTFGGGTKLEI K |
| PiH2004 (54-VH4+VL 3) | SEQ ID NO:54 | SEQ ID NO:71 |
| | QVQLVQSGAEVKKPGASVKVSCKAS GYTFTDYNMHWVRQAPGQSLEWIGY INPNNGATNYNQKFKGRATITVDKSA STAYMELSSLRSEDTAVYNCARWDG YVYYYGMDYWGQGTLVTVSS | DIQMTQSPSSLSASVGDRVTITCKASD HINNWLAWYQQKPGKAPKLLISGAT SLETGVPSRFSGSGSGTDYTFTISSLQP EDIATYYCQQYWNTPYTFGGGTKLEI K |
| PiH2005 (54-VH1+VL 4) | SEQ ID NO:53 | SEQ ID NO:72 |
| | QVQLVQSGAEVKKPGASVKVSCKAS GYTFTDYNMHWVRQAPGQRLEWIGY INPNNGATNYNQKFKGRATITVDTSA STAYMELSSLRSEDTAVYYCARWDG YVYYYGMDYWGQGTLVTVSS | DIQMTQSPSSLSASVGGRVTITCKASD HINNWLAWYQQKPGKAPKLLISGAT SLETGVPSRFSGSGSGTDYTFTISSLQP EDIATYYCQQYWNTPYTFGGGTKLEI K |
| PiH2006 (54-VH2+VL 4) | SEQ ID NO:55 | SEQ ID NO:72 |
| | QVQLVQSGAEVKKPGASVKVSCKAS GYTFTDYNMHWVRQAPGQRLEWIGY INPNNGATNYNQKFKGRVTITVDTSA STAYMELSSLRSEDTAVYNCARWDG YVYYYGMDYWGQGTLVTVSS | DIQMTQSPSSLSASVGGRVTITCKASD HINNWLAWYQQKPGKAPKLLISGAT SLETGVPSRFSGSGSGTDYTFTISSLQP EDIATYYCQQYWNTPYTFGGGTKLEI K |
| PiH2007 (54-VH3+VL 4) | SEQ ID NO:56 | SEQ ID NO:72 |
| | QVQLVQSGAEVKKPGASVKVSCKAS GYTFTDYNMHWVRQAPGQSLEWIGY INPNNGATNYNQKFKGRATITVDTSA STAYMELSSLRSEDTAVYNCARWDG YVYYYGMDYWGQGTLVTVSS | DIQMTQSPSSLSASVGGRVTITCKASD HINNWLAWYQQKPGKAPKLLISGAT SLETGVPSRFSGSGSGTDYTFTISSLQP EDIATYYCQQYWNTPYTFGGGTKLEI K |
| PiH2008 (54-VH4+VL 4) | SEQ ID NO:54 | SEQ ID NO:72 |
| | QVQLVQSGAEVKKPGASVKVSCKAS GYTFTDYNMHWVRQAPGQSLEWIGY INPNNGATNYNQKFKGRATITVDKSA STAYMELSSLRSEDTAVYNCARWDG YVYYYGMDYWGQGTLVTVSS | DIQMTQSPSSLSASVGGRVTITCKASD HINNWLAWYQQKPGKAPKLLISGAT SLETGVPSRFSGSGSGTDYTFTISSLQP EDIATYYCQQYWNTPYTFGGGTKLEI K |
| PiH2009 (54-VH5+VL 5) | SEQ ID NO:57 | SEQ ID NO:73 |
| | QVQLVQSGAEVKKPGASVKVSCKAS GYTFTDYNMHWVRQATGQGLEWIG YINPNNGATNYNQKFKGRATMTRNK SISTAYMELRSLRSEDTAVYYCARWD | DIQMTQSPSSLSASVGDRVTITCKASD HINNWLAWYQQKPGKVPRLLISGATS LETGVPSRFSGSGSGKDYTLTISSLQP EDVATYYCQQYWNTPYTFGGGTKLE |
| | GYVYYYGMDYWGQGTLVTVSS | IK |
| PiH2010 (54-VH6+VL 5) | SEQ ID NO:58 | SEQ ID NO:73 |
| | QVQLVQSGAEVKKPGASVKVSCKAS GYTFTDYNMHWVRQATGQGLEWIG YINPNNGATNYNQKFKGRATMTRNK SISTAYMELRSLRSEDTAVYNCARWD GYVYYYGMDYWGQGTLVTVSS | DIQMTQSPSSLSASVGDRVTITCKASD HINNWLAWYQQKPGKVPRLLISGATS LETGVPSRFSGSGSGKDYTLTISSLQP EDVATYYCQQYWNTPYTFGGGTKLE IK |
| PiH2011 (54-VH7+VL 5) | SEQ ID NO:59 | SEQ ID NO:73 |
| | QVQLVQSGAEVKKPGASVKVSCKAS GYTFTDYNMHWVRQATGQGLEWIG YINPNNGATNYNQKFKGRATLTVNK SISTAYMELRSLRSEDTAVYYCARWD GYVYYYGMDYWGQGTLVTVSS | DIQMTQSPSSLSASVGDRVTITCKASD HINNWLAWYQQKPGKVPRLLISGATS LETGVPSRFSGSGSGKDYTLTISSLQP EDVATYYCQQYWNTPYTFGGGTKLE IK |
| PiH2012 (54-VH8+VL 5) | SEQ ID NO:60 | SEQ ID NO:73 |
| | QVQLVQSGAEVKKPGASVKVSCKAS GYTFTDYNMHWVRKATGQSLEWIGY INPNNGATNYNQKFKGRATMTRNKSI STAYMELRSLRSEDTAVYNCARWDG YVYYYGMDYWGQGTLVTVSS | DIQMTQSPSSLSASVGDRVTITCKASD HINNWLAWYQQKPGKVPRLLISGATS LETGVPSRFSGSGSGKDYTLTISSLQP EDVATYYCQQYWNTPYTFGGGTKLE IK |

**Table C: Amino acid sequences of CDRs in improved antibodies**

| Antibody | | CDR1 | CDR2 | CDR3 |
|---|---|---|---|---|
| 20A3-M1 | CDR H | SEQ ID NO:7 | SEQ ID NO:74 | SEQ ID NO:23 |
| | | SYNMH | YIYPGNAGTNYNQKFK G | WKYYDKTTHYALD Y |
| | CDR L | SEQ ID NO:29 | SEQ ID NO:30 | SEQ ID NO:40 |
| | | RASKSVSTSGYSYI H | LASNLES | HHSRELPFT |
| 51C10-M1 | CDR H | SEQ ID NO:5 | SEQ ID NO:75 | SEQ ID NO:20 |
| | | DYNMH | YVNPNNAATSYNQKFK G | WDGYVYYYGLDY |
| | CDR L | SEQ ID NO:27 | SEQ ID NO:31 | SEQ ID NO:37 |
| | | KASDHINNWLA | GATSLET | QQYWSTPYT |
| 51C10-M2 | CDR H | SEQ ID NO:5 | SEQ ID NO:76 | SEQ ID NO:20 |
| | | DYNMH | YVNPNQGATSYNQKFK G | WDGYVYYYGLDY |
| | CDR L | SEQ ID NO:27 | SEQ ID NO:31 | SEQ ID NO:37 |
| | | KASDHINNWLA | GATSLET | QQYWSTPYT |
| 51C10-M3 | CDR H | SEQ ID NO:5 | SEQ ID NO:12 | SEQ ID NO:77 |
| | | DYNMH | YVNPNNGATSYNQKFK G | WDAYVYYYGLDY |
| | CDR L | SEQ ID NO:27 | SEQ ID NO:31 | SEQ ID NO:37 |
| | | KASDHINNWLA | GATSLET | QQYWSTPYT |
| 51C10-M4 | CDR H | SEQ ID NO:5 | SEQ ID NO:12 | SEQ ID NO:78 |
| | | DYNMH | YVNPNNGATSYNQKFK G | WEGYVYYYGLDY |
| | CDR L | SEQ ID NO:27 | SEQ ID NO:31 | SEQ ID NO:37 |
| | | KASDHINNWLA | GATSLET | QQYWSTPYT |

**Table D: Amino acid sequences of variable regions in improved antibodies**

| Antibody | VH | VL |
|---|---|---|
| 20A3-M1 | SEQ ID NO:79 | SEQ ID NO:68 |
| | QAYLQQSGAELVRSGASVKMSCKAS GYTFTSYNMHWVKQTPGQGLEWIGY IYPGNAGTNYNQKFKGKATLTADTSS STAYMQISSLTSEDSAVYFCARWKYY DKTTHYALDYWGQGTLVTVSS | DIVLTQSPASLAVSLGQWATISCRASK SVSTSGYSYIHWHQQKPGQPPKLLIYL ASNLESGVPARFSGSGSGTDFTLNIHP VEEEDAATYYCHHSRELPFTFGSGTK LEIK |
| 51C10-M1 | SEQ ID NO:80 | SEQ ID NO:65 |
| | EVQLQQSGPELVKPGASVKMSCKAS | DIQMTQSSSYLSVSLGGRVTITCKASD |
| | GYTFTDYNMHWVKKSHGKSLEWIG YVNPNNAATSYNQKFKGKATLTVNK SSSTAYMELRSLTSEDSAVYNCARW DGYVYYYGLDYWGQGTSVTVSS | HINNWLAWYQQKPGNAPRLLISGATS LETGVPSRFSGSGSGKDYTLSITSLQT EDVATYYCQQYWSTPYTFGGGTKLEI K |
| 51C10-M2 | SEQ ID NO:81 | SEQ ID NO:65 |
| | EVQLQQSGPELVKPGASVKMSCKAS GYTFTDYNMHWVKKSHGKSLEWIG YVNPNQGATSYNQKFKGKATLTVNK SSSTAYMELRSLTSEDSAVYNCARW DGYVYYYGLDYWGQGTSVTVSS | DIQMTQSSSYLSVSLGGRVTITCKASD HINNWLAWYQQKPGNAPRLLISGATS LETGVPSRFSGSGSGKDYTLSITSLQT EDVATYYCQQYWSTPYTFGGGTKLEI K |
| 51C10-M3 | SEQ ID NO:82 | SEQ ID NO:65 |
| | EVQLQQSGPELVKPGASVKMSCKAS GYTFTDYNMHWVKKSHGKSLEWIG YVNPNNGATSYNQKFKGKATLTVNK SSSTAYMELRSLTSEDSAVYNCARW DAYVYYYGLDYWGQGTSVTVSS | DIQMTQSSSYLSVSLGGRVTITCKASD HINNWLAWYQQKPGNAPRLLISGATS LETGVPSRFSGSGSGKDYTLSITSLQT EDVATYYCQQYWSTPYTFGGGTKLEI K |
| 51C10-M4 | SEQ ID NO:83 | SEQ ID NO:65 |
| | EVQLQQSGPELVKPGASVKMSCKAS GYTFTDYNMHWVKKSHGKSLEWIG YVNPNNGATSYNQKFKGKATLTVNK SSSTAYMELRSLTSEDSAVYNCARWE GYVYYYGLDYWGQGTSVTVSS | DIQMTQSSSYLSVSLGGRVTITCKASD HINNWLAWYQQKPGNAPRLLISGATS LETGVPSRFSGSGSGKDYTLSITSLQT EDVATYYCQQYWSTPYTFGGGTKLEI K |

**Table E: IgG1 heavy-chain constant region, IgG1 light-chain constant region, and signal peptide adopted in a full-length sequence**

| Antibody | Sequence |
|---|---|
| IgG1 heavy-chain constant region | SEQ ID NO:84 |
| | |
| IgG1 light-chain constant region | SEQ ID NO:85 |
| | |
| Signal peptide | SEQ ID NO:86 |
| | MGWSCIILFLVATATGVHS |

**Table F: Various full-length sequences of exemplary 51C10H4**

| Antibody | Sequence |
|---|---|
| Full-length sequence of a heavy chain of 51C10H4 | SEQ ID NO:87 |
| | EVQLQQSGPELVKPGASVKMSCKASGYTFTDYNMHWVKKSHGKSLEWIGY VNPNNGATSYNQKFKGKATLTVNKSSSTAYMELRSLTSEDSAVYNCARWD GYVYYYGLDYWGQGTSVTVSSASTKGPSVFPLAPSSKSTSGGTAALGCLVK DYFPEPVTVSWNSGALTSGVHTFPAVLQSSGLYSLSSVVTVPSSSLGTQTYIC NVNHKPSNTKVDKKVEPKSCDKTHTCPPCPAPELLGGPSVFLFPPKPKDTLMI SRTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPSRD ELTKNQVSLTCLVKGFYPSDIAVEWESNGQPENNYKTTPPVLDSDGSFFLYS KLTVDKSRWQQGNVFSCSVMHEALHNHYTQKSLSLSPGK |
| Full-length sequence of a light chain of 51C10H4 | SEQ ID NO:88 |
| | DIQMTQSSSYLSVSLGGRVTITCKASDHINNWLAWYQQKPGNAPRLLISGAT SLETGVPSRFSGSGSGKDYTLSITSLQTEDVATYYCQQYWSTPYTFGGGTKLE IKRTVAAPSVFIFPPSDEQLKSGTASVVCLLNNFYPREAKVQWKVDNALQSG |
| | NSQESVTEQDSKDSTYSLSSTLTLSKADYEKHKVYACEVTHQGLSSPVTKSF NRGEC |
| Signal peptide-containing full-length sequence of the heavy chain of 51C10H4 | SEQ ID NO:89 |
| | MGWSCIILFLVATATGVHSEVQLQQSGPELVKPGASVKMSCKASGYTFTDY NMHWVKKSHGKSLEWIGYVNPNNGATSYNQKFKGKATLTVNKSSSTAYME LRSLTSEDSAVYNCARWDGYVVYYYGLDYWGQGTSVTVSSASTKGPSVFPLA PSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISK AKGQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQKSL SLSPGK |
| Signal peptide-containing full-length sequence of the light chain of 51C10H4H | SEQ ID NO:90 |
| | MGWSCIILFLVATATGVHSDIQMTQSSSYLSVSLGGRVTITCKASDHINNWLA WYQQKPGNAPRLLISGATSLETGVPSRFSGSGSGKDYTLSITSLQTEDVATYY CQQYWSTPYTFGGGTKLEIKRTVAAPSVFIFPPSDEQLKSGTASVVCLLNNFY PREAKVQWKVDNALQSGNSQESVTEQDSKDSTYSLSSTLTLSKADYEKHKV YACEVTHQGLSSPVTKSFNRGEC |

**Table G: Amino acid sequences of CDRs in improved antibodies**

| Antibody | | CDR1 | CDR2 | CDR3 |
|---|---|---|---|---|
| PiH1004-VH-Mutation6-VL | CDR H | SEQ ID NO:5 | SEQ ID NO:91 | SEQ ID NO:20 |
| | | DYNMH | YANPNNGATSYNQKFK G | WDGYVYYYGLDY |
| | CDRL | SEQ ID NO:27 | SEQ ID NO:31 | SEQ ID NO:37 |
| | | KASDHINNWLA | GATSLET | QQYWSTPYT |
| PiH1004-VH-Mutation7-VL | CDR H | SEQ ID NO:5 | SEQ ID NO:92 | SEQ ID NO:20 |
| | | DYNMH | YINPNNGATSYNQKFKG | WDGYVYYYGLDY |
| | CDRL | SEQ ID NO:27 | SEQ ID NO:31 | SEQ ID NO:37 |
| | | KASDHINNWLA | GATSLET | QQYWSTPYT |
| PiH1004-VH-Mutation8-VL | CDR H | SEQ ID NO:5 | SEQ ID NO:93 | SEQ ID NO:20 |
| | | DYNMH | YLNPNNGATSYNQKFK G | WDGYVYYYGLDY |
| | CDRL | SEQ ID NO:27 | SEQ ID NO:31 | SEQ ID NO:37 |
| | | KASDHINNWLA | GATSLET | QQYWSTPYT |
| PiH1004-VH-Mutation9-VL | CDR H | SEQ ID NO:5 | SEQ ID NO:94 | SEQ ID NO:20 |
| | | DYNMH | YVNPNNGASSYNQKFK G | WDGYVYYYGLDY |
| | CDRL | SEQ ID NO:27 | SEQ ID NO:31 | SEQ ID NO:37 |
| | | KASDHINNWLA | GATSLET | QQYWSTPYT |
| PiH1004-VH-Mutation10-VL | CDR H | SEQ ID NO:5 | SEQ ID NO:12 | SEQ ID NO:95 |
| | | DYNMH | YVNPNNGATSYNQKFK G | WDGYVYYYGVDY |
| | CDRL | SEQ ID NO:27 | SEQ ID NO:31 | SEQ ID NO:37 |
| | | KASDHINNWLA | GATSLET | QQYWSTPYT |
| PiH1004-VH-Mutation11-VL | CDR H | SEQ ID NO:5 | SEQ ID NO:12 | SEQ ID NO:96 |
| | | DYNMH | YVNPNNGATSYNQKFK G | WDGYVYYYGLDF |
| | CDRL | SEQ ID NO:27 | SEQ ID NO:31 | SEQ ID NO:37 |
| | | KASDHINNWLA | GATSLET | QQYWSTPYT |
| PiH1004-VH-Mutation14-VL | CDR H | SEQ ID NO:5 | SEQ ID NO:12 | SEQ ID NO:97 |
| | | DYNMH | YVNPNNGATSYNQKFK G | WDGYSYYYGLDY |
| | CDRL | SEQ ID NO:27 | SEQ ID NO:31 | SEQ ID NO:37 |
| | | KASDHINNWLA | GATSLET | QQYWSTPYT |
| PiH1004-VH-VL-Mutation1 | CDR H | SEQ ID NO:5 | SEQ ID NO:12 | SEQ ID NO:20 |
| | | DYNMH | YVNPNNGATSYNQKFK G | WDGYVYYYGLDY |
| | CDRL | SEQ ID NO:98 | SEQ ID NO:31 | SEQ ID NO:37 |
| | | KASEHINNWLA | GATSLET | QQYWSTPYT |
| PiH1004-VH-VL-Mutation2 | CDR H | SEQ ID NO:5 | SEQ ID NO:12 | SEQ ID NO:20 |
| | | DYNMH | YVNPNNGATSYNQKFK G | WDGYVYYYGLDY |
| | CDRL | SEQ ID NO:99 | SEQ ID NO:31 | SEQ ID NO:37 |
| | | KASDHVNNWL A | GATSLET | QQYWSTPYT |
| PiH1004-VH-VL-Mutation3 | CDR H | SEQ ID NO:5 | SEQ ID NO:12 | SEQ ID NO:20 |
| | | DYNMH | YVNPNNGATSYNQKFK G | WDGYVYYYGLDY |
| | CDRL | SEQ ID NO:100 | SEQ ID NO:31 | SEQ ID NO:37 |
| | | KASDHINQWLA | GATSLET | QQYWSTPYT |
| PiH1004-VH-VL-Mutation4 | CDR H | SEQ ID NO:5 | SEQ ID NO:12 | SEQ ID NO:20 |
| | | DYNMH | YVNPNNGATSYNQKFK G | WDGYVYYYGLDY |
| | CDRL | SEQ ID NO:27 | SEQ ID NO:101 | SEQ ID NO:37 |
| | | KASDHINNWLA | GGTSLET | QQYWSTPYT |
| PiH1004-VH-VL-Mutation5 | CDR H | SEQ ID NO:5 | SEQ ID NO:12 | SEQ ID NO:20 |
| | | DYNMH | YVNPNNGATSYNQKFK G | WDGYVYYYGLDY |
| | CDRL | SEQ ID NO:27 | SEQ ID NO:31 | SEQ ID NO:102 |
| | | KASDHINNWLA | GATSLET | QQYWSTPYS |
| PiH1004-VH-VL-Mutation6 | CDR H | SEQ ID NO:5 | SEQ ID NO:12 | SEQ ID NO:20 |
| | | DYNMH | YVNPNNGATSYNQKFK G | WDGYVYYYGLDY |
| | CDRL | SEQ ID NO:27 | SEQ ID NO:31 | SEQ ID NO:103 |
| | | KASDHINNWLA | GATSLET | QQYGSTPYT |
| PiH1004-VH-VL-Mutation7 | CDR H | SEQ ID NO:5 | SEQ ID NO:12 | SEQ ID NO:20 |
| | | DYNMH | YVNPNNGATSYNQKFK G | WDGYVYYYGLDY |
| | CDRL | SEQ ID NO:27 | SEQ ID NO:31 | SEQ ID NO:104 |
| | | KASDHINNWLA | GATSLET | QQYWHTPYT |

**Table H: Amino acid sequences of variable regions in improved antibodies**

| Antibody | VH | VL |
|---|---|---|
| PiH1004-VH-Mutation6-VL | SEQ ID NO:105 | SEQ ID NO:69 |
| | QVQLVQSGAEVKKPGASVKVSCKAS GYTFTDYNMHWVRQAPGQSLEWIG YANPNNGATSYNQKFKGRATMTVD KSTSTVYMELSSLRSEDTAVYNCAR WDGYVVYYYGLDYWGQGTLVTVSS | DIQMTQSPSSLSASVGDRVTITCK ASDHINNWLAWYQQKPGKVPKLL ISGATSLETGVPSRFSGSGSGTDYT LTISSLQPEDVATYYCQQYWSTPY TFGGGTKLEIK |
| PiH1004-VH-Mutation7-VL | SEQ ID NO:106 | SEQ ID NO:69 |
| | QVQLVQSGAEVKKPGASVKVSCKAS GYTFTDYNMHWVRQAPGQSLEWIG YINPNNGATSYNQKFKGRATMTVDK STSTVYMELSSLRSEDTAVYNCARW DGYVYYYGLDYWGQGTLVTVSS | DIQMTQSPSSLSASVGDRVTITCK ASDHINNWLAWYQQKPGKVPKLL ISGATSLETGVPSRFSGSGSGTDYT LTISSLQPEDVATYYCQQYWSTPY TFGGGTKLEIK |
| PiH1004-VH-Mutation8-VL | SEQ ID NO:107 | SEQ ID NO:69 |
| | QVQLVQSGAEVKKPGASVKVSCKAS GYTFTDYNMHWVRQAPGQSLEWIG YLNPNNGATSYNQKFKGRATMTVD KSTSTVYMELSSLRSEDTAVYNCAR WDGYVVYYYGLDYWGQGTLVTVSS | DIQMTQSPSSLSASVGDRVTITCK ASDHINNWLAWYQQKPGKVPKLL ISGATSLETGVPSRFSGSGSGTDYT LTISSLQPEDVATYYCQQYWSTPY TFGGGTKLEIK |
| PiH1004-VH-Mutation9-VL | SEQ ID NO:108 | SEQ ID NO:69 |
| | | DIQMTQSPSSLSASVGDRVTITCK |
| | GYTFTDYNMHWVRQAPGQSLEWIG YVNPNNGASSYNQKFKGRATMTVD KSTSTVYMELSSLRSEDTAVYNCAR WDGYVVYYYGLDYWGQGTLVTVSS | ASDHINNWLAWYQQKPGKVPKLL ISGATSLETGVPSRFSGSGSGTDYT LTISSLQPEDVATYYCQQYWSTPY TFGGGTKLEIK |
| PiH1004-VH-Mutation10-VL | SEQ ID NO:109 | SEQ ID NO:69 |
| | QVQLVQSGAEVKKPGASVKVSCKAS GYTFTDYNMHWVRQAPGQSLEWIG YVNPNNGATSYNQKFKGRATMTVD KSTSTVYMELSSLRSEDTAVYNCAR WDGYVVYYYGVDYWGQGTLVTVSS | DIQMTQSPSSLSASVGDRVTITCK ASDHINNWLAWYQQKPGKVPKLL ISGATSLETGVPSRFSGSGSGTDYT LTISSLQPEDVATYYCQQYWSTPY TFGGGTKLEIK |
| PiH1004-VH-Mutation11-VL | SEQ ID NO:110 | SEQ ID NO:69 |
| | QVQLVQSGAEVKKPGASVKVSCKAS GYTFTDYNMHWVRQAPGQSLEWIG YVNPNNGATSYNQKFKGRATMTVD KSTSTVYMELSSLRSEDTAVYNCAR WDGYVVYYYGLDFWGQGTLVTVSS | DIQMTQSPSSLSASVGDRVTITCK ASDHINNWLAWYQQKPGKVPKLL ISGATSLETGVPSRFSGSGSGTDYT LTISSLQPEDVATYYCQQYWSTPY TFGGGTKLEIK |
| PiH1004-VH-Mutation14-VL | SEQ ID NO:111 | SEQ ID NO:69 |
| | QVQLVQSGAEVKKPGASVKVSCKAS GYTFTDYNMHWVRQAPGQSLEWIG YVNPNNGATSYNQKFKGRATMTVD KSTSTVYMELSSLRSEDTAVYNCAR WDGYSYYYGLDYWGQGTLVTVSS | DIQMTQSPSSLSASVGDRVTITCK ASDHINNWLAWYQQKPGKVPKLL ISGATSLETGVPSRFSGSGSGTDYT LTISSLQPEDVATYYCQQYWSTPY TFGGGTKLEIK |
| PiH1004-VH-VL- Mutation1 | SEQ ID NO:52 | SEQ ID NO:112 |
| | QVQLVQSGAEVKKPGASVKVSCKAS GYTFTDYNMHWVRQAPGQSLEWIG YVNPNNGATSYNQKFKGRATMTVD KSTSTVYMELSSLRSEDTAVYNCAR WDGYVVYYYGLDYWGQGTLVTVSS | DIQMTQSPSSLSASVGDRVTITCK ASEHINNWLAWYQQKPGKVPKLL ISGATSLETGVPSRFSGSGSGTDYT LTISSLQPEDVATYYCQQYWSTPY TFGGGTKLEIK |
| PiH1004-VH-VL- Mutation2 | SEQ ID NO:52 | SEQ ID NO:113 |
| | QVQLVQSGAEVKKPGASVKVSCKAS GYTFTDYNMHWVRQAPGQSLEWIG YVNPNNGATSYNQKFKGRATMTVD KSTSTVYMELSSLRSEDTAVYNCAR WDGYVVYYYGLDYWGQGTLVTVSS | DIQMTQSPSSLSASVGDRVTITCK ASDHVNNWLAWYQQKPGKVPKL LISGATSLETGVPSRFSGSGSGTDY TLTISSLQPEDVATYYCQQYWSTP YTFGGGTKLEIK |
| PiH1004-VH-VL- Mutation3 | SEQ ID NO:52 | SEQ ID NO:114 |
| | QVQLVQSGAEVKKPGASVKVSCKAS GYTFTDYNMHWVRQAPGQSLEWIG YVNPNNGATSYNQKFKGRATMTVD KSTSTVYMELSSLRSEDTAVYNCAR WDGYVVYYYGLDYWGQGTLVTVSS | DIQMTQSPSSLSASVGDRVTITCK ASDHINQWLAWYQQKPGKVPKL LISGATSLETGVPSRFSGSGSGTDY TLTISSLQPEDVATYYCQQYWSTP YTFGGGTKLEIK |
| PiH1004-VH-VL- Mutation4 | SEQ ID NO:52 | SEQ ID NO:115 |
| | QVQLVQSGAEVKKPGASVKVSCKAS GYTFTDYNMHWVRQAPGQSLEWIG YVNPNNGATSYNQKFKGRATMTVD KSTSTVYMELSSLRSEDTAVYNCAR WDGYVVYYYGLDYWGQGTLVTVSS | DIQMTQSPSSLSASVGDRVTITCK ASDHINNWLAWYQQKPGKVPKLL ISGGTSLETGVPSRFSGSGSGTDYT LTISSLQPEDVATYYCQQYWSTPY TFGGGTKLEIK |
| PiH1004-VH-VL- Mutation5 | SEQ ID NO:52 | SEQ ID NO:116 |
| | QVQLVQSGAEVKKPGASVKVSCKAS GYTFTDYNMHWVRQAPGQSLEWIG YVNPNNGATSYNQKFKGRATMTVD KSTSTVYMELSSLRSEDTAVYNCAR WDGYVVYYYGLDYWGQGTLVTVSS | DIQMTQSPSSLSASVGDRVTITCK ASDHINNWLAWYQQKPGKVPKLL ISGATSLETGVPSRFSGSGSGTDYT LTISSLQPEDVATYYCQQYWSTPY SFGGGTKLEIK |
| PiH1004-VH-VL- Mutation6 | SEQ ID NO:52 | SEQ ID NO:117 |
| | QVQLVQSGAEVKKPGASVKVSCKAS GYTFTDYNMHWVRQAPGQSLEWIG YVNPNNGATSYNQKFKGRATMTVD KSTSTVYMELSSLRSEDTAVYNCAR WDGYVVYYYGLDYWGQGTLVTVSS | DIQMTQSPSSLSASVGDRVTITCK ASDHINNWLAWYQQKPGKVPKLL ISGATSLETGVPSRFSGSGSGTDYT LTISSLQPEDVATYYCQQYGSTPY TFGGGTKLEIK |
| PiH1004-VH- | SEQ ID NO:52 | SEQ ID NO:118 |
| VL- Mutation7 | QVQLVQSGAEVKKPGASVKVSCKAS GYTFTDYNMHWVRQAPGQSLEWIG YVNPNNGATSYNQKFKGRATMTVD KSTSTVYMELSSLRSEDTAVYNCAR WDGYVVYYYGLDYWGQGTLVTVSS | DIQMTQSPSSLSASVGDRVTITCK ASDHINNWLAWYQQKPGKVPKLL ISGATSLETGVPSRFSGSGSGTDYT LTISSLQPEDVATYYCQQYWHTPY TFGGGTKLEIK |

### Examples

The present disclosure, generally described herein, may be readily understood with reference to the following examples. These examples are provided by way of illustration and are not intended to limit the present disclosure. These examples are not intended to indicate that the experiments presented below are all or the only experiments.

### Example 1 Preparation of materials

### 1.1 Preparation of immunogens

### Immunogens adopted in the present disclosure include plasmid DNA and stable cell lines.

1.1.1 Genetic immunization bullets were prepared. A DNA sequence for human CLDN6 was cloned into a plasmid pCDH-CMV-MCS-EF1-Puro to produce a plasmid pCDH-CMV-MCS-EF1-Pur-CLDN6. The plasmid pCDH-CMV-MCS-EF1-Pur-CLDN6 was transformed into a cloning strain Stbl3. Plasmid amplification was conducted at 30°C. Plasmid DNA was extracted by a conventional method. Genetic immunization bullets were prepared using Bio-rad gold particles and the plasmid pCDH-CMV-MCS-EF1-Pur-CLDN6. Each genetic immunization bullet included approximately 3 µg of the plasmid DNA.

1.1.2 The DNA sequence for human CLDN6 was cloned into a plasmid pcDNA3.4. A target gene-containing plasmid was transfected into CHO-K1 cells. A stable cell line CHO-K1-CLDN6 expressing the target protein CLDN6 was constructed through screening.

### 1.2 Production of benchmark antibodies

In this example, a benchmark antibody IMAB027 was adopted as a positive control. The benchmark antibody IMAB027 was produced by linking variable region sequences of mAb206-LCC in U.S. Patent No. US14/117118 (CN103748112B) (see, SEQ ID NO: 47 and SEQ ID NO: 67 in Table B) to constant region sequences of human IgG1 (see, SEQ ID NO: 84 and SEQ ID NO: 85 in Table E).

In this example, a benchmark antibody AB3-7 was also adopted as a positive control. The benchmark antibody AB3-7 was produced by linking variable region sequences of an antibody 3-7 in Table D of Chinese Patent No. CN114174340A (see, SEQ ID NO: 387 and SEQ ID NO: 389 in Table D) to constant region sequences of human IgG1 (see, SEQ ID NO: 84 and SEQ ID NO: 85 in Table E).

### 1.3 Establishment of stable cell lines

Human CLDN6, mouse CLDN6, monkey CLDN6 (Cyno CLDN6), human CLDN3, human CLDN4, and human CLDN9 were overexpressed in HEK293T and CHO-K1 cells through lentiviral infection. After 72 h of cell infection, an antibiotic was added, and culturing was continued for 2 weeks to 4 weeks. Single colonies were picked, expanded, and cryopreserved to produce a total of 8 cell lines HEK293T-CLDN6, HEK293T-CLDN9, HEK293T-CLDN3, HEK293T-CLDN4, CHO-K1-CLDN9, CHO-K1-CLDN6, CHO-K1-Mouse CLDN6, and CHO-K1-Cyno CLDN6 for subsequent animal immunization and screening.

### Example 2 Production of antibodies

### 2.1 Immunization and cell fusion

### 2.1.1 Immunization

On day 0 and day 20, Balb/c mice (from Beijing Vital River Laboratory Animal Technology Co., Ltd.) were intraperitoneally injected with 3 µg of CLDN6-containing plasmid DNA for immunization. On day 40, day 54, day 63, and day 83, the Balb/c mice were intraperitoneally injected with 0.5 mL of a pcDNA3.4-CLDN6-transfected CHO-K1 cell (CHO-K1-CLDN6) solution at a concentration of 5 × 10⁶ cells/mL for immunization. According to an immunization schedule, on day 47 and day 70, blood was collected from the mandibular vein of an immunized mouse and tested for a serum titer. The serum titer was determined by FACS. Animals with a high serum titer were selected for splenocyte fusion. 3 d before the splenocyte fusion, mice exhibiting the highest antibody titer were selected and subjected to booster immunization.

### 2.1.2 Cell fusion

A spleen was collected from a mouse immunized multiple times and homogenized to produce a single-cell suspension. A single-cell suspension of myeloma cells (SP2/0) was prepared. Approximately 8 × 10⁷ splenocytes were electrofused with SP2/0 mouse myeloma cells. Fused cells were resuspended in an HAT medium (DMEM/10% fetal bovine serum (FBS) medium including hypoxanthine, aminopterin, and thymidine) including a hybridoma cell-selecting agent, transferred to a 96-well cell culture plate, and cultured at 37°C and 5.5% CO₂ for 6 d to 7 d to produce fused hybridoma cells capable of both expressing an antibody and infinitely proliferating *in vitro,* which were referred to as primary hybridoma cells. A binding ability of an antibody expressed in a hybridoma cell supernatant to CLDN6 was detected by FACS.

### 2.2 Screening of hybridoma cell supernatants

The hybridoma cell supernatants were screened by flow cytometry. CLDN6-overexpressing positive cells HEK293T-CLDN6 were used to identify a hybridoma cell supernatant with specific binding to CLDN6 through FACS analysis. Negative screening was conducted using HEK293T-CLDN9, HEK293T-CLDN3, HEK293T-CLDN4, and HEK293T. For a hybridoma supernatant producing a target clone, an antibody-secreting hybridoma was inoculated into a DMEM/10% FBS medium for secondary screening to produce a monoclonal hybridoma.

### 2.3 Hybridoma sequencing

The monoclonal hybridoma capable of specifically binding to both a CLDN6-overexpressing cell and a naturally CLDN6-expressing tumor cell obtained through the screening was subjected to sequencing. Variable regions of the monoclonal hybridoma were obtained through next-generation sequencing (NGS).

NGS: RNA was extracted from hybridoma cells and reverse-transcribed into cDNA, and IgG was amplified using antibody primers. A unique index sequence was added to each hybridoma antibody gene. All gene samples were pooled and sequenced on an NGS sequencer. Sequencing results were analyzed through bioinformatic analysis. IgG sequences were extracted and then traced back to the respective cell lines from which these sequences originate using index sequences.

### 2.4 Chimeric antibodies

The chimerization of an antibody was achieved by linking variable regions of heavy and light chains of a murine antibody to constant regions of human heavy and light chains (for example, as described by Kraus et al. in Methods in Molecular Biology series, Recombinant antibodies for cancer therapy ISBN-0-89603-918-8 or with reference to paragraph [0313] of CN111875703A).

In the present disclosure, the variable regions of the heavy and light chains of the murine antibody (as shown in Table B) were linked to constant regions of heavy and light chains of human IgG1 (as shown in Table E) to produce full-length sequences of a chimeric antibody (for example, as shown in Table F). Those skilled in the art should appreciate that a heavy-chain constant region for producing the chimeric antibody may be IgG1, IgG3, IgG4, IgG2, IgA, IgD, or IgM.

Full-length sequences of the antibodies in the present disclosure can be produced by linking the corresponding variable regions to constant regions of human IgG, such as an IgG1, IgG2, IgG3, or IgG4 constant region. Specifically, as an example, a heavy-chain variable region of each antibody (including the chimeric antibody, the humanized antibody, or the improved antibody) was linked to an IgG1 heavy-chain constant region to produce a full-length heavy-chain sequence of the antibody. Further, a signal peptide may be linked to the other end of the heavy-chain variable region to produce a signal peptide-containing full-length heavy-chain sequence. As an example, a light-chain variable region of each antibody (including the chimeric antibody, the humanized antibody, or the improved antibody) was linked to an IgG1 light-chain constant region to produce a full-length light-chain sequence of the antibody. Further, the signal peptide may be linked to the other end of the light-chain variable region to produce a signal peptide-containing full-length light-chain sequence.

Amino acid sequences of the IgG1 heavy-chain constant region, the IgG1 light-chain constant region, and the signal peptide in the present disclosure were shown in Table E. As an example, a full-length heavy-chain sequence, a signal peptide-containing full-length heavy-chain sequence, a full-length light-chain sequence, and a signal peptide-containing full-length light-chain sequence of 51C10H4 were shown in Table F. Based on the construction of the aforementioned full-length sequences in the present disclosure and the examples in Table F, full-length sequences of all chimeric antibodies, improved antibodies, and humanized antibodies in the present disclosure could be obtained.

For example, as described above, full-length sequences of humanized antibodies could be produced in the same manner by linking variable regions of humanized antibodies from Table B to the IgG1 constant regions from Table E. Specifically, a heavy-chain variable region of a humanized antibody was linked to the IgG1 heavy-chain constant region to produce a full-length heavy-chain sequence of the humanized antibody. Further, the signal peptide may be linked to the other end of the heavy-chain variable region to produce a signal peptide-containing full-length heavy-chain sequence. Specifically, a light-chain variable region of the humanized antibody was linked to the IgG1 light-chain constant region to produce a full-length light-chain sequence of the humanized antibody. Further, the signal peptide may be linked to the other end of the light-chain variable region to produce a signal peptide-containing full-length light-chain sequence.

24 h prior to transfection, Expi293F cells were cultured under normal conditions. For the transfection, an Expi293F cell suspension was diluted to 3 × 10⁶ cells/mL. A pre-warmed OptiPRO medium was added to two centrifuge tubes. A heavy-chain plasmid and a light-chain plasmid were added to one of the two centrifuge tubes, and a transfection reagent was added to the other one of the two centrifuge tubes. Thorough mixing was conducted immediately at room temperature to produce a complex. Incubation was conducted at room temperature for 1 min to 2 min. The complex was added to Expi293F cells cultured normally, and the Expi293F cells were further cultured in a 37°C and 8% CO₂ shaker for 18 h to 22 h. A transfection enhancer and a cell culture medium were added to cells. The cells were then further cultured in a 32°C and 5% CO₂ shaker. On day 4 of transfection, a cell culture medium was added to cells, and the cells were further cultured in a 32°C and 5% CO₂ shaker. On day 9 of transfection, a supernatant in a culture flask was collected and subjected to affinity purification using a Protein A column.

The anti-CLDN6 antibody of the present disclosure is a four-transmembrane protein with four transmembrane hydrophobic domains and two extracellular domains. It is extremely difficult to express a recombinant CLDN6 protein. Consequently, the preparation of a suitable protein antigen targeting an anti-CLDN6 antibody for both immunization and screening presents a significant challenge. Further, due to the high amino acid sequence homology between CLDN6 and family members thereof such as CLDN9, CLDN3, and CLDN4, it is considerably challenging to select monoclonal antibodies that specifically target CLDN6 or do not bind to at least one of the family members of CLDN6, which requires extensive screening efforts.

Specifically, at least 14,400 clones are selected in total through three rounds of screening in the present disclosure, including parental clone supernatant screening, subclone screening, and chimeric antibody screening. The anti-CLDN6 chimeric antibodies that all exhibit desired properties of CLDN6 binding, tumor cell binding, ADCC, CDC, and endocytosis in the present disclosure are finally obtained. On this basis, modification/improvement and/or screening are conducted to produce humanized antibodies.

### 2.5 Humanized antibodies

To reduce the immunogenicity of an antibody in humans, a humanized anti-CLDN6 antibody is produced using sequences of the anti-CLDN6 antibody selected in the present disclosure. CDRs of a murine anti-CLDN6 antibody are combined with a human-derived framework region (for example, from human Ig) to produce the humanized anti-CLDN6 antibody in the present disclosure. The humanized anti-CLDN6 antibody retains the function of binding to human CLDN6 and the function of binding to monkey CLDN6.

To prepare a humanized antibody, murine CDR can be inserted into a human framework sequence by a method well known in the art (see U.S. Patent No. US5,225,539 of Winter; U.S. Patent Nos. US5,530,101, US5,585,089, and US5,693,762 of Queen et al.; and Lo, Benny K.C., editor, in Antibody Engineering: Methods and Protocols, volume 248, Human Press, New Jersey, 2004).

In the present disclosure, full-length sequences of humanized antibodies can be produced by linking variable region sequences of the humanized antibodies from Table B to the IgG1 constant regions from Table E. For example, a heavy-chain variable region of a humanized antibody from Table B is linked to the IgG1 heavy-chain constant region from Table E, and a light-chain variable region of the humanized antibody from Table B is linked to the IgG1 light-chain constant region from Table E. Optionally, the signal peptide sequence shown in Table E may be further linked to a variable region.

### 2.6 Antibody improvement

Antibody improvement methods include recombination and conservative amino acid substitutions, which lead to improved antibodies with excellent/satisfactory CLDN6 binding properties and tumor-treating/inhibiting properties.

Specifically, as an example, in the present disclosure, a heavy chain of 51C10H4 is combined with a light chain of 54B5A7 to produce a novel improved chimeric monoclonal antibody RecA101.

Specifically, as an example, in the present disclosure, a deamidation site NG in CDR2 of 20A3-2A7 is mutated. G in NG is mutated into A to produce an improved antibody 20A3-M1 (including a heavy chain shown in SEQ ID NO: 161 and a light chain shown in SEQ ID NO: 173). It should be understood that this site NG can also undergo other mutations. For example, G in NG can be mutated into S/D/Q, and N in NG can be mutated into S/Q/A, so as to produce other improved antibodies.

Specifically, as an example, in the present disclosure, a deamidation site NG in CDR2 of 20A3-2A7 is mutated. G in NG is mutated into Q to produce an improved antibody 20A3-M2 (including a heavy chain shown in SEQ ID NO: 162 and a light chain shown in SEQ ID NO: 173). It should be understood that this site NG can also undergo other mutations. For example, G in NG can be mutated into S/D/A, and N in NG can be mutated into S/Q/A, so as to produce other improved antibodies.

Specifically, as an example, in the present disclosure, a deamidation site NG in CDR2 of 51C10H4 is mutated. G in NG is mutated into A to produce an improved antibody 51C10-M1 (including a heavy chain shown in SEQ ID NO: 163 and a light chain shown in SEQ ID NO: 88). It should be understood that this site NG can also undergo other mutations. For example, G in NG can be mutated into S/D/Q, and N in NG can be mutated into S/Q/A, so as to produce other improved antibodies.

Specifically, as an example, in the present disclosure, a deamidation site NG in CDR2 of 51C10H4 is mutated. N in NG is mutated into Q to produce an improved antibody 51C10-M2 (including a heavy chain shown in SEQ ID NO: 164 and a light chain shown in SEQ ID NO: 88).

Specifically, as an example, in the present disclosure, an isomerization site DG in CDR3 of 51C10H4 is mutated. G in DG is mutated into A to produce an improved antibody 51C10-M3 (including a heavy chain shown in SEQ ID NO: 165 and a light chain shown in SEQ ID NO: 88). It should be understood that this site DG can also undergo other mutations. For example, D in DG can be mutated into E/S/G, or G in DG can be mutated into A/S/D/Q, so as to produce other improved antibodies.

Specifically, as an example, in the present disclosure, an isomerization site DG in CDR3 of 51C10H4 is mutated. D in DG is mutated into E to produce an improved antibody 51C10-M4 (including a heavy chain shown in SEQ ID NO: 166 and a light chain shown in SEQ ID NO: 88).

It should be understood that, in this manner, identical or similar mutations can be conducted in the deamidation site NG-containing antibodies of the present disclosure, for example, in CDR2 of 15H2D4, CDR2 of 54B5A7, and CDR2 of RecA101. For example, G in NG is mutated into A/S/D/Q or N in NG is mutated into S/Q/A, so as to produce corresponding improved antibodies.

It should be understood that, in this manner, identical or similar mutations can be conducted in the isomerization site DG-containing antibodies of the present disclosure, for example, in CDR2 of 58G3C7, CDR3 of 54B5A7, CDR3 of RecA101, CDR3 of 51C10-M1, and CDR3 of 51C10-M2. For example, G in DG is mutated into A/S/D/Q or D in DG is mutated into E/S/G, so as to produce corresponding improved antibodies.

It should be understood that the aforementioned two sites, the deamidation site NG and the isomerization site DG, can be mutated simultaneously, and other hotspot regions known in the prior art for improving antibody properties can also be adopted for mutations in the antibodies of the present disclosure. It should be understood that the site-directed mutagenesis for improving antibodies in the present disclosure is not limited to the site-directed mutagenesis for improving the chimeric antibodies as described above. Similarly, in an identical or similar manner, site-directed mutagenesis can also be conducted in CDRs of humanized antibodies to produce corresponding improved humanized antibodies.

Specifically, as an example, in the present disclosure, a deamidation site NG in CDR2 of a heavy chain of PiH1004 is mutated. G in NG is mutated into A to produce an improved antibody PiH1004-M1 (including a heavy chain shown in SEQ ID NO: 167 and a light chain shown in SEQ ID NO: 174).

Specifically, as an example, in the present disclosure, V in CDR2 of a heavy chain of PiH1004 is mutated into A/I/L to produce corresponding improved antibodies (including: an improved antibody including a heavy-chain variable region shown in SEQ ID NO: 105 and a light-chain variable region shown in SEQ ID NO: 69; an improved antibody including a heavy-chain variable region shown in SEQ ID NO: 106 and a light-chain variable region shown in SEQ ID NO: 69; and an improved antibody including a heavy-chain variable region shown in SEQ ID NO: 107 and a light-chain variable region shown in SEQ ID NO: 69).

Specifically, as an example, in the present disclosure, T in CDR2 of the heavy chain of PiH1004 is mutated into S to produce a corresponding improved antibody (including a heavy-chain variable region shown in SEQ ID NO: 108 and a light-chain variable region shown in SEQ ID NO: 69).

Specifically, as an example, in the present disclosure, L in CDR3 of the heavy chain of PiH1004 is mutated into V to produce a corresponding improved antibody (including a heavy-chain variable region shown in SEQ ID NO: 109 and a light-chain variable region shown in SEQ ID NO: 69).

Specifically, as an example, in the present disclosure, Y in CDR3 of the heavy chain of PiH1004 is mutated into F to produce a corresponding improved antibody (including a heavy-chain variable region shown in SEQ ID NO: 110 and a light-chain variable region shown in SEQ ID NO: 69).

Specifically, as an example, in the present disclosure, V in CDR3 of the heavy chain of PiH1004 is mutated into S to produce a corresponding improved antibody (including a heavy-chain variable region shown in SEQ ID NO: 111 and a light-chain variable region shown in SEQ ID NO: 69).

Specifically, as an example, in the present disclosure, D in CDR1 of a light chain of PiH1004 is mutated into E to produce a corresponding improved antibody (including a heavy-chain variable region shown in SEQ ID NO: 52 and a light-chain variable region shown in SEQ ID NO: 112).

Specifically, as an example, in the present disclosure, I in CDR1 of the light chain of PiH1004 is mutated into V to produce a corresponding improved antibody (including a heavy-chain variable region shown in SEQ ID NO: 52 and a light-chain variable region shown in SEQ ID NO: 113).

Specifically, as an example, in the present disclosure, N in CDR1 of the light chain of PiH1004 is mutated into Q to produce a corresponding improved antibody (including a heavy-chain variable region shown in SEQ ID NO: 52 and a light-chain variable region shown in SEQ ID NO: 114).

Specifically, as an example, in the present disclosure, A in CDR2 of the light chain of PiH1004 is mutated into G to produce a corresponding improved antibody (including a heavy-chain variable region shown in SEQ ID NO: 52 and a light-chain variable region shown in SEQ ID NO: 115).

Specifically, as an example, in the present disclosure, T in CDR3 of the light chain of PiH1004 is mutated into S to produce a corresponding improved antibody (including a heavy-chain variable region shown in SEQ ID NO: 52 and a light-chain variable region shown in SEQ ID NO: 116).

Specifically, as an example, in the present disclosure, W in CDR3 of the light chain of PiH1004 is mutated into a hydrophobic aliphatic amino acid G to produce a corresponding improved antibody (including a heavy-chain variable region shown in SEQ ID NO: 52 and a light-chain variable region shown in SEQ ID NO: 117).

Specifically, as an example, in the present disclosure, an uncharged polar amino acid S in CDR3 of the light chain of PiH1004 is mutated into a positively charged polar amino acid G to produce a corresponding improved antibody (including a heavy-chain variable region shown in SEQ ID NO: 52 and a light-chain variable region shown in SEQ ID NO: 118).

Specifically, as an example, the present disclosure further includes the improved CDR sequences listed in Table I. Those skilled in the art may select different CDR sequences for combination to produce specific antibodies based on actual needs.

**Table I: Heavy-chain CDR sequences of improved antibodies**

| CDRH1 | SEQ ID NO: |
|---|---|
| DYNMH | 5 |

| CDRH2 | - |
|---|---|
| YANPNNGASSYNQKFKG | 119 |
| YANPNNGATSYNQKFKG | 91 |
| YINPNNGASSYNQKFKG | 120 |
| YINPNNGATSYNQKFKG | 92 |
| YLNPNNGASSYNQKFKG | 121 |
| YLNPNNGATSYNQKFKG | 93 |
| YVNPNNGASSYNQKFKG | 94 |
| YVNPNNGASNYNQKFKG | 122 |
| YINPNNGASNYNQKFKG | 123 |
| YLNPNNGATNYNQKFKG | 124 |
| YANPNNGATNYNQKFKG | 125 |
| YVNPNNGATSYNQKFKG | 12 |

| CDRH3 | - |
|---|---|
| WDGYVYYYGVDY | 95 |
| WDGYVYYYGVDF | 126 |
| WDGYSYYYGMDY | 127 |
| WDGYVYYYGMDF | 128 |
| WDGYVYYYGLDF | 96 |
| WDGYSYYYGVDY | 129 |
| WDGYSYYYGVDF | 130 |
| WDGYSYYYGLDY | 97 |
| WDGYSYYYGLDF | 131 |
| WDGYVYYYGLDY | 20 |

**Table J: Light-chain CDR sequences of improved antibodies**

| CDRL1 | SEQ ID NO: |
|---|---|
| KASEHVNNWLA | 132 |
| KASEHINQWLA | 133 |
| KASDHVNNWLA | 99 |
| KASEHVNQWLA | 134 |
| KASEHINNWLA | 98 |
| KASDHINQWLA | 100 |
| KASDHVNQWLA | 135 |
| KASDHINNWLA | 27 |

| CDRL2 | - |
|---|---|
| GGTSLET | 101 |
| GATSLET | 31 |

| CDRL3 | - |
|---|---|
| QQYWSTPYS | 102 |
| QQYWHTPYS | 136 |
| QQYWHTPYT | 104 |
| QQYGSTPYS | 137 |
| QQYGSTPYT | 103 |
| QQYGHTPYS | 138 |
| QQYGHTPYT | 139 |
| QQYWNTPYS | 140 |
| QQYGNTPYS | 141 |
| QQYGNTPYT | 142 |
| QQYWSTPYT | 37 |

**Table K: CDR amino acid sequences of improved antibodies**

| Antibody | | CDR1 | CDR2 | CDR3 |
|---|---|---|---|---|
| PiH1004-Mutation00 1 | CDR H | SEQ ID NO:5 | SEQ ID NO:119 | SEQ ID NO:20 |
| | | DYNMH | YANPNNGASSYNQKFK G | WDGYVYYYGLDY |
| | CDRL | SEQ ID NO:27 | SEQ ID NO:31 | SEQ ID NO:37 |
| | | KASDHINNWLA | GATSLET | QQYWSTPYT |
| PiH1004-Mutation00 2 | CDR H | SEQ ID NO:5 | SEQ ID NO:91 | SEQ ID NO:20 |
| | | DYNMH | YANPNNGATSYNQKFK G | WDGYVYYYGLDY |
| | CDRL | SEQ ID NO:99 | SEQ ID NO:101 | SEQ ID NO:37 |
| | | KASDHVNNWL A | GGTSLET | QQYWSTPYT |
| PiH1004-Mutation00 3 | CDR H | SEQ ID NO:5 | SEQ ID NO:91 | SEQ ID NO:20 |
| | | DYNMH | YANPNNGATSYNQKFK G | WDGYVYYYGLDY |
| | CDRL | SEQ ID NO:99 | SEQ ID NO:31 | SEQ ID NO:37 |
| | | KASDHVNNWL A | GATSLET | QQYWSTPYT |
| PiH1004-Mutation00 4 | CDR H | SEQ ID NO:5 | SEQ ID NO:91 | SEQ ID NO:20 |
| | | DYNMH | YANPNNGATSYNQKFK G | WDGYVYYYGLDY |
| | CDRL | SEQ ID NO:27 | SEQ ID NO:101 | SEQ ID NO:37 |
| | | KASDHINNWLA | GGTSLET | QQYWSTPYT |
| PiH1004-Mutation00 5 | CDR H | SEQ ID NO:5 | SEQ ID NO:12 | SEQ ID NO:20 |
| | | DYNMH | YVNPNNGATSYNQKFK G | WDGYVYYYGLDY |
| | CDRL | SEQ ID NO:99 | SEQ ID NO:101 | SEQ ID NO:37 |
| | | KASDHVNNW[?] A | GGTSLET | QQYWSTPYT |
| PiH 1004-Mutation00 6 | CDR H | SEQ ID NO:5 | SEQ ID NO:120 | SEQ ID NO:20 |
| | | DYNMH | YINPNNGASSYNQKFKG | WDGYVYYYGLDY |
| | CDRL | SEQ ID NO:27 | SEQ ID NO:31 | SEQ ID NO:37 |
| | | KASDHINNWLA | GATSLET | QQYWSTPYT |
| PiH1004-Mutation00 7 | CDR H | SEQ ID NO:5 | SEQ ID NO:120 | SEQ ID NO:20 |
| | | DYNMH | YINPNNGASSYNQKFKG | WDGYVYYYGLDY |
| | CDRL | SEQ ID NO:99 | SEQ ID NO:31 | SEQ ID NO:37 |
| | | KASDHVNNWL A | GATSLET | QQYWSTPYT |
| PiH1004-Mutation00 8 | CDR H | SEQ ID NO:5 | SEQ ID NO:92 | SEQ ID NO:20 |
| | | DYNMH | YINPNNGATSYNQKFKG | WDGYVYYYGLDY |
| | CDRL | SEQ ID NO:99 | SEQ ID NO:31 | SEQ ID NO:37 |
| | | KASDHVNNWL A | GATSLET | QQYWSTPYT |
| PiH1004-Mutation00 9 | CDR H | SEQ ID NO:5 | SEQ ID NO:92 | SEQ ID NO:20 |
| | | DYNMH | YINPNNGATSYNQKFKG | WDGYVYYYGLDY |
| | CDRL | SEQ ID NO:99 | SEQ ID NO:101 | SEQ ID NO:37 |
| | | KASDHVNNWL A | GGTSLET | QQYWSTPYT |
| PiH1004-Mutation01 0 | CDR H | SEQ ID NO:5 | SEQ ID NO:92 | SEQ ID NO:20 |
| | | DYNMH | YINPNNGATSYNQKFKG | WDGYVYYYGLDY |
| | CDRL | SEQ ID NO:27 | SEQ ID NO:101 | SEQ ID NO:37 |
| | | KASDHINNWLA | GGTSLET | QQYWSTPYT |
| PiH1004-Mutation01 1 | CDR H | SEQ ID NO:5 | SEQ ID NO:121 | SEQ ID NO:20 |
| | | DYNMH | YLNPNNGASSYNQKFK G | WDGYVYYYGLDY |
| | CDRL | SEQ ID NO:27 | SEQ ID NO:31 | SEQ ID NO:37 |
| | | KASDHINNWLA | GATSLET | QQYWSTPYT |
| PiH1004-Mutation01 2 | CDR H | SEQ ID NO:5 | SEQ ID NO:121 | SEQ ID NO:20 |
| | | DYNMH | YLNPNNGASSYNQKFK G | WDGYVYYYGLDY |
| | CDRL | SEQ ID NO:99 | SEQ ID NO:31 | SEQ ID NO:37 |
| | | KASDHVNNWL A | GATSLET | QQYWSTPYT |
| PiH1004-Mutation01 3 | CDR H | SEQ ID NO:5 | SEQ ID NO:121 | SEQ ID NO:20 |
| | | DYNMH | YLNPNNGASSYNQKFK G | WDGYVYYYGLDY |
| | CDRL | SEQ ID NO:99 | SEQ ID NO:101 | SEQ ID NO:37 |
| | | KASDHVNNWL A | GGTSLET | QQYWSTPYT |
| PiH1004-Mutation01 4 | CDR H | SEQ ID NO:5 | SEQ ID NO:121 | SEQ ID NO:20 |
| | | DYNMH | YLNPNNGASSYNQKFK G | WDGYVYYYGLDY |
| | CDRL | SEQ ID NO:27 | SEQ ID NO:101 | SEQ ID NO:37 |
| | | KASDHINNWLA | GGTSLET | QQYWSTPYT |
| PiH1004-Mutation01 5 | CDR H | SEQ ID NO:5 | SEQ ID NO:93 | SEQ ID NO:20 |
| | | DYNMH | YLNPNNGATSYNQKFK G | WDGYVYYYGLDY |
| | CDRL | SEQ ID NO:99 | SEQ ID NO:31 | SEQ ID NO:37 |
| | | KASDHVNNWL A | GATSLET | QQYWSTPYT |
| | | | | |
| | | | | |
| PiH1004-Mutation01 6 | CDR H | SEQ ID NO:5 | SEQ ID NO:93 | SEQ ID NO:20 |
| | | DYNMH | YLNPNNGATSYNQKFK G | WDGYVYYYGLDY |
| | CDRL | SEQ ID NO:27 | SEQ ID NO:101 | SEQ ID NO:37 |
| | | KASDHINNWLA | GGTSLET | QQYWSTPYT |
| PiH 1004-Mutation01 7 | CDR H | SEQ ID NO:5 | SEQ ID NO:93 | SEQ ID NO:20 |
| | | DYNMH | YLNPNNGATSYNQKFK G | WDGYVYYYGLDY |
| | CDRL | SEQ ID NO:99 | SEQ ID NO:101 | SEQ ID NO:37 |
| | | KASDHVNNWL A | GGTSLET | QQYWSTPYT |
| PiH1004-Mutation01 8 | CDR H | SEQ ID NO:5 | SEQ ID NO:94 | SEQ ID NO:20 |
| | | DYNMH | YVNPNNGASSYNQKFK G | WDGYVYYYGLDY |
| | CDRL | SEQ ID NO:99 | SEQ ID NO:31 | SEQ ID NO:37 |
| | | KASDHVNNWL A | GATSLET | QQYWSTPYT |
| PiH1004-Mutation01 9 | CDR H | SEQ ID NO:5 | SEQ ID NO:94 | SEQ ID NO:20 |
| | | DYNMH | YVNPNNGASSYNQKFK G | WDGYVYYYGLDY |
| | CDRL | SEQ ID NO:99 | SEQ ID NO:101 | SEQ ID NO:37 |
| | | KASDHVNNWL A | GGTSLET | QQYWSTPYT |
| PiH1004-Mutation02 3 | CDR H | SEQ ID NO:5 | SEQ ID NO:94 | SEQ ID NO:20 |
| | | DYNMH | YVNPNNGASSYNQKFK G | WDGYVYYYGLDY |
| | CDRL | SEQ ID NO:27 | SEQ ID NO:101 | SEQ ID NO:37 |
| | | KASDHINNWLA | GGTSLET | QQYWSTPYT |
| PiH1004-Mutation02 4 | CDR H | SEQ ID NO:5 | SEQ ID NO:122 | SEQ ID NO:20 |
| | | DYNMH | YVNPNNGASNYNQKFK G | WDGYVYYYGLDY |
| | CDRL | SEQ ID NO:27 | SEQ ID NO:31 | SEQ ID NO:37 |
| | | KASDHINNWLA | GATSLET | QQYWSTPYT |
| PiH1004-Mutation02 5 | CDR H | SEQ ID NO:5 | SEQ ID NO:122 | SEQ ID NO:20 |
| | | DYNMH | YVNPNNGASNYNQKFK G | WDGYVYYYGLDY |
| | CDRL | SEQ ID NO:99 | SEQ ID NO:31 | SEQ ID NO:37 |
| | | KASDHVNNWL A | GATSLET | QQYWSTPYT |
| PiH1004-Mutation02 6 | CDR H | SEQ ID NO:5 | SEQ ID NO:122 | SEQ ID NO:20 |
| | | DYNMH | YVNPNNGASNYNQKFK G | WDGYVYYYGLDY |
| | CDRL | SEQ ID NO:99 | SEQ ID NO:101 | SEQ ID NO:37 |
| | | KASDHVNNWL A | GGTSLET | QQYWSTPYT |
| PiH1004-Mutation02 7 | CDR H | SEQ ID NO:5 | SEQ ID NO:124 | SEQ ID NO:95 |
| | | DYNMH | YLNPNNGATNYNQKFK G | WDGYVYYYGVDY |
| | CDRL | SEQ ID NO:27 | SEQ ID NO:31 | SEQ ID NO:37 |
| | | KASDHINNWLA | GATSLET | QQYWSTPYT |
| PiH1004-Mutation02 8 | CDR H | SEQ ID NO:5 | SEQ ID NO:124 | SEQ ID NO:128 |
| | | DYNMH | YLNPNNGATNYNQKFK G | WDGYVYYYGMDF |
| | CDRL | SEQ ID NO:27 | SEQ ID NO:31 | SEQ ID NO:37 |
| | | KASDHINNWLA | GATSLET | |
| PiH1004-Mutation03 1 | CDR H | SEQ ID NO:5 | SEQ ID NO:124 | SEQ ID NO:96 |
| | | DYNMH | YLNPNNGATNYNQKFK G | WDGYVYYYGLDF |
| | | | | |
| | CDRL | SEQ ID NO:27 | SEQ ID NO:31 | SEQ ID NO:37 |
| | | KASDHINNWLA | GATSLET | QQYWSTPYT |
| PiH 1004-Mutation03 2 | CDR H | SEQ ID NO:5 | SEQ ID NO:124 | SEQ ID NO:20 |
| | | DYNMH | YLNPNNGATNYNQKFK G | WDGYVYYYGLDY |
| | CDRL | SEQ ID NO:99 | SEQ ID NO:31 | SEQ ID NO:37 |
| | | KASDHVNNWL A | GATSLET | QQYWSTPYT |
| PiH1004-Mutation03 3 | CDR H | SEQ ID NO:5 | SEQ ID NO:124 | SEQ ID NO:20 |
| | | DYNMH | YLNPNNGATNYNQKFK G | WDGYVYYYGLDY |
| | CDRL | SEQ ID NO:99 | SEQ ID NO:101 | SEQ ID NO:37 |
| | | KASDHVNNWL A | GGTSLET | QQYWSTPYT |
| PiH1004-Mutation03 4 | CDR H | SEQ ID NO:5 | SEQ ID NO:94 | SEQ ID NO:96 |
| | | DYNMH | YVNPNNGASSYNQKFK G | WDGYVYYYGLDF |
| | CDRL | SEQ ID NO:27 | SEQ ID NO:31 | SEQ ID NO:37 |
| | | KASDHINNWLA | GATSLET | QQYWSTPYT |
| PiH1004-Mutation03 5 | CDR H | SEQ ID NO:5 | SEQ ID NO:92 | SEQ ID NO:96 |
| | | DYNMH | YINPNNGATSYNQKFKG | WDGYVYYYGLDF |
| | CDRL | SEQ ID NO:27 | SEQ ID NO:31 | SEQ ID NO:37 |
| | | KASDHINNWLA | GATSLET | QQYWSTPYT |
| PiH1004-Mutation03 6 | CDR H | SEQ ID NO:5 | SEQ ID NO:93 | SEQ ID NO:96 |
| | | DYNMH | YLNPNNGATSYNQKFK G | WDGYVYYYGLDF |
| | CDRL | SEQ ID NO:27 | SEQ ID NO:31 | SEQ ID NO:37 |
| | | KASDHINNWLA | GATSLET | QQYWSTPYT |
| PiH 1004-Mutation03 7 | CDR H | SEQ ID NO:5 | SEQ ID NO:93 | SEQ ID NO:96 |
| | | DYNMH | YLNPNNGATSYNQKFK G | WDGYVYYYGLDF |
| | CDRL | SEQ ID NO:99 | SEQ ID NO:31 | SEQ ID NO:37 |
| | | KASDHVNNWL A | GATSLET | QQYWSTPYT |
| PiH1004-Mutation03 8 | CDR H | SEQ ID NO:5 | SEQ ID NO:12 | SEQ ID NO:96 |
| | | DYNMH | YVNPNNGATSYNQKFK G | WDGYVYYYGLDF |
| | CDRL | SEQ ID NO:99 | SEQ ID NO:31 | SEQ ID NO:37 |
| | | KASDHVNNWL A | GATSLET | QQYWSTPYT |
| PiH1004-Mutation03 9 | CDR H | SEQ ID NO:5 | SEQ ID NO:12 | SEQ ID NO:96 |
| | | DYNMH | YVNPNNGATSYNQKFK G | WDGYVYYYGLDF |
| | CDRL | SEQ ID NO:99 | SEQ ID NO:101 | SEQ ID NO:37 |
| | | KASDHVNNWL A | GGTSLET | QQYWSTPYT |
| PiH 1004-Mutation04 0 | CDR H | SEQ ID NO:5 | SEQ ID NO:12 | SEQ ID NO:96 |
| | | DYNMH | YVNPNNGATSYNQKFK G | WDGYVYYYGLDF |
| | CDRL | SEQ ID NO:100 | SEQ ID NO:31 | SEQ ID NO:37 |
| | | KASDHINQWLA | GATSLET | QQYWSTPYT |
| PiH1004-Mutation04 1 | CDR H | SEQ ID NO:5 | SEQ ID NO:92 | SEQ ID NO:20 |
| | | DYNMH | YINPNNGATSYNQKFKG | WDGYVYYYGLDY |
| | CDRL | SEQ ID NO:100 | SEQ ID NO:31 | SEQ ID NO:37 |
| | | KASDHINQWLA | GATSLET | QQYWSTPYT |
| PiH1004-Mutation04 2 | CDR H | SEQ ID NO:5 | SEQ ID NO:124 | SEQ ID NO:20 |
| | | DYNMH | YLNPNNGATNYNQKFK G | WDGYVYYYGLDY |
| | CDRL | SEQ ID NO:100 | SEQ ID NO:31 | SEQ ID NO:37 |
| | | KASDHINQWLA | GATSLET | QQYWSTPYT |
| PiH1004-Mutation04 3 | CDR H | SEQ ID NO:5 | SEQ ID NO:124 | SEQ ID NO:20 |
| | | DYNMH | YLNPNNGATNYNQKFK G | WDGYVYYYGLDY |
| | CDRL | SEQ ID NO:100 | SEQ ID NO:101 | SEQ ID NO:37 |
| | | KASDHINQWLA | GGTSLET | QQYWSTPYT |
| PiH1004-Mutation04 4 | CDR H | SEQ ID NO:5 | SEQ ID NO:124 | SEQ ID NO:20 |
| | | DYNMH | YLNPNNGATNYNQKFK G | WDGYVYYYGLDY |
| | CDRL | SEQ ID NO:98 | SEQ ID NO:31 | SEQ ID NO:37 |
| | | KASEHINNWLA | GATSLET | QQYWSTPYT |
| PiH1004-Mutation04 5 | CDR H | SEQ ID NO:5 | SEQ ID NO:92 | SEQ ID NO:20 |
| | | DYNMH | YINPNNGATSYNQKFKG | WDGYVYYYGLDY |
| | CDRL | SEQ ID NO:98 | SEQ ID NO:31 | SEQ ID NO:37 |
| | | KASEHINNWLA | GATSLET | QQYWSTPYT |
| PiH1004-Mutation04 6 | CDR H | SEQ ID NO:5 | SEQ ID NO:12 | SEQ ID NO:20 |
| | | DYNMH | YVNPNNGATSYNQKFK G | WDGYVYYYGLDY |
| | CDRL | SEQ ID NO:98 | SEQ ID NO:31 | SEQ ID NO:102 |
| | | KASEHINNWLA | GATSLET | QQYWSTPYS |
| PiH1004-Mutation04 7 | CDR H | SEQ ID NO:5 | SEQ ID NO:12 | SEQ ID NO:20 |
| | | DYNMH | YVNPNNGATSYNQKFK G | WDGYVYYYGLDY |
| | CDRL | SEQ ID NO:98 | SEQ ID NO:101 | SEQ ID NO:102 |
| | | KASEHINNWLA | GGTSLET | QQYWSTPYS |
| PiH1004-Mutation04 8 | CDR H | SEQ ID NO:5 | SEQ ID NO:124 | SEQ ID NO:20 |
| | | DYNMH | YLNPNNGATNYNQKFK G | WDGYVYYYGLDY |
| | CDRL | SEQ ID NO:27 | SEQ ID NO:31 | SEQ ID NO:102 |
| | | KASDHINNWLA | GATSLET | QQYWSTPYS |
| PiH1004-Mutation04 9 | CDR H | SEQ ID NO:5 | SEQ ID NO:124 | SEQ ID NO:20 |
| | | DYNMH | YLNPNNGATNYNQKFK G | WDGYVYYYGLDY |
| | CDRL | SEQ ID NO:99 | SEQ ID NO:31 | SEQ ID NO:102 |
| | | KASDHVNNWL A | GATSLET | QQYWSTPYS |
| PiH1004-Mutation05 0 | CDR H | SEQ ID NO:5 | SEQ ID NO:12 | SEQ ID NO:96 |
| | | DYNMH | YVNPNNGATSYNQKFK G | WDGYVYYYGLDF |
| | CDRL | SEQ ID NO:98 | SEQ ID NO:101 | SEQ ID NO:37 |
| | | KASEHINNWLA | GGTSLET | QQYWSTPYT |

It should be understood that, for the CDRs of the mutant antibodies shown in Table K, those skilled in the art can not only improve the PiH1004 antibody, but also apply an identical or similar improvement to other sequences in the present application, such as 51C10H4, 54B5A7, RecA101, PiH1001-PiH1008, and PiH2001-PiH2012.

It should be understood that, when other antibodies in the present disclosure include the aforementioned sites, the aforementioned mutation modes can similarly be applied to these other antibodies in the present disclosure.

Experimental results have confirmed that the improved antibodies of the present disclosure substantially retain the binding ability to CLDN6-expressing cells and exhibit binding properties to various tumor cells.

### 2.7 Antibody purification

Expi293F cells carrying a plasmid for an antibody sequence were cultured in a shake flask. Purification was conducted using Protein A Magnetic Beads (GenScript, L00695). Elution was conducted using a 0.1 M citrate and sodium citrate buffer at pH 3.0. Neutralization was conducted using a Tris-HCl buffer at pH 9.0. After dialysis in PBS at pH 7.4, a concentration and purity of an antibody were determined using Nanodrop 2000 (Thermo) with an extinction coefficient of 1.43. An antibody concentration could reach 3 mg/mL. The antibody concentration (0.5 mg/mL to 3 mg/mL) was verified by denaturing and non-denaturing polyacrylamide gel electrophoresis (SDS-PAGE) and size exclusion highperformance liquid chromatography (SEC-HPLC).

### Example 3 Determination of binding capacities of antibodies to CLDN6

To detect a binding capacity of a chimeric or humanized antibody to CLDN6, a CLDN6-expressing cell line HEK293T-CLDN6 and a CLDN6-negative control cell line HEK293T were washed three times with PBS. 1 × 10⁵ test cells and 50 µL of an antibody solution to be tested were added to a 96-well plate, and incubated at 4°C for 1 h. The cells were then washed three times with PBS. An Alexa 647-labeled anti-human IgG antibody was added to bind to a chimeric or humanized antibody that had bound to CLDN6, and incubation was conducted at 4°C for 45 min. Finally, cells were washed three times with PBS, and signals were read using FACS. Results were shown in Table 1.

**Table 1: FACS analysis results of chimeric antibodies on HEK293T and HEK293T-CLDN6**

| Clone No. | HEK 293T | HEK 293T-CLDN6 |
|---|---|---|
| 15H2D4 | 89.9 | 40841 |
| 47D5D6 | 72.5 | 32969 |
| 58G3C7 | 71.5 | 33792 |
| 47A5B10 | 71.2 | 33003 |
| 51C10H4 | 79.7 | 44863 |
| 54B5A7 | 77.8 | 37483 |
| IMAB027 | 107 | 44296 |
| Human IgG | 119 | 136 |

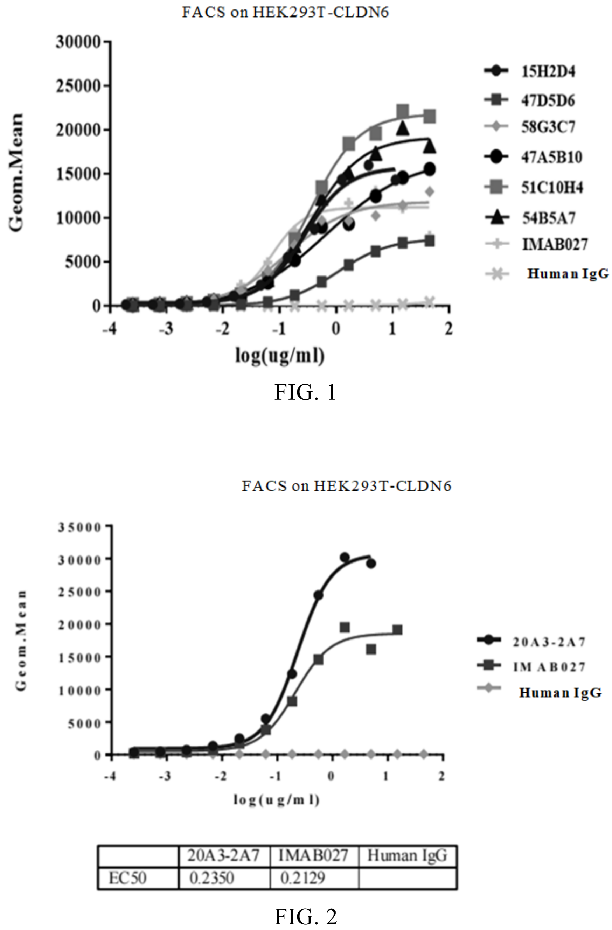

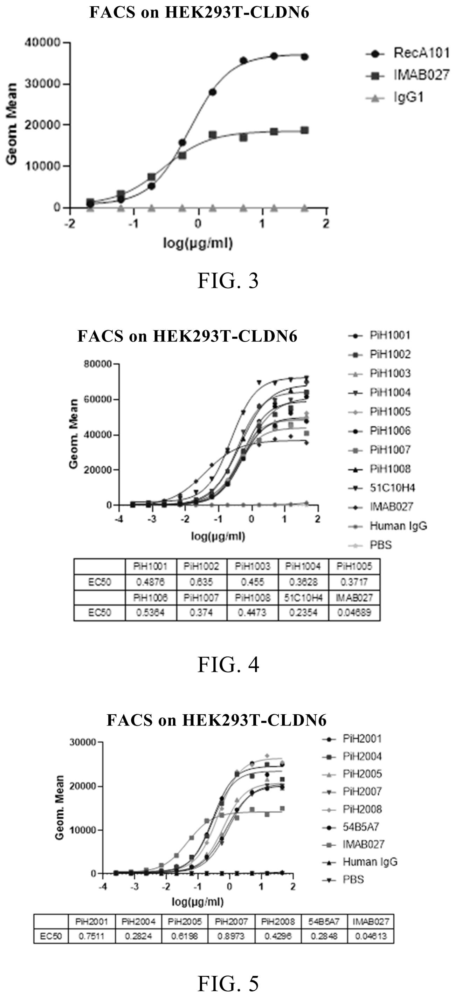

FIG. 1, FIG. 2, FIG. 3, FIG. 4, and FIG. 5 show binding capacities of chimeric antibodies, the improved chimeric monoclonal antibody RecA101, and humanized antibodies on HEK293T-CLDN6, where antibody concentrations are plotted against FACS signals (Geometric mean). An EC50 value (a concentration of an antibody that binds to half of binding sites at equilibrium) is calculated through nonlinear regression. The corresponding EC50 values are presented in Tables 2, 3, 4, 5, and 6.

**Table 2: EC50 values (unit: µg/mL) of some chimeric antibodies on HEK293T-CLDN6**

| Antibody | EC50 (CLDN6) |
|---|---|
| 15H2D4 | 0.2744 |
| 47D5D6 | 1.041 |
| 58G3C7 | 0.135 |
| 47A5B10 | 0.6739 |
| 51C10H4 | 0.3537 |
| 54B5A7 | 0.3522 |
| IMAB027 | 0.07268 |
| Human IgG | ~ 323.3 |

**Table 3: EC50 values (unit: µg/mL) of some chimeric antibodies on HEK293T-CLDN6**

| Antibody | EC50 (CLDN6) |
|---|---|
| 20A3-2A7 | 0.2350 |
| IMAB027 | 0.2129 |
| Human IgG | / |

**Table 4: EC50 values (unit: µg/mL) of improved chimeric monoclonal antibodies on HEK293T-CLDN6**

| Antibody | EC50 (CLDN6) |
|---|---|
| RecA101 | 0.7298 |
| IMAB027 | 0.2816 |

**Table 5: EC50 values (unit: µg/mL) of humanized antibodies (PiH1001 to PiH1008) on HEK293T-CLDN6**

| Antibody | EC50 (CLDN6) |
|---|---|
| PiH1001 | 0.4876 |
| PiH1002 | 0.635 |
| PiH1003 | 0.455 |
| PiH1004 | 0.3628 |
| PiH1005 | 0.3717 |
| PiH1006 | 0.5364 |
| PiH1007 | 0.374 |
| PiH1008 | 0.4473 |
| 51C10H4 | 0.2354 |
| IMAB027 | 0.04689 |

**Table 6: EC50 values of humanized antibodies (PiH2001 to PiH2008) on HEK293T-CLDN6**

| (unit: µg/mL) Antibody | EC50 (CLDN6) |
|---|---|
| PiH2001 | 0.7511 |
| PiH2004 | 0.2824 |
| PiH2005 | 0.6198 |
| PiH2007 | 0.8973 |
| PiH2008 | 0.4296 |
| 54B5A7 | 0.2848 |
| IMAB027 | 0.04613 |

According to the above results, compared to the non-humanized chimeric antibody 54B5A7, the humanized antibodies do not undergo a substantial reduction in binding activity. The chimeric antibodies, improved chimeric monoclonal antibodies, and humanized antibodies selected in the present disclosure all exhibit excellent CLDN6 binding properties. Some chimeric antibodies demonstrate significantly better binding properties than the benchmark antibody IMAB027, particularly at high concentrations. Further, the inventors have experimentally found that, compared to the non-humanized chimeric antibody 54B5A7, PiH2012 exhibits a comparable binding activity to CLDN6 (not shown in the figures).

### Example 4 Determination of binding capacities of chimeric antibodies and the improved antibody RecA101 to CLDN3/4/9

Specific binding conditions of the chimeric antibodies 20A3-M1, 20A3-M2, and 51C10-M1, the improved antibody RecA101, and the improved antibodies resulting from PiH1004 listed in Table G (Table H) to cells expressing CLDN6 and to cells expressing CLDN3, CLDN4, and CLDN9 were analyzed by FACS.

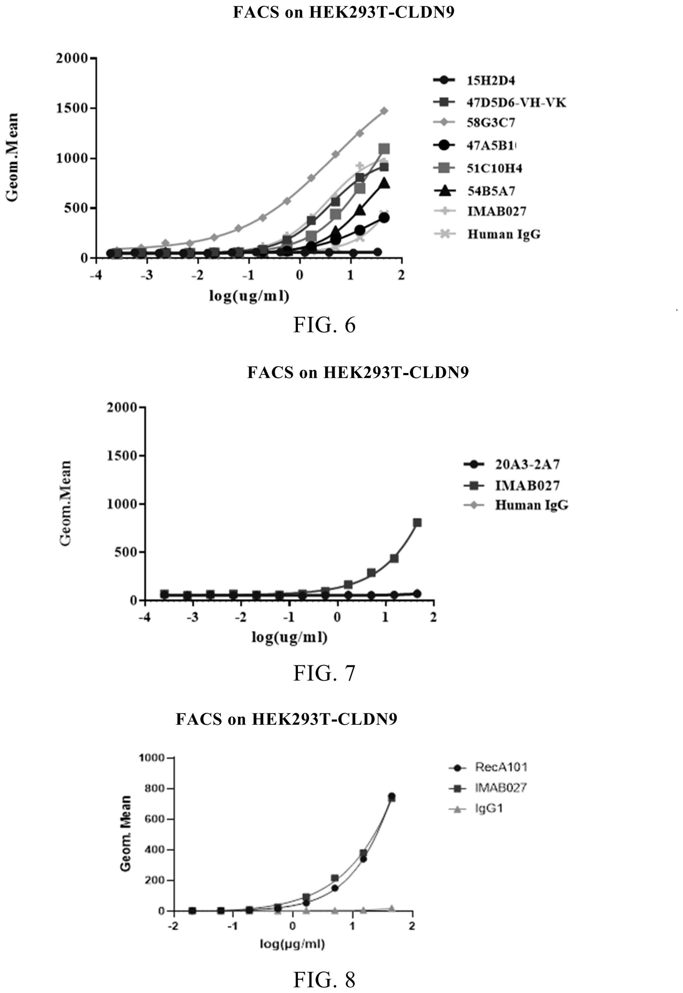

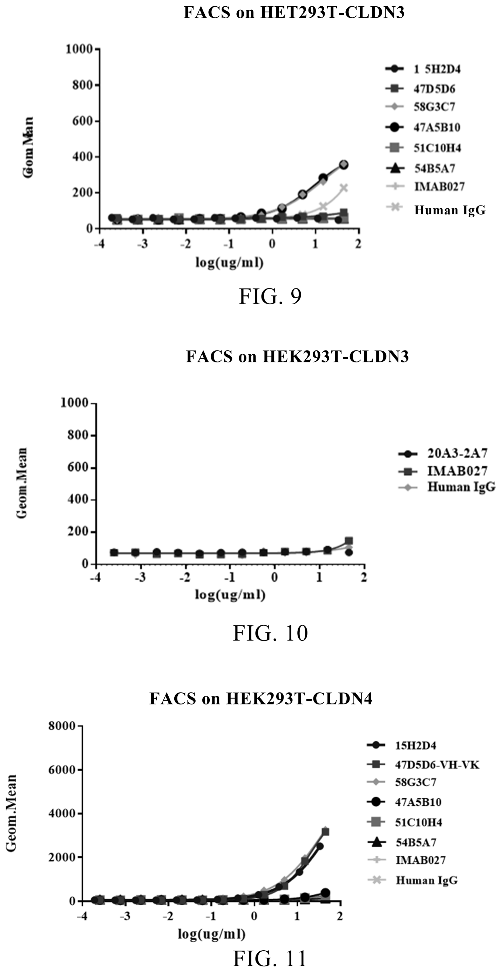

50 µL of an antibody to be tested was added to a 96-well plate, then 1 × 10⁵ cells were added to each well, and incubation was conducted at 4°C for 1 h. Cells were washed three times with PBS. An Alexa 647-labeled anti-human IgG antibody was added to bind to a chimeric or humanized antibody that had bound to CLDN6, and incubation was conducted at 4°C for 45 min. Finally, cells were washed three times with PBS, and signals were read using FACS. Binding capacities of the antibody to CLDN9, CLDN3, and CLDN4 were analyzed separately based on detection data. Results were shown in FIG. 6 to FIG. 12.

Tables 7 to 9 show FACS ratio results for some chimeric antibodies and improved antibodies on 293T-CLDN6 and 293T-CLDN3/4/9.

**Table 7: FACS analysis results for some chimeric antibodies on 293T-CLDN6/293T-CLDN3/4/9**

| Antibody/Geom.M ean | CLDN3 | CLDN4 | CLDN6 | CLDN9 | CLDN6/CLD N9 ratio |
|---|---|---|---|---|---|
| 15H2D4 | 139 | 5280 | 40841 | 88.4 | 462 |
| 47D5D6 | 151 | 5624 | 32969 | 428 | 77 |
| 58G3C7 | 1006 | 8443 | 33792 | 731 | 46 |
| 51C10H4 | 103 | 210 | 44863 | 248 | 181 |
| 54B5A7 | 101 | 104 | 37483 | 141 | 265 |
| IMAB027 | 151 | 399 | 44296 | 985 | 45 |
| PBS | 101 | 90.6 | 136 | 74 | 1.8 |
| Mouse IgG | 168 | 158 | 196 | 119 | 1.6 |

**Table 8: FACS analysis results for improved antibodies on 293T-CLDN6/293T-CLDN3/4/9 (10 µg/mL)**

| Antibody/Geom.Mean | CLDN6 | CLDN9 | HEK293T |
|---|---|---|---|
| 20A3-M1 | 40166 | 243 | 374 |
| 20A3-M2 | 33953 | 297 | 475 |
| IMAB027 | 23648 | 3814 | 262 |
| Human IgG | 163 | 79.4 | 65 |
| PBS | 79.5 | 64.7 | 64.7 |

**Table 9: FACS analysis results for improved antibodies on 293T-CLDN6/293T-CLDN3/4/9 (10 µg/mL)**

| Antibody/Geom.Mean | CLDN6 | CLDN9 | HEK293T |
|---|---|---|---|
| 51C10-M1 | 41411 | 1168 | 224 |
| RecA101 | 41764 | 1740 | 251 |
| IMAB027 | 23574 | 1807 | 208 |
| Human IgG | 222 | 295 | 191 |
| PBS | 107 | 101 | 96.5 |

According to the above results, the chimeric antibodies of the present disclosure all demonstrate significant binding specificity for CLDN6, which is remarkably superior to the binding specificity of the benchmark antibody IMAB027. Although some antibodies show binding signals to CLDN3, CLDN4, or CLDN9, these binding signals are significantly lower than binding signals to CLDN6, with differences even of several hundred-fold. For example, among antibodies with a specific CLDN9 binding affinity, some antibodies such as 54B5A7 and 51C10H4 have a CLDN6/CLDN9 signal ratio of approximately 200-fold. Some antibodies such as 58G3C7, 47D5D6, and 20A3-2A7 exhibit a detectable CLDN9 binding affinity, but still have a CLDN6/CLDN9 signal ratio of around 50-fold.

The improved antibodies of the present disclosure, including 20A3-M1, 20A3-M2, 51C10-M1, and RecA101, also exhibit significant CLDN6 binding specificity. Particularly, 20A3-M1 and 20A3-M2 demonstrate a significantly weaker binding affinity to CLDN9 than the control antibody IMAB027.

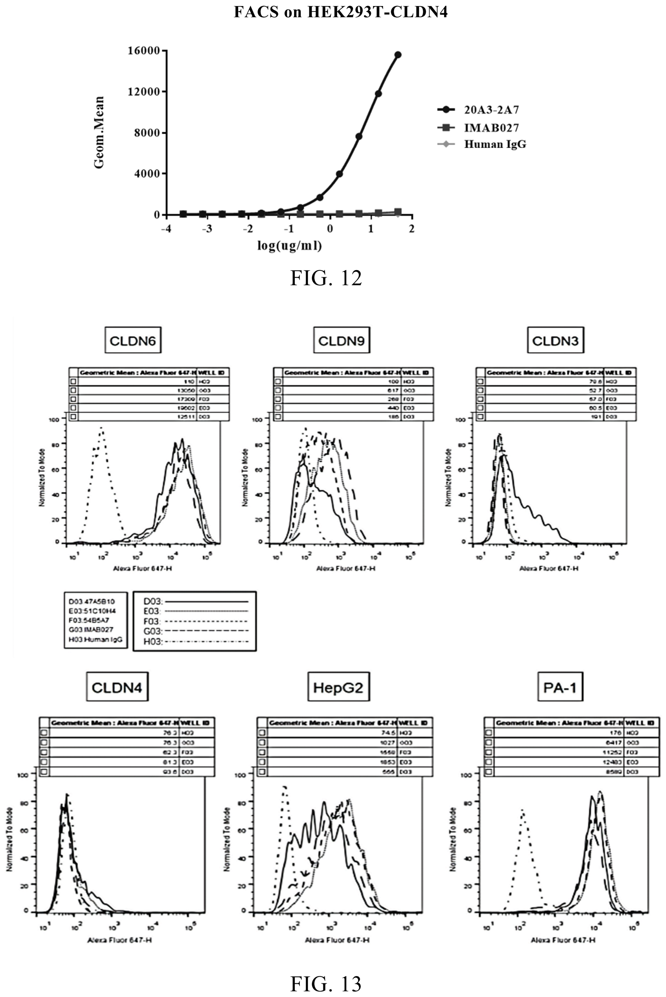

FIG. 13 shows binding results of 51C10H4 and 54B5A7 chimeric antibodies at a concentration of 5 µg/mL to cell lines HEK293T-CLDN6, HEK293T-CLDN9, HEK293T-CLDN3, and HEK293T-CLDN4. According to these results, the chimeric antibodies 51C10H4 and 54B5A7 exhibit better specificity for HEK293T-CLDN9 than the control antibody IMAB027. Further, these two chimeric antibodies exhibit virtually no binding affinity to CLDN3 and CLDN4, and exhibit a several hundred-fold weaker binding affinity to CLDN9 than to CLDN6, which enables the maximized therapeutic efficacy and the minimized systemic toxicity.

The inventors have further discovered that humanized antibodies PiH1001 to PiH1008 demonstrate a comparable binding capacity for CLDN3/4/9 to the non-humanized 51C10H4, and humanized antibodies PiH2001, PiH2004, PiH2005, PiH2006, PiH2007, PiH2008, PiH2009, PiH2010, PiH2011, and PiH2012 demonstrate a comparable binding capacity for CLDN3/4/9 to the non-humanized 54B5A7.

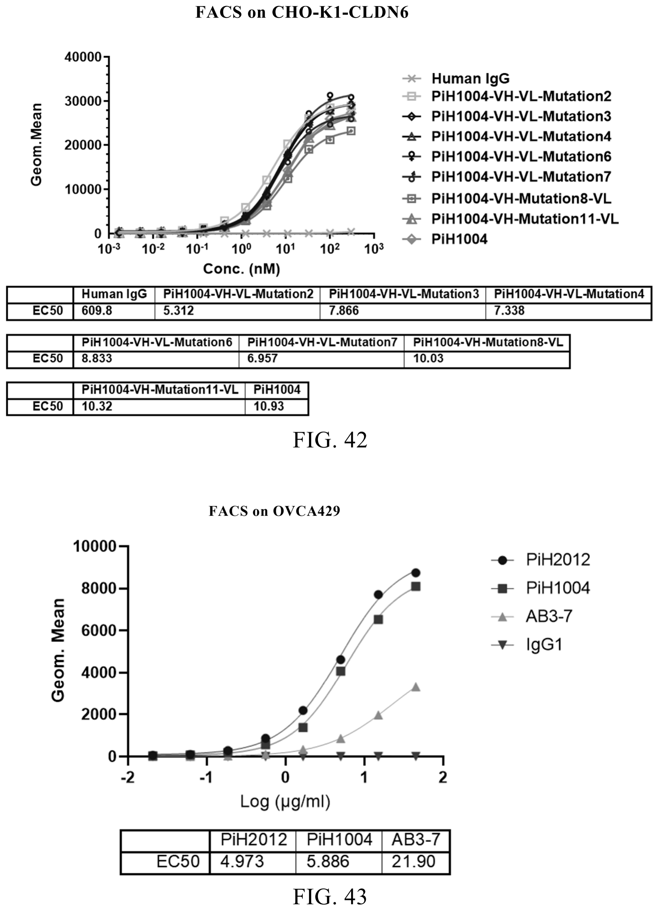

FIG. 42 shows binding capacities of the improved antibodies resulting from PiH1004 in Table G (Table H), such as PiH1004-VH-Mutation1-VL, PiH1004-VH-Mutation8-VL, and PiH1004-VH-VL-Mutation1, to CLDN6. It can be seen that, compared to the antibody PiH1004, the site-directed mutagenesis in these improved antibodies does not reduce the binding capacity to CLDN6. Some improved antibodies resulting from site-directed mutagenesis demonstrate an enhanced binding capacity to CLDN6. Further, in the present disclosure, binding capacities of the antibodies listed in Table K to CLDN6 are tested. Compared to the non-mutated antibody (PiH1004), mutations in the improved antibodies also do not reduce the binding capacity to CLDN6 (not shown in a figure).

### Example 5: Determination of cross-reactivity of antibodies in mice and monkeys

As described above, the binding of a chimeric or humanized antibody to cells overexpressing mouse CLDN6 and cells overexpressing monkey CLDN6 was detected by FACS.

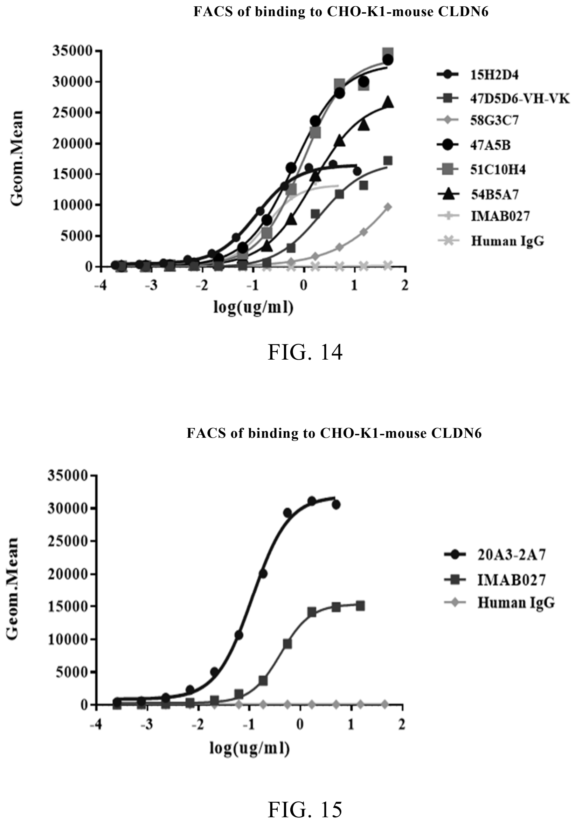

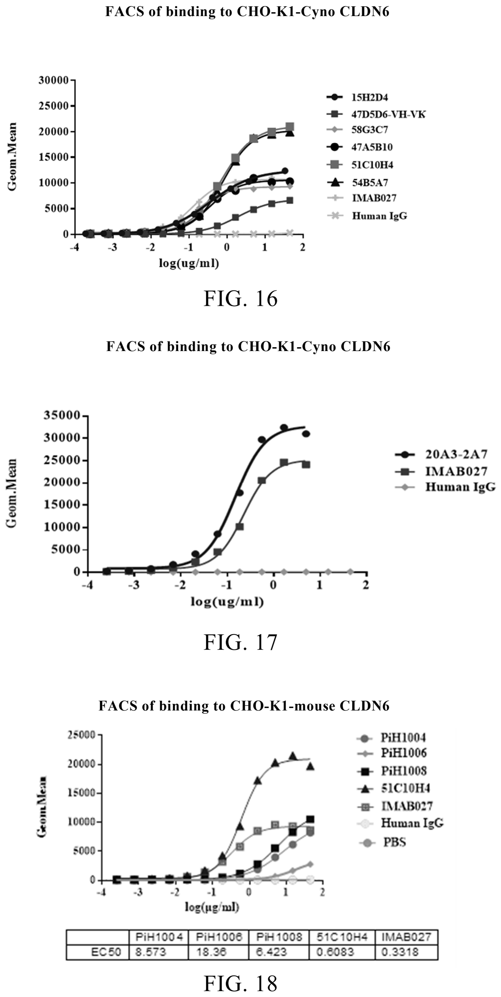

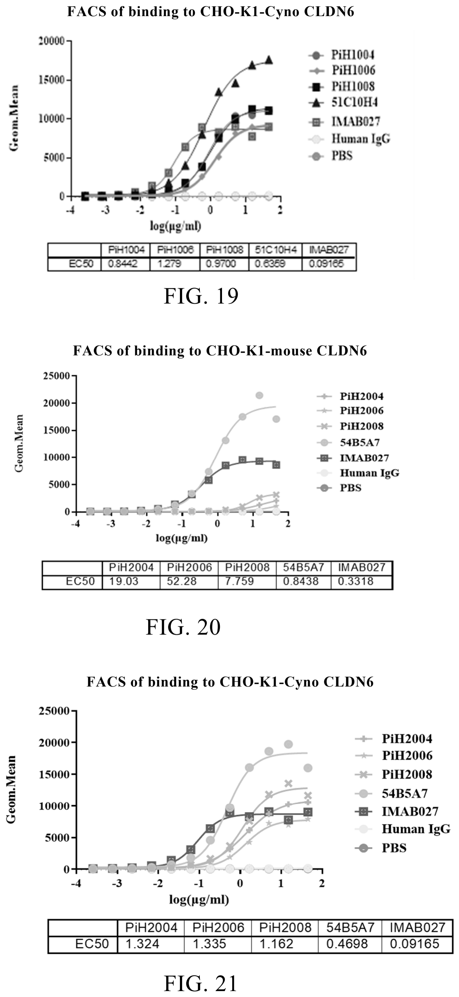

FIG. 14, FIG. 15, FIG. 16, and FIG. 17 show binding results of chimeric antibodies to cells overexpressing mouse CLDN6 and cells overexpressing monkey CLDN6. FIG. 18, FIG. 19, FIG. 20, and FIG. 21 show binding results of humanized antibodies to cells overexpressing mouse CLDN6 and cells overexpressing monkey CLDN6. The corresponding EC50 values are shown in Table 10.

**Table 10: EC50 values (unit: µg/mL) for the binding of chimeric antibodies to cells overexpressing mouse CLDN6 and cells overexpressing monkey CLDN6**

| Antibody | EC50 (CHO-K1-Mouse CLDN6 cells) | EC50 (CHO-K1-Cyno CLDN6 cells) |
|---|---|---|
| 15H2D4 | 0.1167 | 0.3514 |
| 47D5D6 | 2.086 | 1.938 |
| 58G3C7 | / | 0.1599 |
| 47A5B10 | 0.6333 | 0.3433 |
| 51C10H4 | 0.9352 | 0.7222 |
| 54B5A7 | 1.499 | 0.7801 |
| IMAB027 | 0.1808 | 0.1314 |
| Human IgG | 78.78 | 132.0 |

According to the results in Table 10 and FIG. 14 to FIG. 17, the chimeric antibodies of the present disclosure exhibit a strong binding capacity to cells overexpressing mouse CLDN6 and cells overexpressing monkey CLDN6.

According to the results in FIG. 18 to FIG. 21, compared to the non-humanized antibody, the humanized antibodies of the present disclosure exhibit a significantly reduced binding capacity to cells overexpressing mouse CLDN6. In particular, the humanized antibody of the antibody 54B5A7 shows almost no binding to cells overexpressing mouse CLDN6. It indicates that the humanization in the present disclosure successfully reduces/avoids the species cross-reactivity.

### Example 6 Determination of binding capacities and inhibitory effects of antibodies for tumor cells

The specific binding of a chimeric antibody to a tumor cell was detected by FACS, and an EC50 value was calculated accordingly to evaluate an inhibitory effect of the chimeric antibody on the tumor cell.

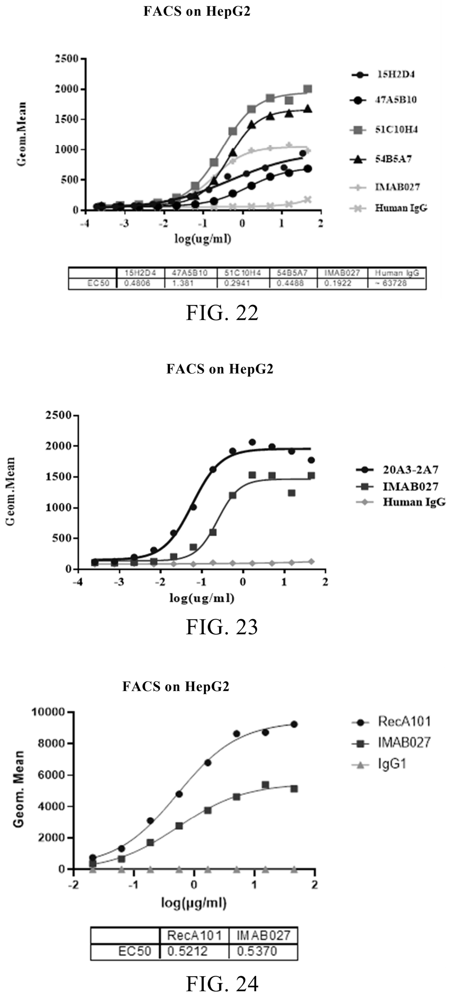

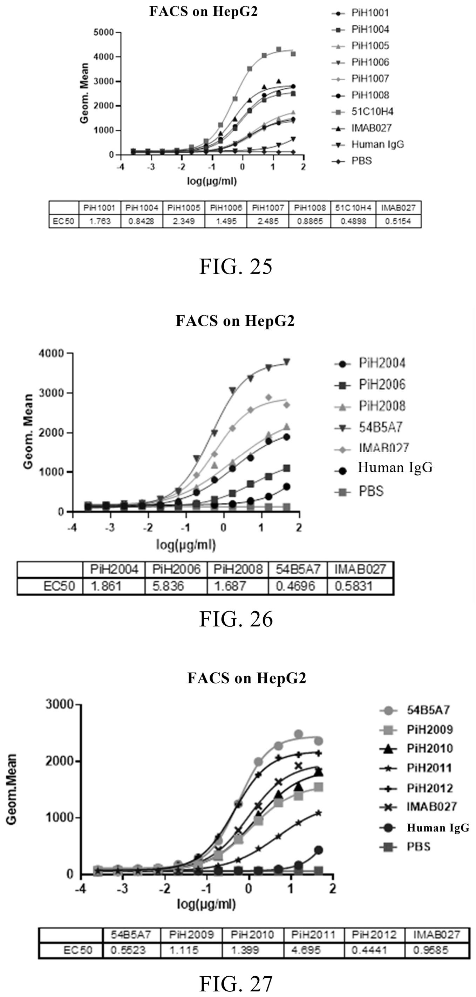

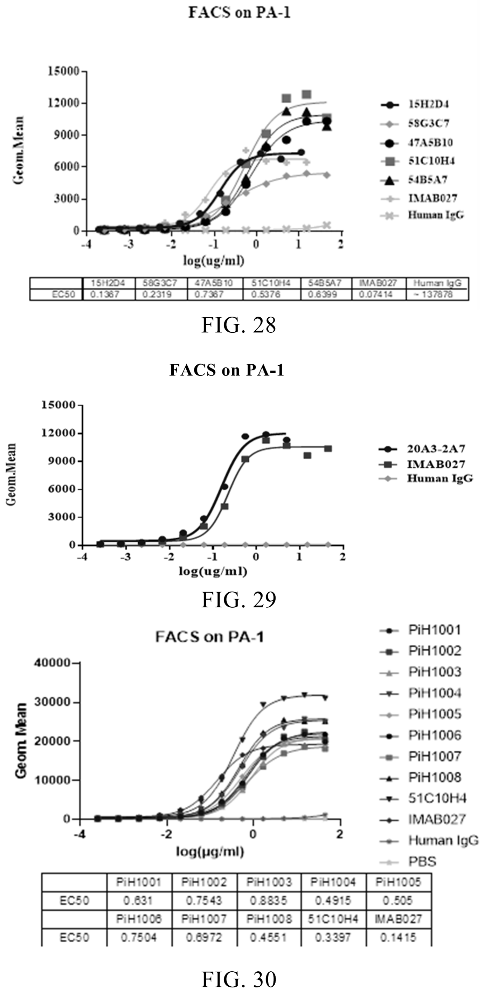

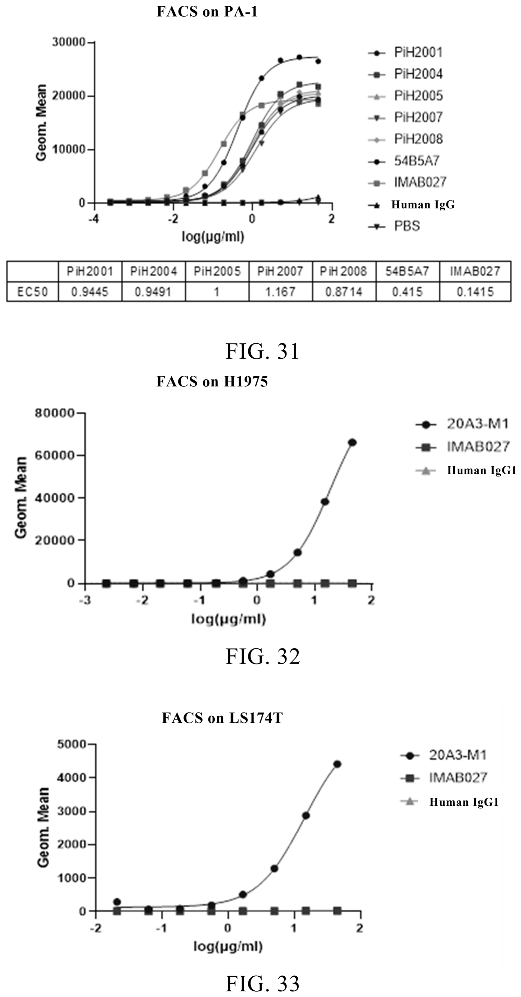

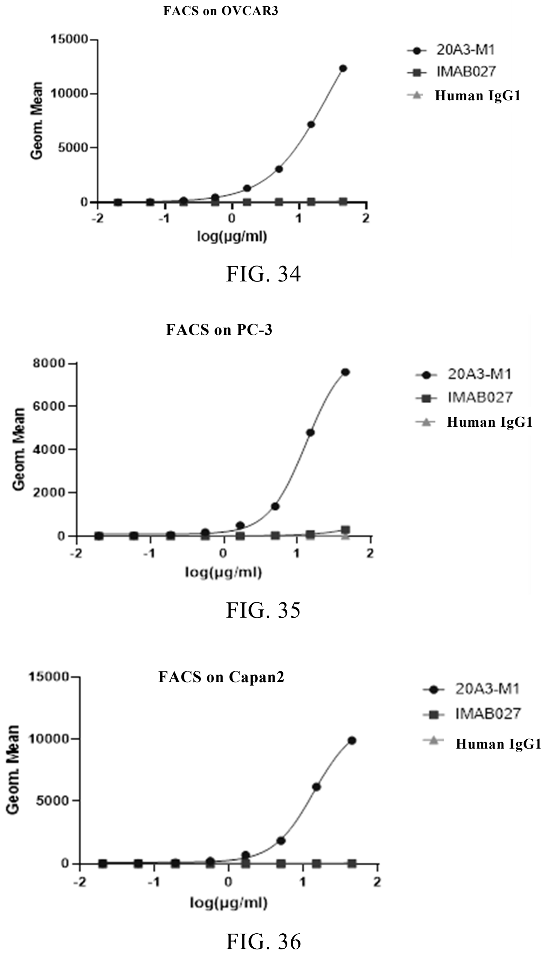

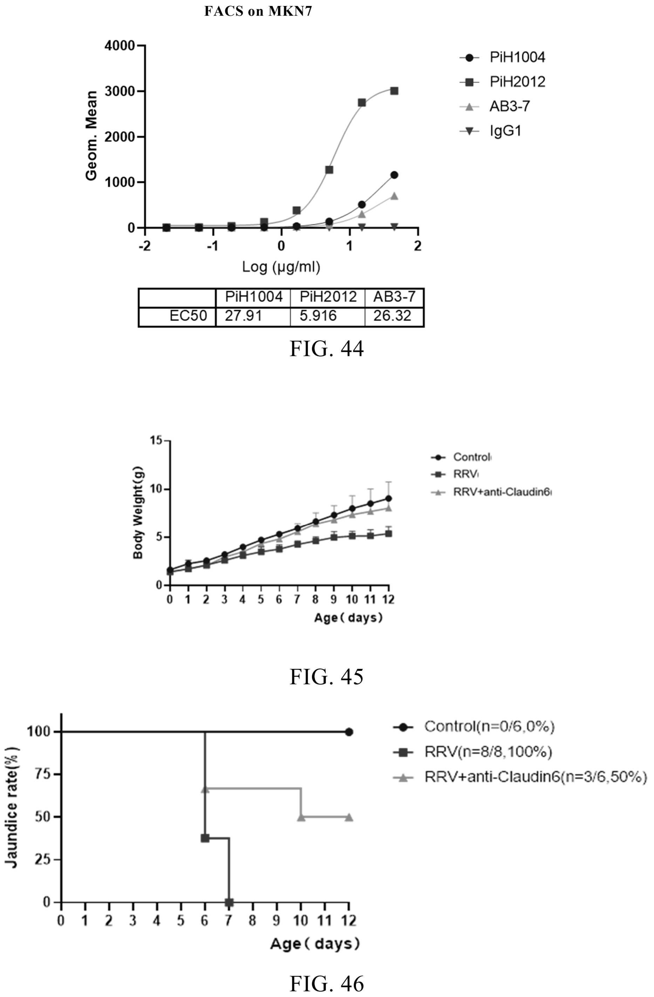

50 µL of an antibody to be tested was added to a 96-well plate, then 1 × 10⁵ cells were added to each well, and incubation was conducted at 4°C for 1 h. Cells were washed three times with PBS. An Alexa 647-labeled anti-human IgG antibody was added to bind to the antibody that had bound to a corresponding tumor cell, and incubation was conducted at 4°C for 45 min. Finally, cells were washed three times with PBS, and signals were read using FACS. A binding capacity of the antibody to each tumor cell was analyzed based on detection data. Results were shown in FIG. 22 to FIG. 36. FIG. 22 to FIG. 27 show binding capacities of chimeric antibodies, the improved chimeric monoclonal antibody Rec101A, and humanized antibodies to HepG2. FIG. 28 to FIG. 31 show binding capacities of chimeric antibodies and humanized antibodies to PA-1. FIG. 32 to FIG. 36 show binding capacities of the improved antibody 20A3-M1 to a plurality of tumor cell lines, including a human lung adenocarcinoma cell line H1975, a human colon adenocarcinoma cell line LS174T, a human ovarian cancer cell line OVCAR3, a human prostate cancer cell line PC-3, and a human pancreatic cancer cell line Capan-2. FIG. 43 shows binding capacities of PiH1004 and PiH2012 to an ovarian cancer cell line OVCA429. FIG. 44 shows binding capacities of PiH1004 and PiH2012 to a human gastric cancer cell line MKN7 expressing CLDN6 at a high level.

Tables 11 to 15 also show EC50 values of these antibodies on tumor cells that are calculated based on FIG. 22 to FIG. 36. Table 16 shows FACS analysis results for improved antibodies targeting HepG2 at a concentration of 5 µg/mL.

**Table 11: EC50 values of some chimeric antibodies against tumor cells**

| Antibody | EC50 (HepG2) | EC50 (PA-1) |
|---|---|---|
| 15H2D4 | 0.4806 | 0.1367 |
| 58G3C7 | / | 0.2319 |
| 47A5B10 | 1.381 | 0.7367 |
| 51C10H4 | 0.2941 | 0.5376 |
| 54B5A7 | 0.4488 | 0.6399 |
| IMAB027 | 0.1922 | 0.07414 |
| Human IgG | ~63728 | ~137878 |

**Table 12: EC50 values of some chimeric antibodies against tumor cells**

| Antibody | EC50 (CLDN6) | (HepG2) | (PA-1) |
|---|---|---|---|
| 20A3-2A7 | 0.2350 | 0.05866 | 0.1585 |
| IMAB027 | 0.2129 | 0.2417 | 0.2226 |
| Human IgG | / | ~3.688e+012 | ~ 1.415e+015 |

**Table 13: EC50 values of humanized antibodies**

| Antibody | EC50 (HepG2) | EC50 (PA-1) |
|---|---|---|
| PiH1001 | 1.763 | 0.631 |
| PiH1002 | / | 0.7543 |
| PiH 1003 | / | 0.8835 |
| PiH1004 | 0.8428 | 0.4915 |
| PiH 1005 | 2.349 | 0.505 |
| PiH1006 | 1.495 | 0.7504 |
| PiH 1007 | 2.485 | 0.6972 |
| PiH1008 | 0.8865 | 0.4551 |
| 51C10H4 | 0.4898 | 0.3397 |
| IMAB027 | 0.5154 | 0.1415 |
| Human IgG | 77.46 | ~ 194.1 |
| PBS | ~ 0.0005495 | ~ 0.0 |

**Table 14: EC50 values of humanized antibodies**

| Antibody | EC50 (HepG2) | EC50 (PA-1) |
|---|---|---|
| PiH2001 | / | 0.9445 |
| PiH2004 | 1.861 | 0.9491 |
| PiH2005 | / | 1 |
| PiH2006 | 5.836 | / |
| PiH2007 | / | 1.167 |
| PiH2008 | 1.687 | 0.8714 |
| 54B5A7 | 0.4696 | 0.415 |
| IMAB027 | 0.5831 | 0.1415 |

**Table 15: EC50 values of humanized antibodies**

| Antibody | EC50 (HepG2) |
|---|---|
| PiH2009 | 1.115 |
| PiH2010 | 1.399 |
| PiH2011 | 4.695 |
| PiH2012 | 0.4441 |
| 54B5A7 | 0.5523 |
| IMAB027 | 0.9585 |

**Table 16: FACS analysis results of improved antibodies (antibody concentration: 5 µg/mL)**

| Antibody/Geom.Mean | HepG2 |
|---|---|
| PiH1004 | 851.0 |
| PiH1004-M1 | 892.0 |
| IMAB027 | 970.0 |
| Human IgG | 57.6 |
| PBS | 54.3 |

According to the above results, the chimeric antibodies and humanized antibodies of the present disclosure can significantly bind to tumor cells PA-1 and HepG2 with very low EC50 values, and demonstrate remarkable inhibitory/cytotoxic activities against tumor cells *in vitro.* Some chimeric antibodies show a comparable or superior inhibitory/cytotoxic activity to the benchmark antibody IMAB027. It indicates the therapeutic potential of the antibodies of the present disclosure for ovarian cancer and liver cancer. As shown in Table 16, an improved antibody resulting from a humanized antibody in the present disclosure exhibits a comparable binding effect for the tumor cell line HepG2 to the humanized antibody, indicating that the improvement/mutation in the present disclosure does not lead to a decline in tumor-inhibiting activity.

The various antibodies in the present disclosure also demonstrate favorable effects in other tumor cell lines. It has been verified that, as shown in FIG. 24, the recombined chimeric monoclonal antibody Rec101A exhibits a better binding capacity to HepG2 cells than the positive antibody. As shown in FIG. 32 to FIG. 38, the improved antibody 20A3-M1 of the present disclosure also demonstrates a significant binding activity to a plurality of tumor cell lines, including a human lung adenocarcinoma cell line H1975, a human colon adenocarcinoma cell line LS174T, a human ovarian cancer cell line OVCAR3, a human prostate cancer cell line PC-3, a pancreatic cancer cell line Capan-2, a human ovarian teratoma cell line PA-1, and a liver cancer cell line HepG2. It is observed that other improved antibodies 51C10-M1, 51C10-M2, 51C10-M3, and 51C10-M4 also have similar effects. As shown in FIG. 43, PiH1004 and PiH2012 demonstrate a stronger binding capacity to the ovarian cancer cell line OVCA429 than the positive control AB3-7. As shown in this figure, PiH1004 and PiH2012 exhibit a stronger binding capacity to the human gastric cancer cell line MKN7 than the positive control AB3-7.

Further, public documents have shown that CLDN6 is expressed at a high level in various cancer tissues and corresponding tumor cells, including ovarian cancer, lung cancer, gastric cancer, breast cancer, liver cancer, pancreatic cancer, skin cancer, malignant melanoma, head and neck cancer, sarcoma, cholangiocarcinoma, renal cell carcinoma, and uterine cancer. Therefore, the chimeric antibodies, humanized antibodies, and improved antibodies of the present disclosure can be expected to exhibit a therapeutic effect against these tumors.

### Example 7: ADCC assay

Effector cells (NK92/CD16A-VV) were collected and resuspended in an ADCC assay buffer (99% MEM α, nucleosides, phenol red-free + 1% fetal bovine serum). Target cells (CHO-Kl-CLDN6) were collected and resuspended in the ADCC assay buffer. Working solutions of a test sample and a control sample were prepared using the ADCC assay buffer. A density of target cells was adjusted using the ADCC assay buffer. A target cell suspension was transferred to a 96-well assay plate. The working solutions of the test sample and the control sample and the ADCC assay buffer were added to the respective wells of the 96-well assay plate. The 96-well assay plate was incubated at room temperature for 30 min. According to an E/T ratio, a density of effector cells was adjusted using the ADCC assay buffer. An effector cell suspension was transferred to the corresponding wells of the 96-well assay plate. The 96-well assay plate was incubated in a cell culture incubator (37°C/5% CO₂) for 6 h. After the incubation, the 96-well assay plate was taken out and centrifuged. A resulting supernatant was carefully collected and transferred to an additional 96-well assay plate. A lactate dehydrogenase (LDH)-detection working solution was added to the corresponding wells of the additional 96-well assay plate, and a chromogenic reaction was conducted at room temperature. OD values were read on a microplate reader with a detection wavelength of 492 nm and a reference wavelength of 650 nm.

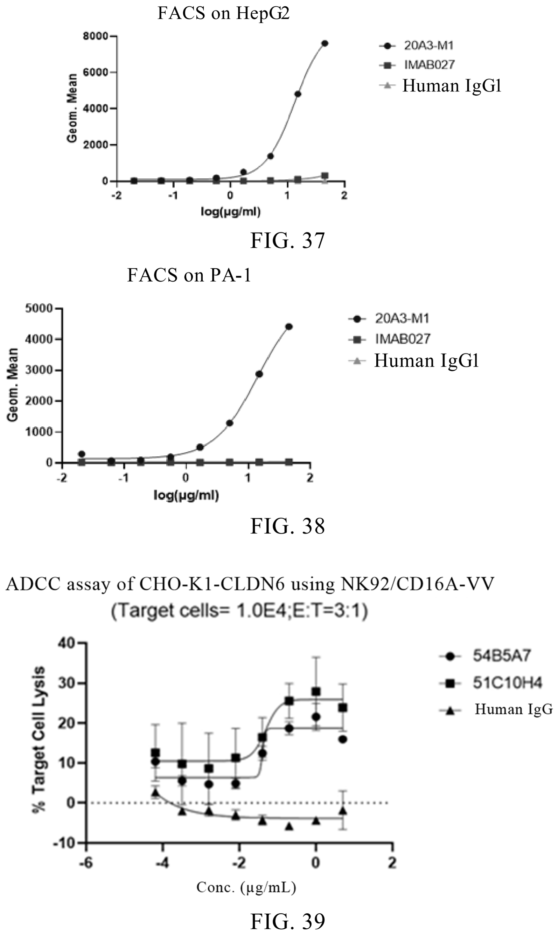

In this experiment, NK92/CD16A-VV was adopted as the effector cells, and CHO-K1-CLDN6 was adopted as the target cells. At an effector-to-target ratio of 3:1, a test sample (54B5A7 and 51C10H4) and human IgG1 were serially diluted and co-incubated with the corresponding target cells for 6 h. A percentage of target cells killed was measured. Results were shown in FIG. 39 and Table 17. The percentage of target cells killed in the figure was expressed as mean±SEM.

**Table 17: Fitting parameters of an ADCC dose-response assay**

| **Log(agonist) vs. response -- Variable slope (four parameters) best-fit values** | | | |
|---|---|---|---|
| Sample ID | 54B5A7 | 51C10H4 | Human IgG1 |
| Bottom | 6.341 | 10.5 | N/A |
| Top | 18.84 | 25.94 | |
| LogEC₅₀ | -1.385 | -1.316 | |
| HillSlope | ~ 2.500 | 2.426 | |
| EC₅₀ (µg/mL) | 0.04126 | 0.04834 | |
| Span | 12.49 | 15.45 | |

As shown in Table 17 and FIG. 39, under the current experimental conditions, the chimeric antibodies 54B5A7 and 51C10H4 exhibit significant ADCC effects against CHO-K1-CLDN6 cells and both can effectively induce killing of CLDN6-expressing tumor cells. In contrast, the negative control (human IgG1) does not demonstrate an ADCC effect against CHO-K1-CLDN6 cells.

### Example 8: CDC assay

Target cells (CHO-K1-CLDN6) were cultured with a complete medium in a 37°C/5% CO₂ cell culture incubator. An effector molecule was 5% normal human serum complement (NHSC). With the highest concentration of 50 µg/mL, an antibody solution was 5-fold diluted to provide 8 concentrations.

Target cells were collected and resuspended in a CDC assay buffer. Working solutions of a test sample and a control sample were prepared using a CDC assay buffer. A density of target cells was adjusted, and a target cell suspension was transferred to a 384-well assay plate. A control sample/test sample working solution was added to the corresponding wells of the 384-well assay plate, and incubation was conducted at room temperature for 30 min. An NHSC working solution was prepared and added to the corresponding wells of the 384-well assay plate. The 384-well assay plate was incubated in a cell culture incubator (37°C/5% CO₂) for 4 h. After the incubation, the 384-well assay plate was taken out, a Cell Titer-Glo^{®} (Promega, Item No. G7573) working solution was added, and incubation was conducted at room temperature for 10 min. A chemiluminescence value was read using a microplate reader.

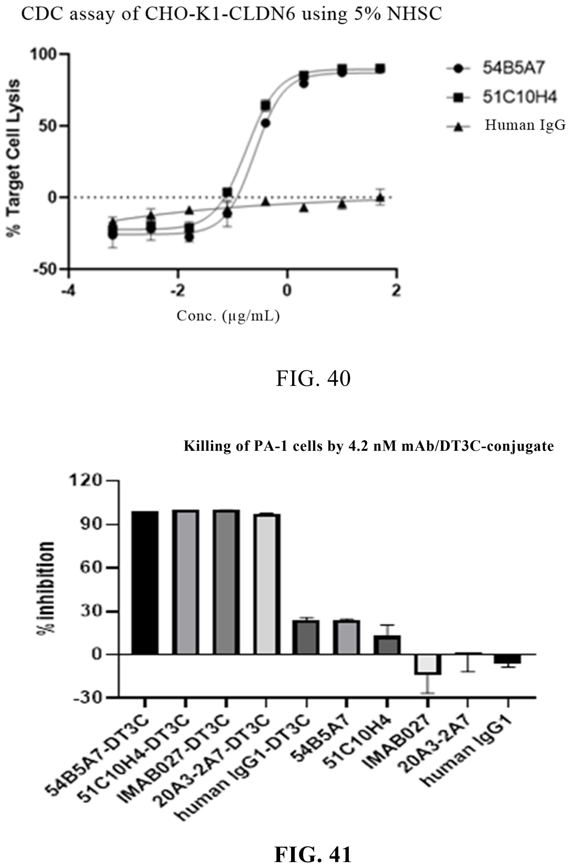

Raw data of the CDC assay was exported by the PHERA Star FSX system and analyzed using the software Microsoft Office Excel 2016 and GraphPad Prism 6. Under a 5% NHSC condition, a positive control, the test sample, and the human IgG1 were serially diluted and co-incubated with the corresponding target cells (CHO-K1-CLDN6) for 4 h. A percentage of target cell killed was measured. Results were shown in FIG. 40 and Table 18. The percentage of target cells killed in the figure was expressed as mean±SEM.

**Table 18: Fitting parameters of a CDC dose-response assay**

| **Log(agonist) vs. response -- Variable slope (four parameters) best-fit values** | | | |
|---|---|---|---|
| Sample ID | 54B5A7 | 51C10H4 | Human IgG1 |
| Bottom | -25.68 | -22.4 | N/A |
| Top | 86.96 | 89.49 | |
| LogEC₅₀ | -0.5995 | -0.7504 | |
| HillSlope | 1.642 | 1.505 | |
| EC₅₀ (µg/mL) | 0.2515 | 0.1777 | |
| Span | 112.6 | 111.9 | |

As shown in FIG. 40 and Table 18, the chimeric antibodies 54B5A7 and 51C10H4 both can effectively mediate a CDC effect against CHO-K1-CLDN6 cells, but the negative control (human IgG1) cannot induce a CDC effect against CHO-K1-CLDN6 cells.

### Example 9 Aggregation Stability

According to operating instructions of an Uncle high-throughput multi-parameter protein stability analyzer, information and a location of each sample were input. Samples were diluted to a same concentration and centrifuged at 12,000 rmp for 3 min. 9 µL of a sample was added to a corresponding UNi tube, and both ends of the UNi tube were sealed with silica gel. Heating parameters were set as follows: heating from 25°C to 95°C at a heating rate of 0.5°C/min. A sample was loaded, and a program was run. Tm and Tagg were calculated using the Uncle analysis software. Results were shown in Table 19.

**Table 19: Tm and Tagg results of antibodies**

| Sample | Concentration (mg/mL) | Tm1 (average temperature + %CV) | Tm2 (average temperature + %CV) | Tagg 266 (average temperature + %CV) |
|---|---|---|---|---|
| 54B5A7 | 0.855 | 63.25+0.11 | 74.25+0.48 | 62.79+0.24 |
| 51C10H4 | 0.855 | 63.30+0.22 | 74.55+0.28 | 62.94+0.18 |
| IMAB027 | 0.855 | 63.82+0.25 | 77.35+0.03 | 56.89+0.76 |

According to the results in Table 19, compared to the benchmark antibody IMAB027, the chimeric antibodies 54B5A7 and 51C10H4 of the present disclosure exhibit outstanding aggregation stability, indicating a wide window for the binding of these chimeric antibodies to CLDN6-expressing cells.

### Example 10 Endocytosis test

In the present disclosure, a recombinant protein DT3C (Cusabio, Item No. CSB-EP360556CQR1) was used to determine internalization and immunotoxin activity of an antibody. DT3C is a protein generated by genetically fusing a catalytic domain of diphtheria toxin (DT) with an antibody-binding domain of a streptococcal protein G. DT3C specifically binds to a Fc portion of an antibody to produce a complex, and if internalized into cells, the complex induces cell death by inhibiting protein synthesis. With this system, antibody internalization and a cell-killing effect induced by an immunotoxin can be observed simultaneously.

PA-1 cells were cultured. Cells at a logarithmic growth phase were harvested. 40 µL of a resulting cell suspension was added to each well of a 384-well plate at a density of 500 cells/well, and cultured overnight at 37°C and 5% CO₂. Chimeric antibodies, the benchmark antibody, and the negative control human IgG1 were each mixed with DT3C in a ratio of 1:2 for 30 min at room temperature to produce mAb-DT3C conjugates. Each conjugate was added to the 384-well plate, and the cells were cultured for 3 d at 37°C and 5% CO₂.

20 µL of a CellTiter-Glo assay kit (CTG) (Promega, Item No. G7573) was added to each well, and incubation was conducted at room temperature for 30 min. A luminescence value was recorded using a microplate reader Envision 2105. Results were shown in FIG. 41.

As shown in FIG. 41, DT3C conjugates of 54B5A7, 20A3-2A7, and 51C10H4 exhibit an inhibitory rate of 90% or more on PA-1 cells at a concentration of 4.2 nM, which is comparable to an inhibitory rate of the benchmark antibody IMAB027. It indicates that the chimeric antibodies 54B5A7, 20A3-2A7, and 51C10H4 allow a significant internalization effect on PA-1 cells.

### Example 11 Therapeutic efficacy of an anti-CLDN6 antibody in an RRV-induced mouse biliary atresia (BA) model

The anti-CLDN6 antibody adopted in this example was an antibody including variable regions from 54B5A7 and constant regions from mouse IgG2a.

Dosing regimen: Neonatal mice were intraperitoneally injected with RRV within 24 h after birth, and then intraperitoneally injected with an anti-CLDN6 antibody solution 4 h to 6 h later. The administration was started on day 0. The administration was conducted once every two days with a single dose of 1 mg/kg for 4 doses in total. A body weight of each neonatal mouse was recorded daily, and a jaundice phenotype of each neonatal mouse was observed. On day 12 after birth, a mouse liver tissue was collected, embedded in paraffin, and subjected to H&E and Masson staining to observe liver pathology. RNA was extracted from the mouse liver tissue by a Trizol method. Changes in mRNA levels of relevant genes were analyzed by real-time fluorescent quantitative PCR.

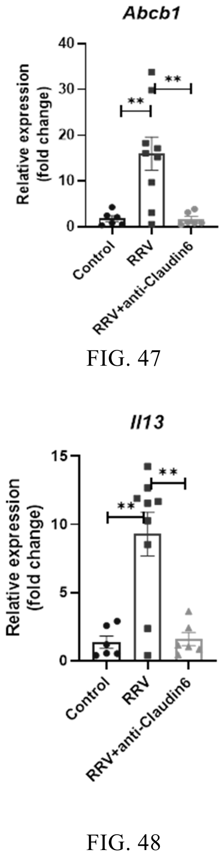

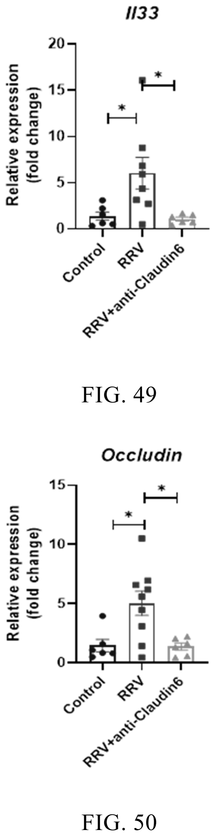

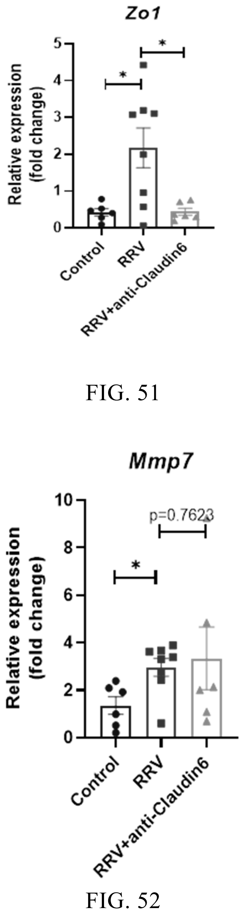

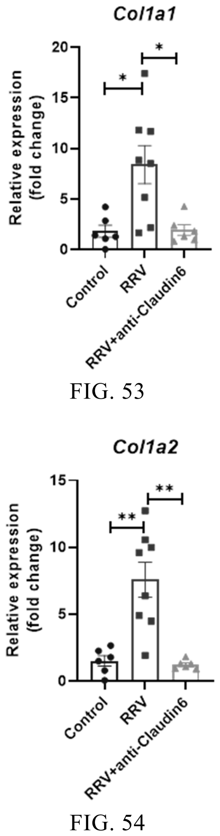

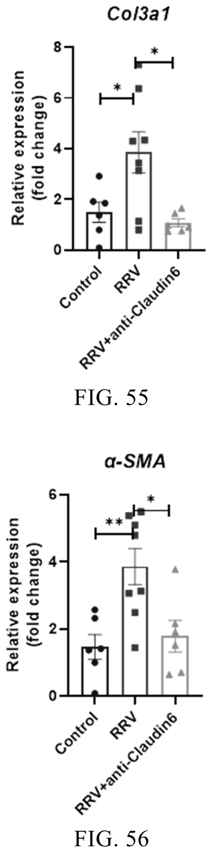

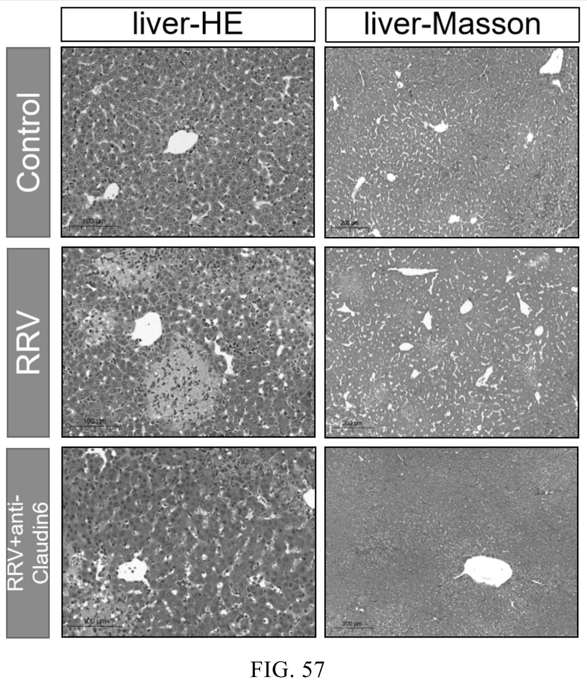

Results were shown in FIG. 45 to FIG. 57. During the progression of the RRV-induced mouse BA model, mRNA levels of liver fibrosis-associated genes and CLDN-associated genes were up-regulated (FIG. 47 to FIG. 56). After mice were administered with the anti-CLDN6 antibody, a body weight of the mice increased to approach a body weight of a control group (FIG. 45), but a jaundice rate of the mice decreased (FIG. 46). It can be seen that the anti-CLDN6 antibody can reduce the mRNA levels of liver fibrosis-associated genes and CLDN-associated genes (FIG. 47 to FIG. 56), and can improve the expression of CLDNs, thereby delaying liver injury and alleviating liver fibrosis (FIG. 57).

### INCORPORATION BY REFERENCE

The entire contents of patent documents and scientific documents mentioned in the present disclosure are incorporated herein by reference for all purposes.

### Equivalence

The present disclosure may be embodied in other specific forms without departing from the spirit or essential characteristics of the present disclosure. Therefore, the above embodiments should be considered illustrative in all cases, and do not limit the invention described herein. Therefore, the scope of the present disclosure is defined by the appended claims rather than the above description, and is intended to encompass all modifications falling within the meaning and scope equivalent to the claims.

## Claims

1. An isolated anti-claudin-6 (CLDN6) antibody or an antigen-binding fragment thereof, comprising: a heavy-chain variable region and a light-chain variable region,
wherein the isolated anti-CLDN6 antibody comprises:
(1) CDRH1 comprising or consisting of a sequence X₁YNMH, wherein X₁ is D or S;
(2) CDRH2 comprising or consisting of a sequence YX₁X₂PX₃X₄X₅X₆TX₇YNQKFKG, wherein X₁ is V or I, X₂ is N or Y, X₃ is N or G, X₄ is N, Q, S, or A, X₅ is A, G, D, Q, or S, X₆ is A or G, and X₇ is S or N;
(3) CDRH3 comprising or consisting of a sequence WX₁X₂YVYYYGX₃DY or a sequence shown in SEQ ID NO: 23, wherein X₁ is D, E, S, or G, X₂ is A, G, S, D, or Q, and X₃ is L or M;
(4) CDRL1 comprising or consisting of a sequence shown in SEQ ID NO: 27 or a sequence shown in SEQ ID NO: 29;
(5) CDRL2 comprising or consisting of a sequence shown in SEQ ID NO: 30 or a sequence shown in SEQ ID NO: 31; and
(6) CDRL3 comprising or consisting of a sequence QQYWX₁TPYT or a sequence shown in SEQ ID NO: 40, wherein X₁ is S or N;
preferably, the isolated anti-CLDN6 antibody comprises:
(1) CDRH1 comprising or consisting of a sequence shown in SEQ ID NO: 5;
(2) CDRH2 comprising or consisting of a sequence YX₁NPNX₂X₃ATX₄YNQKFKG, wherein X₁ is V or I, X₂ is N, Q, S, or A, X₃ is A, G, D, Q, or S, and X₄ is S or N;
(3) CDRH3 comprising or consisting of a sequence WDGYVYYYGX₁DY, wherein X₁ is L or M;
(4) CDRL1 comprising or consisting of the sequence shown in SEQ ID NO: 27;
(5) CDRL2 comprising or consisting of the sequence shown in SEQ ID NO: 31; and
(6) CDRL3 comprising or consisting of the sequence QQYWX₁TPYT, wherein X₁ is S or N; and
further preferably, the isolated anti-CLDN6 antibody comprises:
(1) CDRH1 comprising or consisting of the sequence shown in SEQ ID NO: 5;
(2) CDRH2 comprising or consisting of a sequence YX₁NPNNGATX₂YNQKFKG, wherein X₁ is V or I and X₂ is S or N;
(3) CDRH3 comprising or consisting of the sequence WDGYVYYYGX₁DY, wherein X₁ is L or M;
(4) CDRL1 comprising or consisting of the sequence shown in SEQ ID NO: 27;
(5) CDRL2 comprising or consisting of the sequence shown in SEQ ID NO: 31; and
(6) CDRL3 comprising or consisting of the sequence QQYWX₁TPYT, wherein X₁ is S or N; or
the isolated anti-CLDN6 antibody comprises:
(1) CDRH1 comprising or consisting of a sequence SYX₁IX₂, wherein X₁ is N or G and X₂ is H or S;
(2) CDRH2 comprising or consisting of a sequence X₁IYPX₂X₃GX₄TX₅X₆NEX₇FKG, wherein X₁ is Y or E, X₂ is G or R, X₃ is N or S, X₄ is G or N, X₅ is K or Y, X₆ is Y or H, and X₇ is R or K; (3) CDRH3 comprising a sequence shown in SEQ ID NO: 16 or a sequence shown in SEQ ID NO: 19;
(4) CDRL1 comprising or consisting of a sequence shown in SEQ ID NO: 24 or a sequence shown in SEQ ID NO: 26;
(5) CDRL2 comprising or consisting of a sequence X₁X₂SNLX₃S, wherein X₁ is L or G, X₂ is A or T, and X₃ is E or A; and (6) CDRL3 comprising or consisting of a sequence shown in SEQ ID NO: 34 or a sequence shown in SEQ ID NO: 179; or
the isolated anti-CLDN6 antibody comprises:
(1) CDRH1 comprising or consisting of a sequence X₁YX₂MS, wherein X₁ is S or N and X₂ is G or A;
(2) CDRH2 comprising or consisting of a sequence TISX1GGSYTX₂YPDX₃X₄KG, wherein X₁ is S or D, X₂ is Y or S, X₃ is S or N, and X₄ is V or I;
(3) CDRH3 comprising or consisting of a sequence shown in SEQ ID NO: 17 or a sequence shown in SEQ ID NO: 18;
(4) CDRL1 comprising or consisting of a sequence shown in SEQ ID NO: 25;
(5) CDRL2 comprising or consisting of a sequence X₁X₂TSLET, wherein X₁ is G or A and X₂ is A or T; and
(6) CDRL3 comprising or consisting of a sequence QQYWSX₁PX₂T, wherein X₁ is T or F and X₂ is P or R.

2. An isolated anti-CLDN6 antibody or an antigen-binding fragment thereof, comprising:
a heavy-chain variable region and a light-chain variable region,
wherein the heavy-chain variable region comprises:
(i) CDRH1 comprising any one of sequences shown in SEQ ID NOs: 1-5 and 7, or a sequence having a sequence identity of at least 90%, 91%, 92%, 93%, 94%, 95%, 96%, 97%, 98%, or 99% with any one of the sequences shown in SEQ ID NOs: 1-5 and 7;
(ii) CDRH2 comprising any one of sequences shown in SEQ ID NOs: 8-13, 15, and 74-76, or a sequence having a sequence identity of at least 90%, 91%, 92%, 93%, 94%, 95%, 96%, 97%, 98%, or 99% with any one of the sequences shown in SEQ ID NOs: 8-13, 15, and 74-76; and
(iii) CDRH3 comprising any one of sequences shown in SEQ ID NOs: 16-21, 23, and 77-78, or a sequence having a sequence identity of at least 90%, 91%, 92%, 93%, 94%, 95%, 96%, 97%, 98%, or 99% with any one of the sequences shown in SEQ ID NOs: 16-21, 23, and 77-78; and
the light-chain variable region comprises:
(i) CDRL1 comprising any one of sequences shown in SEQ ID NOs: 24-27 and 29, or a sequence having a sequence identity of at least 90%, 91%, 92%, 93%, 94%, 95%, 96%, 97%, 98%, or 99% with any one of the sequences shown in SEQ ID NOs: 24-27 and 29;
(ii) CDRL2 comprising any one of sequences shown in SEQ ID NOs: 30-33, or a sequence having a sequence identity of at least 90%, 91%, 92%, 93%, 94%, 95%, 96%, 97%, 98%, or 99% with any one of the sequences shown in SEQ ID NOs: 30-33; and
(iii) CDRL3 comprising any one of sequences shown in SEQ ID NOs: 34-38, 40, and 179, or a sequence having a sequence identity of at least 90%, 91%, 92%, 93%, 94%, 95%, 96%, 97%, 98%, or 99% with any one of the sequences shown in SEQ ID NOs: 34-38, 40, and 179.

3. The isolated anti-CLDN6 antibody or the antigen-binding fragment thereof according to claim 1 or 2, wherein the isolated anti-CLDN6 antibody or the antigen-binding fragment thereof comprises:
(a) CDRH1 comprising any one of the sequences shown in SEQ ID NOs: 1-5 and 7;
(b) CDRH2 comprising any one of the sequences shown in SEQ ID NOs: 8-13, 15, and 74-76;
(c) CDRH3 comprising any one of the sequences shown in SEQ ID NOs: 16-21, 23, and 77-78;
(d) CDRL1 comprising any one of the sequences shown in SEQ ID NOs: 24-27 and 29;
(e) CDRL2 comprising any one of the sequences shown in SEQ ID NOs: 30-33; and
(f) CDRL3 comprising any one of the sequences shown in SEQ ID NOs: 34-38, 40, and 179.

4. The isolated anti-CLDN6 antibody or the antigen-binding fragment thereof according to claim 1 or 2, wherein the isolated anti-CLDN6 antibody or the antigen-binding fragment thereof comprises:
(a) CDRH1 comprising or consisting of a sequence shown in SEQ ID NO: 1; CDRH2 comprising or consisting of a sequence shown in SEQ ID NO: 8; CDRH3 comprising or consisting of the sequence shown in SEQ ID NO: 16; CDRL1 comprising or consisting of the sequence shown in SEQ ID NO: 24; CDRL2 comprising or consisting of the sequence shown in SEQ ID NO: 30; and CDRL3 comprising or consisting of the sequence shown in SEQ ID NO: 34;
(b) CDRH1 comprising or consisting of a sequence shown in SEQ ID NO: 2; CDRH2 comprising or consisting of a sequence shown in SEQ ID NO: 9; CDRH3 comprising or consisting of the sequence shown in SEQ ID NO: 17; CDRL1 comprising or consisting of the sequence shown in SEQ ID NO: 25; CDRL2 comprising or consisting of the sequence shown in SEQ ID NO: 31; and CDRL3 comprising or consisting of a sequence shown in SEQ ID NO: 35;
(c) CDRH1 comprising or consisting of a sequence shown in SEQ ID NO: 3; CDRH2 comprising or consisting of a sequence shown in SEQ ID NO: 10; CDRH3 comprising or consisting of the sequence shown in SEQ ID NO: 18; CDRL1 comprising or consisting of the sequence shown in SEQ ID NO: 25; CDRL2 comprising or consisting of a sequence shown in SEQ ID NO: 32; and CDRL3 comprising or consisting of a sequence shown in SEQ ID NO: 36;
(d) CDRH1 comprising or consisting of a sequence shown in SEQ ID NO: 4; CDRH2 comprising or consisting of a sequence shown in SEQ ID NO: 11; CDRH3 comprising or consisting of the sequence shown in SEQ ID NO: 19; CDRL1 comprising or consisting of the sequence shown in SEQ ID NO: 26; CDRL2 comprising or consisting of a sequence shown in SEQ ID NO: 33; and CDRL3 comprising or consisting of the sequence shown in SEQ ID NO: 179;
(e) CDRH1 comprising or consisting of the sequence shown in SEQ ID NO: 5; CDRH2 comprising or consisting of a sequence shown in SEQ ID NO: 12; CDRH3 comprising or consisting of a sequence shown in SEQ ID NO: 20; CDRL1 comprising or consisting of the sequence shown in SEQ ID NO: 27; CDRL2 comprising or consisting of the sequence shown in SEQ ID NO: 31; and CDRL3 comprising or consisting of a sequence shown in SEQ ID NO: 37;
(f) CDRH1 comprising or consisting of the sequence shown in SEQ ID NO: 5; CDRH2 comprising or consisting of a sequence shown in SEQ ID NO: 13; CDRH3 comprising or consisting of a sequence shown in SEQ ID NO: 21; CDRL1 comprising or consisting of the sequence shown in SEQ ID NO: 27; CDRL2 comprising or consisting of the sequence shown in SEQ ID NO: 31; and CDRL3 comprising or consisting of a sequence shown in SEQ ID NO: 38;
(g) CDRH1 comprising or consisting of a sequence shown in SEQ ID NO: 7; CDRH2 comprising or consisting of a sequence shown in SEQ ID NO: 15; CDRH3 comprising or consisting of the sequence shown in SEQ ID NO: 23; CDRL1 comprising or consisting of the sequence shown in SEQ ID NO: 29; CDRL2 comprising or consisting of the sequence shown in SEQ ID NO: 30; and CDRL3 comprising or consisting of the sequence shown in SEQ ID NO: 40;
(h) CDRH1 comprising or consisting of the sequence shown in SEQ ID NO: 5; CDRH2 comprising or consisting of the sequence shown in SEQ ID NO: 12; CDRH3 comprising or consisting of the sequence shown in SEQ ID NO: 20; CDRL1 comprising or consisting of the sequence shown in SEQ ID NO: 27; CDRL2 comprising or consisting of the sequence shown in SEQ ID NO: 31; and CDRL3 comprising or consisting of the sequence shown in SEQ ID NO: 38;
(i) CDRH1 comprising or consisting of the sequence shown in SEQ ID NO: 7; CDRH2 comprising or consisting of the sequence shown in SEQ ID NO: 24; CDRH3 comprising or consisting of the sequence shown in SEQ ID NO: 23; CDRL1 comprising or consisting of the sequence shown in SEQ ID NO: 29; CDRL2 comprising or consisting of the sequence shown in SEQ ID NO: 30; and CDRL3 comprising or consisting of the sequence shown in SEQ ID NO: 40;
(j) CDRH1 comprising or consisting of the sequence shown in SEQ ID NO: 5; CDRH2 comprising or consisting of a sequence shown in SEQ ID NO: 75; CDRH3 comprising or consisting of the sequence shown in SEQ ID NO: 20; CDRL1 comprising or consisting of the sequence shown in SEQ ID NO: 27; CDRL2 comprising or consisting of the sequence shown in SEQ ID NO: 31; and CDRL3 comprising or consisting of the sequence shown in SEQ ID NO: 37;
(k) CDRH1 comprising or consisting of the sequence shown in SEQ ID NO: 5; CDRH2 comprising or consisting of a sequence shown in SEQ ID NO: 76; CDRH3 comprising or consisting of the sequence shown in SEQ ID NO: 20; CDRL1 comprising or consisting of the sequence shown in SEQ ID NO: 27; CDRL2 comprising or consisting of the sequence shown in SEQ ID NO: 31; and CDRL3 comprising or consisting of the sequence shown in SEQ ID NO: 37;
(l) CDRH1 comprising or consisting of the sequence shown in SEQ ID NO: 5; CDRH2 comprising or consisting of the sequence shown in SEQ ID NO: 12; CDRH3 comprising or consisting of a sequence shown in SEQ ID NO: 77; CDRL1 comprising or consisting of the sequence shown in SEQ ID NO: 27; CDRL2 comprising or consisting of the sequence shown in SEQ ID NO: 31; and CDRL3 comprising or consisting of the sequence shown in SEQ ID NO: 37; or
(m) CDRH1 comprising or consisting of the sequence shown in SEQ ID NO: 5; CDRH2 comprising or consisting of the sequence shown in SEQ ID NO: 12; CDRH3 comprising or consisting of a sequence shown in SEQ ID NO: 78; CDRL1 comprising or consisting of the sequence shown in SEQ ID NO: 27; CDRL2 comprising or consisting of the sequence shown in SEQ ID NO: 31; and CDRL3 comprising or consisting of the sequence shown in SEQ ID NO: 37.

5. The isolated anti-CLDN6 antibody or the antigen-binding fragment thereof according to any one of claims 1 to 4, wherein the heavy-chain variable region comprises or consists of any one of amino acid sequences shown in SEQ ID NOs: 41-46, 48-60, and 79-83, or comprises or consists of a sequence having a sequence identity of at least 85% comprising at least 90%, 91%, 92%, 93%, 94%, 95%, 96%, 97%, 98%, or 99% with any one of the amino acid sequences shown in SEQ ID NOs: 41-46, 48-60, and 79-83; and the light-chain variable region comprises or consists of any one of amino acid sequences shown in SEQ ID NOs: 61-66 and 68-73, or comprises or consists of a sequence having a sequence identity of at least 85% comprising at least 90%, 91%, 92%, 93%, 94%, 95%, 96%, 97%, 98%, or 99% with any one of the amino acid sequences shown in SEQ ID NOs: 61-66 and 68-73.

6. The isolated anti-CLDN6 antibody or the antigen-binding fragment thereof according to any one of claims 1 to 5, wherein the isolated anti-CLDN6 antibody or the antigen-binding fragment thereof comprises:
(a) a heavy-chain variable region comprising or consisting of an amino acid sequence shown in SEQ ID NO: 41 and a light-chain variable region comprising or consisting of an amino acid sequence shown in SEQ ID NO: 61;
(b) a heavy-chain variable region comprising or consisting of an amino acid sequence shown in SEQ ID NO: 42 and a light-chain variable region comprising or consisting of an amino acid sequence shown in SEQ ID NO: 62;
(c) a heavy-chain variable region comprising or consisting of an amino acid sequence shown in SEQ ID NO: 43 and a light-chain variable region comprising or consisting of an amino acid sequence shown in SEQ ID NO: 63;
(d) a heavy-chain variable region comprising or consisting of an amino acid sequence shown in SEQ ID NO: 44 and a light-chain variable region comprising or consisting of an amino acid sequence shown in SEQ ID NO: 64;
(e) a heavy-chain variable region comprising or consisting of any one of amino acid sequences shown in SEQ ID NOs: 45 and 80-83 and a light-chain variable region comprising or consisting of an amino acid sequence shown in SEQ ID NO: 65;
(f) a heavy-chain variable region comprising or consisting of an amino acid sequence shown in SEQ ID NO: 46 or 45 and a light-chain variable region comprising or consisting of an amino acid sequence shown in SEQ ID NO: 66;
(g) a heavy-chain variable region comprising or consisting of an amino acid sequence shown in SEQ ID NO: 48 or 79 and a light-chain variable region comprising or consisting of an amino acid sequence shown in SEQ ID NO: 68;
(h) a heavy-chain variable region comprising or consisting of any one of amino acid sequences shown in SEQ ID NOs: 49-52 and a light-chain variable region comprising or consisting of an amino acid sequence shown in SEQ ID NO: 69;
(i) a heavy-chain variable region comprising or consisting of any one of the amino acid sequences shown in SEQ ID NOs: 49-52 and a light-chain variable region comprising or consisting of an amino acid sequence shown in SEQ ID NO: 70;
(j) a heavy-chain variable region comprising or consisting of an amino acid sequence shown in SEQ ID NO: 53 or 54 and a light-chain variable region comprising or consisting of an amino acid sequence shown in SEQ ID NO: 71;
(k) a heavy-chain variable region comprising or consisting of any one of amino acid sequences shown in SEQ ID NOs: 53-56 and a light-chain variable region comprising or consisting of an amino acid sequence shown in SEQ ID NO: 72; or
(l) a heavy-chain variable region comprising or consisting of any one of amino acid sequences shown in SEQ ID NOs: 57-60 and a light-chain variable region comprising or consisting of an amino acid sequence shown in SEQ ID NO: 73.

7. The isolated anti-CLDN6 antibody or the antigen-binding fragment thereof according to any one of claims 1 to 6, wherein the isolated anti-CLDN6 antibody or the antigen-binding fragment thereof has one or more of following properties:
(a) binding to human or monkey CLDN6 with EC50 of 10 µg/mL or less;
(b) demonstrating a lower binding property to human CLDN3, human CLDN4, and human CLDN9 than to human CLDN6, and preferably, demonstrating a lower binding property to the human CLDN3 and the human CLDN4 than to the human CLDN6 and the human CLDN9;
(c) killing and/or inhibiting a cell expressing the human CLDN6.

8. An isolated anti-CLDN6 antibody or an antigen-binding fragment thereof, wherein the isolated anti-CLDN6 antibody or the antigen-binding fragment thereof competitively binds to CLDN6 expressed on a surface of a cell with the isolated anti-CLDN6 antibody or the antigen-binding fragment thereof according to any one of claims 1 to 7.

9. An isolated anti-CLDN6 antibody or an antigen-binding fragment thereof, comprising complementarity determining region (CDR) sequences selected from the group consisting of sequences shown in SEQ ID NOs: 41-46, 48-60, and 79-83 and CDR sequences selected from the group consisting of sequences shown in SEQ ID NOs: 61-66 and 68-73.

10. An isolated anti-CLDN6 antibody or an antigen-binding fragment thereof, comprising a heavy-chain variable region and a light-chain variable region,
wherein the isolated anti-CLDN6 antibody comprises:
(1) CDRH1 comprising or consisting of a sequence shown in SEQ ID NO: 5;
(2) CDRH2 comprising or consisting of a sequence shown in SEQ ID NO: 12, or comprising or consisting of a sequence YX₁NPNNGAX₂X₃YNQKFKG, wherein X₁ is A, I, L, or V, X₂ is S or T, and X₃ is S or N; and preferably, CDRH2 comprising or consisting of a sequence shown in SEQ ID NO: 12, 91, 92, 93, 94, 119, 120, 121, 122, 123, 124, or 125;
(3) CDRH3 comprising or consisting of a sequence shown in SEQ ID NO: 20, or comprising or consisting of a sequence WDGYX₁YYYGX₂DX₃, wherein X₁ is V or S, X₂ is V, L, or M, and X₃ is Y or F; and preferably, CDRH3 comprising or consisting of a sequence shown in SEQ ID NO: 20, 95, 96, 97, 126, 127, 128, 129, 130, or 131;
(4) CDRL1 comprising or consisting of a sequence shown in SEQ ID NO: 27, or comprising or consisting of a sequence KASX₁HX₂NX₃WLA, wherein X₁ is E or D, X₂ is I or V, and X₃ is H or Q; and preferably, CDRL1 comprising or consisting of a sequence shown in SEQ ID NO: 27, 98, 99, 100, 132, 133, 134, or 135;
(5) CDRL2 comprising or consisting of a sequence shown in SEQ ID NO: 31, or comprising or consisting of a sequence shown in SEQ ID NO: 101; and
(6) CDRL3 comprising or consisting of a sequence shown in SEQ ID NO: 37, or comprising or consisting of a sequence QQYX₁X₂TPYX₃, wherein X₁ is W or G, X₂ is S, H, or N, and X₃ is S or T; and preferably, CDRL3 comprising or consisting of a sequence shown in SEQ ID NO: 37, 102, 103, 104, 136, 137, 138, 139, 140, 141, or 142;
preferably, the isolated anti-CLDN6 antibody comprises:
(1) CDRH1 comprising or consisting of the sequence shown in SEQ ID NO: 5;
(2) CDRH2 comprising or consisting of the sequence shown in SEQ ID NO: 12, or comprising or consisting of the sequence YX₁NPNNGAX₂X₃YNQKFKG, wherein X₁ is A, I, L, or V, X₂ is S or T, and X₃ is S or N; and preferably, CDRH2 comprising or consisting of the sequence shown in SEQ ID NO: 12, 91, 92, 93, 94, 119, 120, 121, 122, 123, 124, or 125;
(3) CDRH3 comprising or consisting of the sequence shown in SEQ ID NO: 20;
(4) CDRL1 comprising or consisting of the sequence shown in SEQ ID NO: 27;
(5) CDRL2 comprising or consisting of the sequence shown in SEQ ID NO: 31; and
(6) CDRL3 comprising or consisting of the sequence shown in SEQ ID NO: 37; or
the isolated anti-CLDN6 antibody comprises:
(1) CDRH1 comprising or consisting of the sequence shown in SEQ ID NO: 5;
(2) CDRH2 comprising or consisting of the sequence shown in SEQ ID NO: 12;
(3) CDRH3 comprising or consisting of the sequence shown in SEQ ID NO: 20, or comprising or consisting of the sequence WDGYX₁YYYGX₂DX₃, wherein X₁ is V or S, X₂ is V, L, or M, and X₃ is Y or F; and preferably, CDRH3 comprising or consisting of the sequence shown in SEQ ID NO: 20, 95, 96, 97, 126, 127, 128, 129, 130, or 131; (4) CDRL1 comprising or consisting of the sequence shown in SEQ ID NO: 27;
(5) CDRL2 comprising or consisting of the sequence shown in SEQ ID NO: 31; and
(6) CDRL3 comprising or consisting of the sequence shown in SEQ ID NO: 37; or
the isolated anti-CLDN6 antibody comprises:
(1) CDRH1 comprising or consisting of the sequence shown in SEQ ID NO: 5;
(2) CDRH2 comprising or consisting of the sequence shown in SEQ ID NO: 12;
(3) CDRH3 comprising or consisting of the sequence shown in SEQ ID NO: 20;
(4) CDRL1 comprising or consisting of the sequence shown in SEQ ID NO: 27, or comprising or consisting of the sequence KASX₁HX₂NX₃WLA, wherein X₁ is E or D, X₂ is I or V, and X₃ is H or Q; and preferably, CDRL1 comprising or consisting of the sequence shown in SEQ ID NO: 27, 98, 99, 100, 132, 133, 134, or 135;
(5) CDRL2 comprising or consisting of the sequence shown in SEQ ID NO: 31; and
(6) CDRL3 comprising or consisting of the sequence shown in SEQ ID NO: 37; or
the isolated anti-CLDN6 antibody comprises:
(1) CDRH1 comprising or consisting of the sequence shown in SEQ ID NO: 5;
(2) CDRH2 comprising or consisting of the sequence shown in SEQ ID NO: 12;
(3) CDRH3 comprising or consisting of the sequence shown in SEQ ID NO: 20;
(4) CDRL1 comprising or consisting of the sequence shown in SEQ ID NO: 27;
(5) CDRL2 comprising or consisting of the sequence shown in SEQ ID NO: 31, or comprising or consisting of the sequence shown in SEQ ID NO: 101; and
(6) CDRL3 comprising or consisting of the sequence shown in SEQ ID NO: 37; or
the isolated anti-CLDN6 antibody comprises:
(1) CDRH1 comprising or consisting of the sequence shown in SEQ ID NO: 5;
(2) CDRH2 comprising or consisting of the sequence shown in SEQ ID NO: 12;
(3) CDRH3 comprising or consisting of the sequence shown in SEQ ID NO: 20;
(4) CDRL1 comprising or consisting of the sequence shown in SEQ ID NO: 27;
(5) CDRL2 comprising or consisting of the sequence shown in SEQ ID NO: 31; and
(6) CDRL3 comprising or consisting of the sequence shown in SEQ ID NO: 37, or comprising or consisting of the sequence QQYX₁X₂TPYX₃, wherein X₁ is W or G, X₂ is S, H, or N, and X₃ is S or T; and preferably, CDRL3 comprising or consisting of the sequence shown in SEQ ID NO: 37, 102, 103, 104, 136, 137, 138, 139, 140, 141, or 142; or
the isolated anti-CLDN6 antibody comprises:
(1) CDRH1 comprising or consisting of the sequence shown in SEQ ID NO: 5;
(2) CDRH2 comprising or consisting of any one of sequences shown in SEQ ID NOs: 12, 91-94, and 119-125;
(3) CDRH3 comprising or consisting of any one of sequences shown in SEQ ID NOs: 20, 95-97, and 126-131;
(4) CDRL1 comprising or consisting of any one of sequences shown in SEQ ID NOs: 27, 98-99, and 132-135;
(5) CDRL2 comprising or consisting of a sequence shown in SEQ ID NO: 31 or 101; and
(6) CDRL3 comprising or consisting of any one of sequences shown in SEQ ID NOs: 37, 102-104, and 136-142; and
preferably, the isolated anti-CLDN6 antibody comprises or consists of CDRs shown in Table K.

11. An isolated anti-CLDN6 antibody or an antigen-binding fragment thereof, comprising a heavy-chain variable region and a light-chain variable region,
wherein the heavy-chain variable region comprises or consists of any one of amino acid sequences shown in SEQ ID NOs: 105-111, or comprises or consists of a sequence having a sequence identity of at least 80% comprising at least 85%, 90%, 91%, 92%, 93%, 94%, 95%, 96%, 97%, 98%, or 99% with any one of the amino acid sequences shown in SEQ ID NOs: 105-111; and the light-chain variable region comprises or consists of an amino acid sequence shown in SEQ ID NO: 69, or comprises or consists of a sequence having a sequence identity of at least 85% comprising at least 90%, 91%, 92%, 93%, 94%, 95%, 96%, 97%, 98%, or 99% with the amino acid sequence shown in SEQ ID NO: 69; or
the heavy-chain variable region comprises or consists of an amino acid sequence shown in SEQ ID NO: 52, or comprises or consists of a sequence having a sequence identity of at least 80% comprising at least 85%, 90%, 91%, 92%, 93%, 94%, 95%, 96%, 97%, 98%, or 99% with the amino acid sequence shown in SEQ ID NO: 52; and the light-chain variable region comprises or consists of any one of amino acid sequences shown in SEQ ID NOs: 112-118, or comprises or consists of a sequence having a sequence identity of at least 80% comprising at least 85%, 90%, 91%, 92%, 93%, 94%, 95%, 96%, 97%, 98%, or 99% with any one of the amino acid sequences shown in SEQ ID NOs: 112-118.

12. The isolated anti-CLDN6 antibody or the antigen-binding fragment thereof according to any one of claims 1 to 11, wherein the isolated anti-CLDN6 antibody is a monoclonal antibody.

13. The isolated anti-CLDN6 antibody or the antigen-binding fragment thereof according to any one of claims 1 to 11, wherein the isolated anti-CLDN6 antibody is a chimeric antibody or a humanized antibody.

14. The isolated anti-CLDN6 antibody or the antigen-binding fragment thereof according to any one of claims 1 to 13, wherein the isolated anti-CLDN6 antibody comprises a constant region of immunoglobulin G (IgG).

15. The isolated anti-CLDN6 antibody or the antigen-binding fragment thereof according to claim 14, wherein the constant region of the IgG is selected from a constant region of IgG1, IgG2, IgG3, or IgG4.

16. The isolated anti-CLDN6 antibody or the antigen-binding fragment thereof according to claim 15, wherein the constant region of the IgG is selected from a constant region of the IgG1.

17. The isolated anti-CLDN6 antibody or the antigen-binding fragment thereof according to any one of claims 1 to 16, comprising: a heavy chain comprising or consisting of any one selected from the group consisting of polypeptides having a sequence identity of at least 90%, 91%, 92%, 93%, 94%, 95%, 96%, 97%, 98%, 99%, or 100% with a sequence shown in any of SEQ ID NOs: 143-167 or SEQ ID NO: 87 or SEQ ID NO: 89; and a light chain comprising or consisting of any one selected from the group consisting of polypeptides having a sequence identity of at least 90%, 91%, 92%, 93%, 94%, 95%, 96%, 97%, 98%, 99%, or 100% with a sequence shown in any of SEQ ID NOs: 168-178 or SEQ ID NO: 88 or SEQ ID NO: 90.

18. An isolated nucleic acid comprising a nucleic acid sequence encoding the isolated anti-CLDN6 antibody or the antigen-binding fragment thereof according to any one of claims 1 to 17.

19. A vector comprising the nucleic acid according to claim 18.

20. A host cell comprising the nucleic acid according to claim 18 or the vector according to claim 19.

21. A conjugate comprising the isolated anti-CLDN6 antibody or the antigen-binding fragment thereof according to any one of claims 1 to 17 that is conjugated to at least one detectable label.

22. An antibody-drug conjugate comprising an antibody, wherein the antibody-drug conjugate comprises one or more drug moieties, and the one or more drug moieties are linked to the isolated anti-CLDN6 antibody or the antigen-binding fragment thereof according to any one of claims 1 to 17 directly or indirectly through a linker (preferably, covalent linking).

23. A multispecific molecule comprising the isolated anti-CLDN6 antibody or the antigen-binding fragment thereof according to any one of claims 1 to 17, wherein preferably, the multispecific molecule specifically binds to CLDN6 and additionally specifically binds to one or more other targets; and more preferably, the multispecific molecule further comprises at least one molecule with a second binding specificity for a second target.

24. A chimeric antigen receptor (CAR), comprising the isolated anti-CLDN6 antibody or the antigen-binding fragment thereof according to any one of claims 1 to 17 and an immune cell activation moiety, wherein the immune cell activation moiety comprises a transmembrane domain of an immune cell receptor and/or an intracellular signaling domain of the immune cell receptor.

25. A pharmaceutical composition or kit, comprising the isolated anti-CLDN6 antibody or the antigen-binding fragment thereof according to any one of claims 1 to 17, the nucleic acid according to claim 18, the vector according to claim 19, the host cell according to claim 20, the conjugate according to claim 21, the antibody-drug conjugate according to claim 22, the multispecific molecule according to claim 23, or the CAR according to claim 24; and a pharmaceutically acceptable carrier.

26. A method for preparing the isolated anti-CLDN6 antibody or the antigen-binding fragment thereof according to any one of claims 1 to 17, comprising following steps:
(i) expressing the isolated anti-CLDN6 antibody or the antigen-binding fragment thereof according to any one of claims 1 to 17 in the host cell according to claim 20; and optionally
(ii) isolating the isolated anti-CLDN6 antibody or the antigen-binding fragment thereof from the host cell.

27. A method for detecting CLDN6 in a sample or determining a content of the CLDN6 in the sample, comprising following steps:
(i) allowing the sample to be in contact with the isolated anti-CLDN6 antibody or the antigen-binding fragment thereof according to any one of claims 1 to 17 or the conjugate according to claim 21; and
(ii) detecting formation of a complex of the isolated anti-CLDN6 antibody, the antigen-binding fragment thereof, or the conjugate with the CLDN6, or determining an amount of the complex.

28. The method according to claim 27, wherein the sample comprises a cell.

29. A use of the isolated anti-CLDN6 antibody or the antigen-binding fragment thereof according to any one of claims 1 to 17, the nucleic acid according to claim 18, the vector according to claim 19, the host cell according to claim 20, the conjugate according to claim 21, the antibody-drug conjugate according to claim 22, the multispecific molecule according to claim 23, the CAR according to claim 24, or the pharmaceutical composition or kit according to claim 25 in preparation of a kit for diagnosing, detecting, or monitoring a disease associated with CLDN6 expression.

30. A use of the isolated anti-CLDN6 antibody or the antigen-binding fragment thereof according to any one of claims 1 to 17, the nucleic acid according to claim 18, the vector according to claim 19, the host cell according to claim 20, the conjugate according to claim 21, the antibody-drug conjugate according to claim 22, the multispecific molecule according to claim 23, the CAR according to claim 24, or the pharmaceutical composition or kit according to claim 25 in preparation of a drug for treating a disease associated with CLDN6 expression or for determining prognosis of the disease.

31. The use according to claim 29 or 30, wherein the disease associated with the CLDN6 expression is cancer; and the cancer is preferably selected from the group consisting of ovarian cancer, lung cancer, gastric cancer, breast cancer, liver cancer, pancreatic cancer, skin cancer, malignant melanoma, head and neck cancer, sarcoma, cholangiocarcinoma, bladder cancer, kidney cancer, colon cancer, placental choriocarcinoma, cervical cancer, testicular cancer, and uterine cancer.

32. The use according to claim 29 or 30, wherein the disease associated with the CLDN6 expression is a fibrotic disease; and the fibrotic disease is preferably selected from the group consisting of pulmonary fibrosis, hepatic fibrosis, colonic fibrosis, biliary fibrosis, and gastric fibrosis and is more preferably selected from the group consisting of hepatitis, pulmonary fibrosis, hepatic fibrosis, cirrhosis, scleroderma, and biliary atresia.

33. The use according to claim 29 or 30, wherein a subject is a mammal and preferably a human.
